# EUROPEAN PATENT APPLICATION

(11) **EP 3 838 266 A1**
(43) Date of publication of application: **23.06.2021**
(21) Application number: 19218881.1
(22) Date of filing: 20.12.2019
(51) Int. Cl.: A61K 9/127, A61K 9/50, B01L 3/00, A61J 3/00, B01J 13/00

(54) **MICROFLUIDIC PRODUCTION OF BIOFUNCTIONALIZED GIANT UNILAMELLAR VESICLES FOR TARGETED INTRACELLULAR CARGO DELIVERY**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to a method for preparation of monodisperse cell-targeting giant unilamellar vesicles based on symmetrically division of a parent polymer shell-stabilized giant unilamellar vesicle into smaller polymer shell-stabilized giant unilamellar vesicles with a diameter smaller than 10 µm using a microfluidic splitting device.

The inventive method allows preparation of differently charged giant unilamellar vesicles as well as bioligand- and PEG-conjugated giant unilamellar vesicles, which are useful for targeted cellular delivery at high efficiency and specificity. A further advantage of the present invention is that the giant unilamellar vesicles can deliver huge cargos such as drug releasing porous microparticles, high amounts of in vivo imaging probes, viruses, or up-and-coming DNA origami robots.

## Description

The present invention relates to a method for preparation of monodisperse cell-targeting giant unilamellar vesicles based on symmetrically division of a parent polymer shell-stabilized giant unilamellar vesicle into smaller polymer shell-stabilized giant unilamellar vesicles with a diameter smaller than 10 µm using a microfluidic splitting device.

The inventive method allows preparation of differently charged giant unilamellar vesicles as well as bioligand- and PEG-conjugated giant unilamellar vesicles, which are useful for targeted cellular delivery at high efficiency and specificity. A further advantage of the present invention is that the giant unilamellar vesicles can deliver huge cargos such as drug releasing porous microparticles, high amounts of in vivo imaging probes, viruses, or up-and-coming DNA origami robots.

### Background of the invention

Lipid-based small unilamellar vesicles (SUVs), with diameters smaller than 100 nm, have been extensively studied as transport systems in the food, cosmetic and pharmaceutical industries. Particularly, they have been implemented to serve as nanosensors for monitoring food quality during storage, as vehicles for the transdermal delivery of cosmetic agents, or most recently as carrier systems for targeted drug therapy. Such liposomal delivery systems offer improved control over drug pharmacokinetics and pharmacodynamics, provide potential to decrease toxicity and adverse side effects, enhance targeted delivery to specific tissues and increase compound circulation times.

Despite the beneficial impact of delivery systems based on small unilamellar vesicles, the road towards their broad applicability is complex. The major problems are the following:
First, the total amount of pharmaceutically active compounds that can be entrapped in a single liposome is quite low, potentially not reaching the desired therapeutic dose at its destination.
Second, the methods known in the state of the art possess very low entrapment efficiency into liposomes, and the method comprising active loading approaches are expensive and time consuming.

Third, efficient methods to load large cargos, like nanoparticles or supramolecular DNA complexes, into small liposomes for therapeutic approaches are still missing.

Hence, for drug delivery applications, giant unilamellar vesicles (GUVs) with diameters comprised between 1 and 10 µm might offer great potential, as they can carry massive quantities of active compounds as well as particles up to several micrometres in size.

Despite the growing demand for biomedical and synthetic biology applications based on giant unilamellar vesicles, the potential of giant unilamellar vesicles to deliver large and complex cargos into the intracellular space or, in general, their ability to interact with living cells have been only superficially explored.

Microfluidic approaches offer the ability to produce monodisperse giant unilamellar vesicles at high throughput with various contents (Stein H. et al., 2017 "Production of isolated giant unilamellar vesicles under high salt concentrations", Frontiers in Phisiology).

Funakoshi et al. introduced jetting as a microfluidic method for giant unilamellar vesicle formation (Funakoshi et al., 2007 "Formation of giant lipid vesicle like compartments from a planar lipid membrane by a pulsed jet flow", J. Am. Chem. Soc.). Here, the buffer solution is shot onto a pre-assembled lipid bilayer at a water-oil interface using a micronozzle or micropipette and giant unilamellar vesicles are formed out of this bilayer. The size, the inner and outer solution as well as the lipid composition of the inner and outer leaflets can be adapted. However, a residual amount of oil needed to form the bilayer at the interface with a reservoir of dissolved lipids stays in the hydrophobic core of the membrane. This can lead to altered mechanical properties and diffusional behavior in and across the membrane.

Shum et al. developed another microfluidic approach in 2008 to produce monodisperse giant unilamellar vesicles at a water-oil-water interface, the so-called double emulsion method (Shum et al., 2008, Double emulsion templated monodisperse phospholipid vesicles. Langmuir). Here, water-in-oil-in-water droplets are formed, and solvents that are contained in the oil phase evaporate, thus forming unilamellar vesicles out of the double emulsion droplets. However, also here some residual solvents can remain trapped within the bilayer, rendering the unilamellar vesicles unsuitable for diffusion studies or domain formation. This method has been improved to form ultrathin shell double emulsion vesicles with minimized amount of residual solvents, thus allowing for microdomain formation (Stein et al., 2007). Following this method, Stein et al. use a microfluidic capillary device to produce giant unilamellar vesicles with a diameter bigger than 200 µm.

The assembly of giant unilamellar vesicles from small unilamellar vesicles precursors within microfluidic water-in-oil droplet architectures offers great control over giant unilamellar vesicle formulation as well as high production rates.

This approach has previously been used to produce cell sized or even larger giant unilamellar vesicles with diameters of up to 50 µm (Spatz et al., WO 2018/228894 A1). However, giant unilamellar vesicles equally sized or larger than typical cells cannot be used for drug-delivery applications. Formation of polymer shell stabilized unilamellar vesicles of size comprised between 1 µm and 10 µm involves sophisticated lithography procedures and complex channel architectures. These drawbacks usually restrict reliable high throughput production of polymer shell stabilized unilamellar vesicles in size comprised between 1 µm and 10 µm.

Thus, it is the objective of the present invention to provide giant unilamellar vesicles with an optimal size comprised between 1 µm and 10 µm for drug-delivery applications, and with a controlled lipid composition and optionally surface bioligands allowing a cell specific drug delivery. Importantly, the giant unilamellar vesicles disclosed herein are able to incorporate complex cargos, such as virus particles. The high level of control over lipid and luminal composition is possible by producing the giant unilamellar vesicles within a polymer shell that stabilizes the inner unilamellar vesicle.

The objective of the present invention is solved by the teachings of the independent claims. Further advantageous features, aspects and details of the invention are evident from the dependent claims, the description, the figures, and the examples of the present application.

### Brief description of the invention

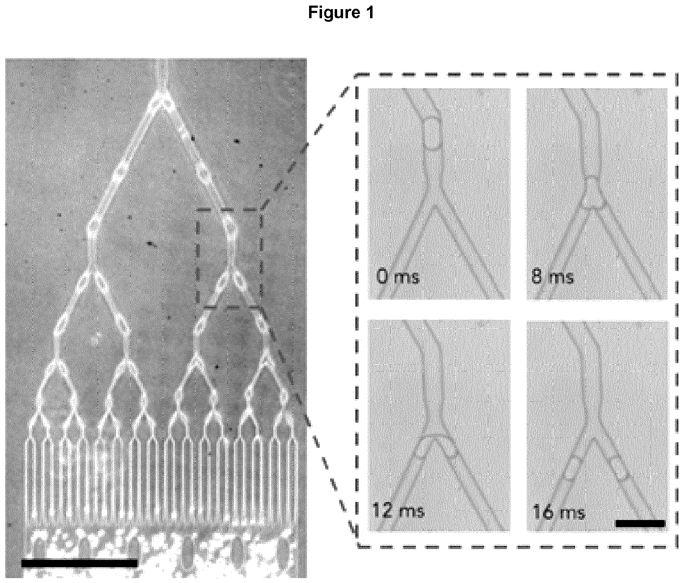

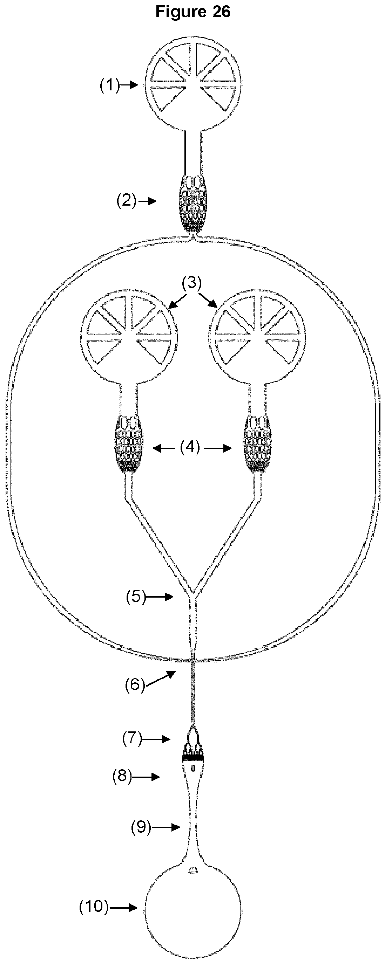

In order to overcome the limitations of the prior art described above, the present invention provides a method for production of polymer shell stabilized unilamellar vesicles with a diameter comprised between 1 µm and 10 µm, which can be used for efficient and specific delivery of advanced cargos. This has been reached by developing a microfluidic device comprising a multi-Y-shaped droplet splitting unit (division zone, Figure 1, (7) in Figure 26) generating small polymer shell stabilized unilamellar vesicles with a diameter below 10 µm, starting from a parent polymer shell stabilized vesicles, with a diameter of preferably more than 10µm.

The presented technology is based on three subsequent steps:
1) well-controlled assembly of polymer shell stabilized giant unilamellar vesicles, including use of functionalized lipids, fine-tuning of charge-mediated cell interactions, decrease of non-specific interactions, lysosomal escape mechanisms for efficient cytoplasmic release, loading of heavy duty cargos;
2) mechanical splitting of the polymer shell stabilized giant unilamellar vesicles;
3) release of the splitted polymer shell stabilized giant unilamellar vesicles into the physiological environment.

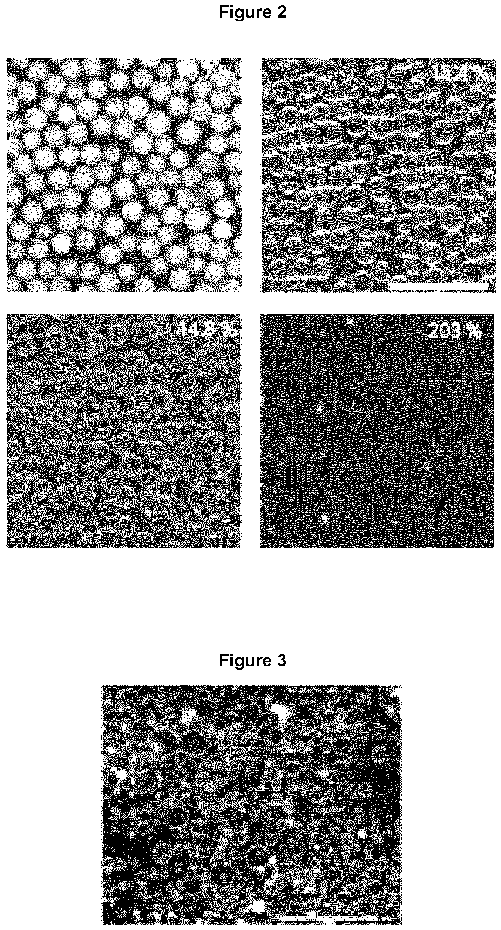

A first technical advantage of the inventive method is the high throughput production by mechanical splitting of a large amount of polymer shell stabilized unilamellar vesicles with a final diameter of 1 µm - 10 µm (Figures 2, 3), which can be functionalized to interact at high specificity with the target cells for delivery of advanced cargos.

A second technical advantage of the invention is that it provides giant unilamellar vesicles with highly efficient cargo loading and unpreceded controlled biological and physicochemical properties, which are essential for high specific drug delivery applications.

A third technical advantage is that the inventive biofunctionalized giant unilamellar vesicles allow for targeted transport of their cargo, avoiding cargo degradation inside the vesicle lumen and immune-recognition. Once at its destination, the giant unilamellar vesicle cargo can be intracellularly discharged via the developed lysosomal escape mechanism.

A fourth technical advantage of the present invention is that the inventive giant unilamellar vesicles can encapsulate and deliver complex and heavy duty as well as high molecular weight cargos, such as drug releasing porous microparticles, high amounts of *in vivo* imaging probes, gigabase long DNA, viruses, or up-and-coming DNA origami robots.

In particular, the present invention is directed to a method for preparation of monodisperse cell-targeting giant unilamellar vesicles comprising the following steps:
a) providing a polymer shell-stabilized giant unilamellar vesicle;
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) optionally removing the polymer shell from the polymer shell-stabilized giant unilamellar vesicles obtained in step c).

In accordance with one embodiment of the invention, the polymer shell-stabilized unilamellar vesicle provided in step a) is obtained by the following step:
a') merging a water phase comprising at least one lipid, and cations, and an oil phase comprising an amphiphilic copolymer,
a") optionally integrating one or more proteins or fragments thereof into the polymer shell stabilized giant unilamellar vesicle provided in step a')

According to an aspect of the present invention, the step d) comprises removing the polymer shell from the polymer shell-stabilized giant unilamellar vesicles obtained in step c) by adding a deemulsifier.

According to an aspect of the present invention, the method for preparation of monodisperse cell-targeting giant unilamellar vesicles further comprises the following step after step d):
e) purifying giant unilamellar vesicles from step d) by centrifugation .

According to a further aspect of the present invention, the water phase of step a') comprises at least one lipid selected from the group comprising:
a neutral lipid selected from the group comprising ceramide, sphingomyelin, cephalin, cholesterol, cerebrosides, diacylglycerols, phosphatidylcholines, lysophosphatidylcholines, phosphatidylethanolamines, lysophosphatidylethanolamine, lysoethanolamines, inverted headgroup lipids, sphingosins, sterol-modified phospholipids, ether ester lipids, diether lipids, vinyl ether (plasmalogen);
an anionic lipid selected from the group comprising phosphatidic acids, lysophosphatidic acid derivatives, phosphatidylglycerols, lysophosphatidylglycerols, phosphatidylserines, lysophosphatidylserines, phosphatidylinositols, phosphatidylinositolphosphates, cardiolipins, Bis(Monoacylglycero)Phosphate derivatives;
a cationic lipid selected from the group comprising dioleyl-N,N-dimethylammonium chloride; N-(2,3-dioleyloxy)propyl)-N,N,N-trimethylammonium chloride; N,N-distearyl-N,N-dimethylammonium bromide; N-(2,3- dioleyloxy)propyl)-N,N,N-trimethylammonium chloride; 3β-(N-(N',N'-dimethylaminoethane)-carbamoyl)cholesterol; 1,2-dimyristyloxypropyl-3-dimethyl-hydroxy ethyl ammonium bromide; 2,3-dioleyloxy-N-[2(sperminecarboxamido) ethyl]-N,N-dimethyl-1-propanaminium trifluoroacetate; dioctadecylamidoglycyl carboxyspermine; N-(2,3-dioleyloxy)propyl)-N,N-dimethylammonium chloride and 1,2-dioleoyl-3-dimethylammonium-propane;
a pH-sensitive lipid selected from the group comprising lipid N-(4-carboxybenzyl)-N,N-dimethyl-2,3-bis(oleoyloxy)propan-1-aminium, 1,2-distearoyl-3-dimethylammonium-propane, 1,2-dipalmitoyl-sn-glycero-3-succinate, 1,2-dioleoyl-sn-glycero-3-succinate, N-palmitoyl homocysteine ;
a photoswitchable lipid;
Acylglycine derivatives, prenol derivatives, prostaglandine derivatives, glycosylated diacyl glycerols, eicosanoid derivatives, (palmitoyloxy)octadecanoic acid derivatives, diacetylene derivatives, diphytanoyl derivatives, fluorinated lipids, brominated lipids, lipopolysaccharides;
one of the aforementioned lipids coupled to a functional ligand selected from biotin, N-hydroxysuccinimide ester, nitrilotriacetic acid -nickel, polyethylene glycol, amine, carboxylic acid, maleimides, dithiopyridinyl, pyridyl disulfide, pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, a chelator.

According to a further aspect of the present invention, the water phase of step a') comprises at least one anionic lipid, at least one neutral lipid, and optionally one neutral lipid functionalized with a fluorescent dye molecule.

According to a further aspect of the present invention, the water phase of step a') comprises at least one cationic lipid, at least one neutral lipid, and optionally one neutral lipid functionalized with a fluorescent dye molecule.

According to a further aspect of the present invention, the water phase of step a') comprises at least one lipid functionalized with a functional ligand selected from biotin, N-hydroxysuccinimide (NHS) ester, nitrilotriacetic acid (NTA)-nickel, amine, carboxylic acid, maleimide, dithiopyridinyl, N-benzylguanine, carboxyacyl, cyanur, square, galloyl, thiol,
wherein the method optionally comprises after step d) the following step:
d') coupling the giant unilamellar vesicles with at least one macromolecule comprising at least one moiety reacting with one of said functional ligands, wherein the macromolecule is selected from the group comprising a carbohydrate, a nucleic acid, a protein or a fragment thereof, a polypeptide, a cell receptor, an imaging probe, a nanoparticle.

According to a further aspect of the present invention, the water phase of step a') comprises at least one lipid coupled to polyethyleneglycol with a molecular weight comprised between 350 and 50,000 g/mol.

According to a further aspect of the present invention the water phase of step a') comprises at least one pH-sensitive lipid at a molar percentage comprised between 20 % - 80 %, or wherein the water phase of step a') further comprises poly-ethylene-imine at a concentration comprised between 2 - 100 µg/ml

According to a further aspect of the present invention, wherein the water phase of step a') further comprises at least one agent selected from the group comprising drug releasing porous particles, molecular imaging agents, diagnostic agents, therapeutic agents, proteins or fragments thereof, polypeptides, peptides, enzymes or fragments thereof, nucleic acids, oligonucleotides, polynucleotides, up-and-coming DNA origami robots, small molecule drugs, virus particles, virus-like particles, microbial antigens, steroids, proteoglycans, lipids, monosaccharides, oligosaccharides, polysaccharides, magnetic particles, nanorods, carbon nanotubes, dentritosomes, polymerosomes, metal nanoparticles and combinations or conjugates thereof.

According to a further aspect of the present invention, the amphiphilic copolymer of step b) consists of (i) a triblock copolymer comprising two perfluorinated polymer end blocks and one polyether glycol block, or of (ii) a diblock copolymer comprising one perfluorinated polymer end block and a polyether glycol block, wherein the triblock or diblock copolymer is folded so that the perfluorinated polymer end blocks are arranged at the outer side and that the polyether glycol block is arranged at the inner side of the polymer shell.

According to a further aspect of the present invention, the step b) comprises mechanically dividing said polymer shell stabilized giant unilamellar vesicle into two smaller polymer shell stabilized giant unilamellar vesicles using a microfluidic device comprising at least one Y-shaped junction, wherein said Y-shaped junction consists of one inlet channel and two outlet channels, and wherein step c) comprises repeating the step b) by using four or more sequential generations of Y-shaped junctions, wherein the inlet channel of each Y-shaped junction consists of the outlet channel of the previous Y-shaped junction.

Moreover, the present invention is directed to a microfluidic device for preparing polymer shell stabilized giant unilamellar vesicles having a diameter smaller than 10 µm, comprising a division zone and a flow-rate control system,
wherein the division zone comprises one or more sequential generations of Y-shaped junctions, wherein each Y-shaped junction consists of one inlet channel and two outlet channels, and wherein the inlet channel of each Y-shaped junction consists of the outlet channel of the previous junction.

In a further embodiment, the microfluidic device for preparing polymer shell stabilized giant unilamellar vesicles further comprises a generation zone of a parent polymer shell stabilized giant unilamellar vesicle, positioned upstream the division zone, said generation zone comprising a flow-focusing junction consisting of a horizontal inlet channel and two vertical inlet channels, wherein said three inlet channels converge into an outlet channel through a narrow orifice, wherein said outlet channel is connected to the division zone.

### Definitions

Liposomes are composed of a lipid bilayer separating an aqueous internal compartment from the bulk aqueous phase. A liposome is a spherical vesicle having at least one lipid bilayer. The major types of liposomes are the multilamellar vesicles (MLV, with several lamellar phase lipid bilayers), the small unilamellar liposome vesicles (SUV, with one lipid bilayer), the large unilamellar vesicles (LUV), and the giant unilamellar vesicles (GUV).

Small unilamellar vesicles, large unilamellar vesicles and giant unilamellar vesicles are usually spherical with a diameter of typically 25 to 50 nm for small unilamellar vesicles, with a diameter of typically more than 50 to 1,000 nm for large unilamellar vesicles, and with a diameter of typically more than 1 to 1,000 µm for giant unilamellar vesicles. However, the chemical and mechanical instabilities of unsaturated fatty acids under high ionic strength conditions, especially multivalent cations, and their sensitivity to pH changes are considered to be the main challenges in utilizing these vesicles. In addition, inserting molecules into these vesicles represents a particular challenge given their impermeability and mechanical instability.

The term "vesicle" refers to a non-natural or synthetic membranous and usually fluid-filled pouch resulting from the supramolecular assembly of lipids including, but not limited to, phospholipids. The interior contents of a phospholipid vesicle are separated from the exterior surroundings by at least one phospholipid bilayer. A phospholipid bilayer is a sheet of lipids two molecules thick, arranged so that the hydrophilic phosphate heads point "out" to the solution on either side of the bilayer and the hydrophobic tails point "in" to the core of the bilayer. This results in two "leaflets" which are each a single molecular layer of phospholipids. A "unilamellar vesicle" refers to a vesicle comprising only one phospholipid bilayer; "multilamellar vesicle" refers to a vesicle comprising more than one phospholipid bilayer. A "symmetric bilayer" is defined as a bilayer possessing two leaflets of the same composition, whereas an "asymmetric bilayer" is defined as a bilayer possessing two leaflets which differ in composition.

The vesicle of the present invention can have a unilamellar bilayer including a lipid and a component integrated by the bilayer. The integrated component can be a protein or a fragment thereof, a peptide, a polypeptide, an enzyme or a fragment thereof. Exemplary proteins are ATPase, integrins, alpha-haemolisin. An exemplary polypeptide is the antibiotic gramicidin.

A "water-based droplet" is a droplet, which contains water or a dispersion of any substance in water. Also, a water-based droplet is a droplet, which consists of water or of a dispersion of any substance in water. More specifically, a water-based droplet in the sense of the present invention is a droplet which is composed of water including salts and the at least one lipid.

In accordance with the present invention, a "polymer shell stabilized unilamellar vesicle" comprises a polymer shell surrounding a "water based droplet" and forming a so-called "water-in-oil droplet" or "water-in-oil emulsion". Preferably, the droplet is at least substantially ellipsoidal or at least substantially spherical. More preferably, the parent polymer shell stabilized giant unilamellar vesicle provided in step a) has an outer diameter of at least 10 µm, preferably at least 15 µm, preferably at least 20 µm, preferably at least 30 µm, preferably at least 40 µm, preferably at least 80 µm.

In the present invention, the term "water in oil droplet" is used as synonym of "water in oil emulsion", and "polymer shell stabilized unilamellar vesicle".

"Giant unilamellar vesicles" are, according to the present invention, unilamellar vesicles, are preferably spherical, and with a diameter comprised between 1 µm and 100 µm. The splitted giant unilamellar vesicles formed with the disclosed method, comprising releasing from the polymer shell, have an outer diameter smaller than 1 µm, preferably smaller than 2 µm, preferably smaller than 3 µm, preferably smaller than 4 µm, preferably smaller than 5 µm, preferably smaller than 6 µm, preferably smaller than 7 µm, preferably smaller than 8 µm, preferably smaller than 9 µm, preferably smaller than 10 µm.

Slightly reworded, the splitted giant unilamellar vesicles of the present invention produced after mechanical splitting and release are preferably from 1 µm to 20 µm in diameter. In some embodiments, the splitted giant unilamellar vesicles can be from 2 µm to 20 µm in diameter. In other embodiments, the splitted giant unilamellar vesicles can be from 3 µm to 20 µm in diameter. In other embodiments, the splitted giant unilamellar vesicles can be from 4 µm to 20 µm in diameter. In other embodiments, the splitted giant unilamellar vesicles can be from 5 µm to 20 µm in diameter. In a preferred embodiment, the splitted giant unilamellar vesicles of the present invention can be from 1 µm to 10 µm in diameter, preferably from 2 µm to 10 µm in diameter, preferably from 3 µm to 10 µm in diameter, preferably from 4 µm to 10 µm in diameter, preferably from 5 µm to 10 µm in diameter. In a further embodiment, the splitted giant unilamellar vesicles of the present invention can be from 1 µm to 15 µm in diameter, preferably from 2 µm to 15 µm in diameter, preferably from 3 µm to 15 µm in diameter, preferably from 4 µm to 15 µm in diameter, preferably from 5 µm to 15 µm in diameter. In a more preferred embodiment, the splitted giant unilamellar vesicles of the present invention can be from 1 µm to 5 µm in diameter, preferably from 2 µm to 5 µm in diameter.

The polymer shell stabilized splitted giant unilamellar vesicles formed with the disclosed method have an outer diameter smaller than 1.5 µm, preferably smaller than 2 µm, preferably smaller than 3 µm, preferably smaller than 4 µm, preferably smaller than 5 µm, preferably smaller than 6 µm, preferably smaller than 7 µm, preferably smaller than 8 µm, preferably smaller than 9 µm, preferably smaller than 10 µm.

Slightly reworded, the polymer shell stabilized giant unilamellar vesicles of the present invention produced after mechanical splitting are preferably from 1.5 µm to 23 µm in diameter. In some embodiments, the polymer shell stabilized giant unilamellar vesicles can be from 2 µm to 23 µm in diameter. In other embodiments, the polymer shell stabilized giant unilamellar vesicles can be from 3 µm to 23 µm in diameter. In other embodiments, the polymer shell stabilized giant unilamellar vesicles can be from 4 µm to 23 µm in diameter. In other embodiments, the polymer shell stabilized giant unilamellar vesicles can be from 5 µm to 23 µm in diameter. In a preferred embodiment, the polymer shell stabilized giant unilamellar vesicles of the present invention can be from 1.5 µm to 12 µm in diameter, preferably from 2 µm to 12 µm in diameter, preferably from 3 µm to 12 µm in diameter, preferably from 4 µm to 12 µm in diameter, preferably from 5 µm to 12 µm in diameter. In a further embodiment, the polymer shell stabilized giant unilamellar vesicles of the present invention can be from 1.5 µm to 16 µm in diameter, preferably from 2 µm to 16 µm in diameter, preferably from 3 µm to 16 µm in diameter, preferably from 4 µm to 16 µm in diameter, preferably from 5 µm to 16 µm in diameter. In a more preferred embodiment, the polymer shell stabilized giant unilamellar vesicles of the present invention can be from 1.5 µm to 6 µm in diameter, preferably from 2.5 µm to 6 µm in diameter.

According to a particular preferred embodiment of the present invention, in steps a)-c) is provided a dispersion, in which the droplets (polymer shell stabilized vesicles) are dispersed in an oil-phase. In this embodiment, the giant unilamellar vesicle is not only stabilized by the polymer shell, but also by the outer oil phase so that the chemical as well as mechanical stability of the polymer shell-stabilized giant unilamellar vesicle is drastically improved in comparison to the respective giant unilamellar vesicle without the polymer shell.

In a preferred embodiment, during step d) the polymer shell is removed from the giant unilamellar vesicle, which is then transferred from the oil to the water phase.

The terms "protein" and "peptide" are used interchangeably herein to refer to a polymer of amino acid residues. These terms apply to naturally occurring amino acid polymers and non-naturally occurring amino acid polymers, as well as to amino acid polymers in which one or more amino acid residues are an artificial chemical mimetic of a corresponding naturally occurring amino acid. As used herein, the terms encompass amino acid chains of any length, including full-length proteins, wherein the amino acid residues are linked by covalent peptide bonds, and fragments thereof.

The terms "nucleic acid", "oligonucleotide," and "polynucleotide" refer to deoxyribonucleic acids (DNA) or ribonucleic acids (RNA) and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides.

The terms "production buffer" or "intraluminal buffer" refers to an aqueous solution used to disperse the lipids, and optionally nucleic acid molecules or other cargos to be encapsulated in the giant unilamellar vesicles. As this solution is then incorporated in the lumen of the formed giant unilamellar vesicles, it is also named "intraluminal buffer". Preferred production buffers are PBS, water, and a suitable cell culture medium such as DMEM.

The production buffer comprises preferably a water phase comprising at least one lipid and "cations". Preferential "cations" are Mg^{2+,} Ca²⁺. However, the present invention is not limited concerning the type of suitable cations.

The term "release buffer" refers to an aqueous solution added to the dispersion of giant unilamellar vesicles after destabilization, in order to release the giant unilamellar vesicles into an aqueous solution. Preferred release buffers are PBS, water, and a suitable cell culture medium such as DMEM. It is also preferred when the release buffer is the same as the production buffer.

**"Aqueous mixture" or "aqueous phase"** refers to a solution or suspension of lipids or other molecules substantially in water. The aqueous mixtures are immiscible with the oil-based mixtures of the present invention.

"Oil mixture" or "oil phase" refers to a solution or suspension of lipids or other molecules in perfluorated water-immiscible solvents. Exemplary water-immiscible solvents, referred to as "oils," suitable for the preparation of certain lipid membranes and vesicles in the present invention include, but are not limited to, HFE-7000 (1,1,1,2,2,3,3-heptafluoro-3-methoxy-propane), methylnonafluor-n-butylethers, HFE-7100 (mixture of the isomers [methy nonafluoroisobutyl ether and methyl non-afluorobutyl ether] 1,1,1,2,2,3,3,4,4-nonafluoro-4-methoxy-butane and 1,1,1,2,3,3-hexafluoro-3-methoxy-2-(trifluoromethyl)propane), HFE-7200 (mixture of the isomers 1-ethoxy-1,1,2,2,3,3,4,4,4-nonafluorobutane and 1-ethoxy-1,1,2,3,3,3-hexafluoro-2-(trifluoromethyl)propane), HFE-7300 (1,1,1,2,2,3,4,5,5,5-decafluoro-3-methoxy-4-(trifluoromethyl)pentane), and HFE-7500 (3-ethoxy-1,1,1,2,3,4,4,5,5,6,6,6-dodecafluoro-2-(trifluoromethyl)hexane), FC-40, One of skill in the art will appreciate that other solvents are useful as the oil mixture in the present invention.

"Destabilizing agents" are deemulsifier surfactants able to destabilize the structure of the polymer shell surrounding the giant unilamellar vesicles allowing their release in an aqueous release buffer. Exemplary suitable deemulsifier are 1H, 1H-perfluoro-1-pentanol, 1H,1H-perfluoro-1-octanol, 1H,1H,8H-perfluoro-1-octanol, 1H,1H,2H,2H-perfluoro-1-octanol,

The "polymer shell" of the giant unilamellar vesicles is made of an amphiphilic copolymer having a lipophilic end arranged at the outer side and an hydrophilic end arranged at the inner side of the polymer shell. The structure is described in details in the paragraphs above.

The term "monodisperse" or "monodispersed" refers to vesicles of uniform size in a dispersed phase, where the vesicles are present in an unaggregate and discrete state. The dispersed phase according to the invention is preferably an oil phase for the polymer shell stabilized giant unilamellar vesicles.

Vesicles of "uniform size" means that the polymer shell stabilized giant unilamellar vesicles obtained after splitting, as well as the giant unilamellar vesicles released from the polymer shell, show a coefficient of variation in size lower than 16 %, lower than 15 %, lower than 14 %, lower than 13 %, lower than 12 %, lower than 11 %, lower than 10 %.

The term "symmetric division", "symmetrically dividing", "symmetrically splitting" mean that the polymer shell stabilized giant unilamellar vesicles obtained after splitting, as well as the giant unilamellar vesicles released from the polymer shell, have a similar lipid composition, expressed as molar percentage of a reference lipid, as the original lipid mixture used to generate the parent polymer shell stabilized giant unilamellar vesicle.. In other words, the giant unilamellar vesicles obtained according to the inventive method have a lipid composition, expressed as molar percentage of a reference lipid, similar to the lipid composition of the water phase of step a') of the method. A "similar" lipid composition means that the change of the molar percentage between the original water phase and the splitted giant unilamellar vesicles is less than 5%, preferably less than 4%, less than 3%.

The term "symmetric division" also refers to the fact that the splitted giant unilamellar vesicles have similar intraluminal content as the mother or parent giant unilamellar vesicle. The symmetric division of the luminal content can be observed by incorporating in the initial water phase of step a') a fluorescent molecule, which is then encapsulated in the mother or parent polymer shell stabilized giant unilamellar vesicles. Thus, the coefficient of variation of the intraluminal content is calculated on the basis of the coefficient of variation of the fluorescence intensity of the splitted vesicles, calculated as standard deviation / mean fluorescence * 100. The coefficient of variation is preferably lower than 20 %.

The term "loading efficiency" refers to the capacity of the splitted giant unilamellar vesicles to encapsulate the cargo of interest at high efficiency. The "loading efficiency" is calculated as the percent of encapsulated entity relative to the amount taken into the encapsulation process.
Because of high loading efficiencies achieved in the giant unilamellar vesicles and/or by the methods of the present invention, the cargo-to-lipid ratio for the entity entrapped in the giant unilamellar vesicles is over 60 %, over 70 %, over 80%, over 90%, and typically more than 95% of the cargo-to-lipid ratio (the "input" ratio) calculated on the basis of the amount of the cargo and the giant unilamellar vesicle lipid taken into the loading process. Indeed, practically 100% (quantitative) encapsulation is common.
The cargo-to lipid ratio in the giant unilamellar vesicles can be characterized in terms of weight ratio (weight amount of the cargo per weight or molar unit of the giant unilamellar vesicle lipid) or molar ratio (moles of the cargo per weight or molar unit of the giant unilamellar vesicle lipid). The weight ratio of a cargo in the giant unilamellar vesicles of the present invention is typically at least 0.05, 0.1, 0.2, 0.35, 0.5, or at least 0.65 mg of the cargo per 1 mg of lipid. In terms of molar ratio, the cargo-to-lipid ratio according to the present invention is at least from about 0.02, to about 5, preferably at least 0.1 to about 2, and more preferably, from about 0.15 to about 1.5 moles of the cargo per mole of the giant unilamellar vesicle lipid.

"Interface" is defined as the area of contact between two or more entities that possess distinct boundaries. In certain cases, the interface is a small contact area between closely opposed compartments resulting from the exclusion of the surrounding medium.

The term "cell-targeting" refers to vesicles displaying tropism for a particular cell. Cell targeting vesicles are able to selectively interact with the target cells, and to be incorporated in the target cells by endocitosis (negative charged vesicles) or membrane fusion (positive charged vesicles). Cell targeting vesicles can thus selectively deliver drugs to their intended targets while avoiding not specific cells, and the consequent off-target unwanted effects.

Cell-targeting properties are influenced by the particular composition of the giant unilamellar vesicles, and in particular by: content of positive or negative charged lipids; content of PEGylated lipids; conjugation to particular ligand / bioligands such as membrane receptors, antigens, antibodies, glycan, transmembrane proteins, such as integrins, fibronectin, tetraspanins.

### Description of the invention

The present invention is directed to a method for preparation of monodisperse cell-targeting giant unilamellar vesicles comprising the following steps:
a) providing a polymer shell-stabilized giant unilamellar,
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter of smaller than 10 µm,
d) optionally removing the polymer shell from the polymer shell-stabilized giant unilamellar vesicles obtained in step c).

More in particular, the present invention is directed to a method for preparation of monodisperse cell-targeting giant unilamellar vesicles comprising the following steps:
a) providing a polymer shell-stabilized giant unilamellar vesicle having a diameter of at least 40 µm,
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter of smaller than 10 µm,
d) optionally removing the polymer shell from the polymer shell-stabilized giant unilamellar vesicles obtained in step c).

Also more in particular, the present invention is directed to a method for preparation of monodisperse cell-targeting giant unilamellar vesicles comprising the following steps:
a) providing a polymer shell-stabilized giant unilamellar vesicle having a diameter of at least 20 µm,
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm,
d) optionally removing the polymer shell from the polymer shell-stabilized giant unilamellar vesicles obtained in step c).

In accordance with one embodiment, the present invention is directed to a method for preparation of monodisperse cell-targeting giant unilamellar vesicles comprising the following steps:
a) providing a polymer shell-stabilized giant unilamellar vesicle having a diameter of at least 10 µm,
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm,
d) optionally removing the polymer shell from the polymer shell-stabilized giant unilamellar vesicles obtained in step c).

In accordance with another embodiment, the present invention is directed to a method for preparation of monodisperse cell-targeting giant unilamellar vesicles comprising the following steps:
a) providing a polymer shell-stabilized giant unilamellar vesicle having a diameter of at least 8 µm,
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm,
d) optionally removing the polymer shell from the polymer shell-stabilized giant unilamellar vesicles obtained in step c).

In accordance with a preferred embodiment, the present invention is directed to a method for preparation of monodisperse cell-targeting giant unilamellar vesicles comprising the following steps:
a) providing a polymer shell-stabilized giant unilamellar vesicle, by the following step:
   a') merging a water phase comprising at least one lipid, and cations, and an oil phase comprising an amphiphilic copolymer,
   a") optionally integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm,
d) optionally removing the polymer shell from the polymer shell-stabilized giant unilamellar vesicles obtained in step c).

In accordance with a more preferred embodiment, the present invention is directed to a method for preparation of monodisperse cell-targeting giant unilamellar vesicles comprising the following steps:
a) providing a polymer shell-stabilized giant unilamellar vesicle having a diameter of at least 10 µm, by the following step:
   a') merging a water phase comprising at least one lipid, and cations, and an oil phase comprising an amphiphilic copolymer,
   a") optionally integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm,
d) optionally removing the polymer shell from the polymer shell-stabilized giant unilamellar vesicles obtained in step c).

### Release of the splitted giant unilamellar vesicles from the polymer shell.

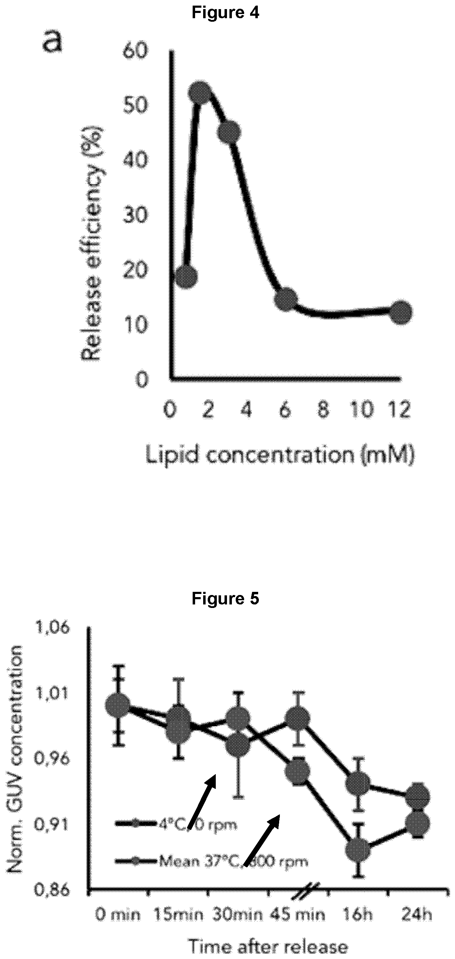

The inventors have found that the giant unilamellar vesicles can be efficiently released from the polymer shell by adding a deemulsifier to the collected polymer shell stabilized giant unilamellar vesicles after mechanical splitting. The deemulsifier destabilizes the structure of the surrounding polymer shell and thus, allows releasing the giant unilamellar vesicles from the polymer shell into an aqueous buffer, also named "release buffer".
This procedure allowed to release the giant unilamellar vesicles from the polymer shell at high efficiency up to 50%, meaning that one every second giant unilamellar vesicle is released into the aqueous phase (Figure 4), reaching a production rare of approximatively 4x10⁶ giant unilamellar vesicles/min.

The deemulsifier is preferably selected from the group comprising1H,1H,2H,2H-perfluoro-1-octanol, 1H,1H-perfluoro-1-pentanol, 1H,1H-perfluoro-1-octanol, 1H,1H,8H-perfluoro-1-octanol. Moreover, the deemulsifier is preferably a short chain perfluorated aliphatic carbon chain.

The deemulsifier is preferentially added at a ratio ranging from 1 : 1 to 10 : 1 with the intraluminalbuffer (also named production buffer).

Therefore, in accordance with one embodiment, the present invention is directed to a method for preparation of monodisperse cell-targeting giant unilamellar vesicles comprising the following steps:
a) providing a polymer shell-stabilized giant unilamellar vesicle;
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) optionally removing the polymer shell from the polymer shell-stabilized giant unilamellar vesicles obtained in step c) by adding a deemulsifier.

Thus, the present invention is also directed to a method for preparation of monodisperse cell-targeting giant unilamellar vesicles comprising the following steps:
a) providing a polymer shell-stabilized giant unilamellar vesicle, by the following step:
   a') merging a water phase comprising at least one lipid, cations and an oil phase comprising an amphiphilic copolymer,
   a") optionally integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm,
d) optionally removing the polymer shell from the polymer shell-stabilized giant unilamellar vesicles obtained in step c) by adding a deemulsifier.

In accordance with a preferred embodiment, the present invention is directed to a method for preparation of monodisperse cell-targeting giant unilamellar vesicles comprising the following steps:
a) providing a polymer shell-stabilized giant unilamellar vesicle;
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm,
d) optionally removing the polymer shell from the polymer shell-stabilized giant unilamellar vesicles obtained in step c) by adding a deemulsifier selected from the group comprising 1H,1H,2H,2H-perfluoro-1-octanol, 1H,1H-perfluoro-1-pentanol, 1H,1H-perfluoro-1-octanol, 1H,1H,8H-perfluoro-1-octanol.

Thus, the present invention is also directed to a method for preparation of monodisperse cell-targeting giant unilamellar vesicles comprising the following steps:
a) providing a polymer shell-stabilized giant unilamellar vesicle, by the following step:
   a') merging a water phase comprising at least one lipid, and cations, and an oil phase comprising an amphiphilic copolymer,
   a") optionally integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm,
d) optionally removing the polymer shell from the polymer shell-stabilized giant unilamellar vesicles obtained in step c) by adding a deemulsifier selected from the group comprising 1H,1H,2H,2H-perfluoro-1-octanol, 1H,1H-perfluoro-1-pentanol, 1H,1H-perfluoro-1-octanol, 1H,1H,8H-perfluoro-1-octanol.

More in particular, the present invention is also directed to a method for preparation of monodisperse cell-targeting giant unilamellar vesicles comprising the following steps:
a) providing a polymer shell-stabilized giant unilamellar vesicle having a diameter of at least 10 µm,
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm,
d) optionally removing the polymer shell from the polymer shell-stabilized giant unilamellar vesicles obtained in step c) by adding a deemulsifier selected from the group comprising 1H,1H,2H,2H-perfluoro-1-octanol, 1H,1H-perfluoro-1-pentanol, 1H,1H-perfluoro-1-octanol, 1H,1H,8H-perfluoro-1-octanol.

Thus, in accordance with optional step d), the polymer shell and the oil phase are removed from the polymer shell-stabilized giant unilamellar vesicle. Since the polymer shell is not necessary any more after the incorporation of the at least one agent and of the one or more proteins or fragments thereof into the polymer shell-stabilized giant unilamellar vesicle and after the mechanical splitting, which represent steps requiring the mechanical stability effected by the polymer shell, it is actually preferred to perform the step d) so as to obtain the giant unilamellar vesicles into an aqueous phase.
The removal step performed by adding a deemulsifier as described in the Method section and Example 1.

The polymer shell and the oil phase may also be removed from the polymer shell-stabilized giant unilamellar vesicle during step d) by other techniques, such as that using a microfluidic device as described in the international patent application WO2018228894A1, Example 5.

### PURIFICATION OF THE SPLITTED GIANT UNILAMELLAR VESICLES

The splitted giant unilamellar vesicles are usually centrifuged after release from the polymer shell to allow purification from vesicles of unwanted dimensions and other impurities.

Thus, an embodiment of the present invention is directed to a method for preparation of monodisperse cell-targeting giant unilamellar vesicles comprising the following steps:
a) providing a polymer shell-stabilized giant unilamellar,
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter of smaller than 10 µm,
d) optionally removing the polymer shell from the polymer shell-stabilized giant unilamellar vesicles obtained in step c),
e) purifying the giant unilamellar vesicles by centrifugation.

In accordance with a preferred embodiment, the present invention is directed to a method for preparation of monodisperse cell-targeting giant unilamellar vesicles comprising the following steps:
a) providing a polymer shell-stabilized giant unilamellar vesicle, by the following step:
   a') merging a water phase comprising at least one lipid, and cations, and an oil phase comprising an amphiphilic copolymer,
   a") optionally integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm,
d) optionally removing the polymer shell from the polymer shell-stabilized giant unilamellar vesicles obtained in step c),
e) purifying the giant unilamellar vesicles by centrifugation.

In accordance with a more preferred embodiment, the present invention is directed to a method for preparation of monodisperse cell-targeting giant unilamellar vesicles comprising the following steps:
a) providing a polymer shell-stabilized giant unilamellar vesicle having a diameter of at least 10 µm, by the following step:
   a') merging a water phase comprising at least one lipid, and cations, and an oil phase comprising an amphiphilic copolymer,
   a") optionally integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm,
d) optionally removing the polymer shell from the polymer shell-stabilized giant unilamellar vesicles obtained in step c),
e) purifying the giant unilamellar vesicles by centrifugation.

The centrifugation can be performed for a time comprised between 5 - 60 min and at acceleration comprised between 800 g - 100.000 g depending on the dimension of the splitted giant unilamellar vesicles of interest. For giant unilamellar vesicles with diameter comprised between 1 - 3 µm, the centrifugation is preferentially performed at acceleration comprised between 10,000 - 30,000 g and a time comprised between 10 - 60 min.
For giant unilamellar vesicles with diameter comprised between 3 - 6 µm, the centrifugation is preferentially performed at acceleration comprised between 5,000 - 20,000 g and a time comprised between 10 - 60 min.
For giant unilamellar vesicles with diameter comprised between 6 - 10 µm, the centrifugation is preferentially performed at acceleration comprised between 2,000 - 10,000 g and a time comprised between 10 - 60min.

### LIPID COMPOSITION OF THE GIANT UNILAMELLAR VESICLES

The giant unilamellar vesicles can comprise one or more lipids. Preferably, the inventive giant unilamellar vesicles comprise at least two lipids, preferably at least three lipids, more preferably at least four lipids. The lipids may be isolated from a naturally occurring source or they may be synthesized apart from any naturally occurring source.

The present invention is not particularly limited concerning the chemical nature of the at least one lipid contained in the water phase of step a') and thus in the inner space of the polymer shell stabilized giant unilamellar vesicle, as long as it is able to form a lipid bilayer. Good results are in particular achieved with phospholipids and in particular with a lipid being selected from the group comprising phosphocholine, phosphocholine derivatives, phosphoethanolamine, phosphoethanolamine derivatives, phosphatidylcholine, phosphatidylcholine derivatives, phosphatidylglycerol, phosphatidylglycerol derivatives and arbitrary combinations of two or more of the aforementioned lipids.

At least one of the lipids is an amphiphilic lipid, defined as having a hydrophilic and a hydrophobic portion, typically a hydrophilic head and a hydrophobic tail. The hydrophobic portion typically orients into a hydrophobic phase, e.g., within the bilayer, while the hydrophilic portion typically orients toward the aqueous phase, e.g., outside the bilayer, and possibly between adjacent apposed bilayer surfaces. The hydrophilic portion may comprise polar or charged groups such as carbohydrates, phosphate, carboxylic, sulfato, amino, sulfhydryl, nitro, hydroxy and other like groups. The hydrophobic portion may comprise apolar groups that include without limitation long chain saturated and unsaturated aliphatic hydrocarbon groups and groups substituted by one or more aromatic, cycloaliphatic or heterocyclic groups. Examples of amphipathic lipids include, but are not limited to, phospholipids, aminolipids and sphingolipids.

Typically, the lipids are phospholipids. Phospholipids include without limitation phosphatidylcholine, phosphatidylethanolamine, phosphatidylglycerol, phosphatidylinositol, phosphatidylserine, and their derivatives. It is to be understood that other lipid membrane components, such as cholesterol, sphingomyelin, cardiolipin, etc. may be used.

Lipids can be "uncharged lipids" or "charged lipids." "Uncharged lipids" refer to lipids that do not carry any charged or ionizable groups such as phosphate groups or choline groups. Examples of uncharged lipids include, but are not limited to, diacyl glycerols and prostaglandins.

"Charged lipids" include neutrally charged, i.e. zwitterionic lipids, cationic lipids and anionic lipids. Generally, lipids bearing a net positive or negative charge exhibit poor solubility in oil phases.

Neutral lipids exist in an uncharged or neutral zwitterionic form at a selected pH.

"Zwitterionic lipids" carry both positively-charged groups and ionizable groups such as amino groups and choline groups that bear a net positive charge, and negatively-charged groups and ionizable groups, such as phosphates, sulfates and carboxylates. Examples of zwitterionic lipids include, but are not limited to, phosphorylcholine and phosphorylethanolamine

"Anionic lipids" are lipids negatively charged at physiological pH. "Cationic lipids" are lipids positively charged at physiological pH.

Further suitable lipids are pH sensitive lipids. A "pH-sensitive" lipid refers to a lipid whose ability to form and/or maintain formation of a lipid bilayer depends at least in part on the pH of the surrounding environment. Liposomes and unilamellar vesicles containing such lipids are destabilized under acidic conditions of the endocytotic pathway. Therefore, the encapsulated content is delivered into the intracellular bio-environment through destabilization or its fusion with the endosomal membrane.

Specific examples of the lipids suitable to synthetize the giant unilamellar vesicles according to the method disclosed herein are listed in Table 1.

Preferably, the lipids are biodegradable in order to allow release of encapsulated agent in vivo and/or in vitro. Biodegradable lipids include but are not limited to 1,2-dioleoyl-sn-glycero-3-phosphocholine (dioleoyl-phosphocholine, DOPC), anionic 1,2-di-(9Z-octadecenoyl)-sn-glycero-3-phospho-(1'-rac-glycerol) (dioleoyl-phosphoglycerol, DOPG), and 1,2-distearoyl-sn-glycero-3-phosphoethanolamine (distearoyl-phosphoethanolamine, DSPE).

### Functionalized Lipids

According to an embodiment of the present invention, the at least one lipid comprised in the water phase of step a') is a lipid functionalized with a functional ligand and/or with polyethylenglycol. Specific examples of the suitable functional moieties, the reacting ligands, and of the functionalized lipids containing are listed in Table 2.

**Table 1: Suitable lipids**

| **Charge** | **Class** | **Specific example** | **Abbreviation** |
|---|---|---|---|
| **Neutral lipids** | ceramide | | Cer |
| | sphingomyelin | Egg sphingomyelin, brain sphingomyelin, Milk sphingomyelin, Lyso sphingomyelin, | SM |
| | cholesterol | | Chol |
| | cerebrosides | Galactocerebroside, Glucocerebroside | Gal-Cer, Glc-Cer |
| | diacylglycerols | 1-oleoyl-2-acetyl-sn-glycerol | DAG, DG |
| | phosphatidylcholines | egg L-α-phosphatidylcholine | EggPC |
| | | distearoylphosphatidylcholine | DSPC |
| | | 1-palmitoyl-2-oleoyl-sn-glycero-3-phosphocholine | POPC |
| | | 1,2-dimyristoyl-sn-glycero-3-phosphocholine | DMPC |
| | | 1,2-dipalmitoyl-sn-glycero-3-phosphocholine | DPPC |
| | | 1,2-dioleoyl-sn-glycero-3-phosphocholine | 18:1 DOPC |
| | | dioleoylphosphatidylglycerol | DOPG |
| | | dipalmitoylphosphatidylglycerol | DPPG |
| | | palmitoyloleyolphosphatidylglycerol | POPG |
| | lysophosphatidylcholines | 1-palmitoyl-sn-glycero-3-phosphocholine | PC(16:0/0:0) |
| | phosphatidylethanolamines (also named "cephalin") | 1-stearoyl-2-oleoyl-sn-glycero-3-phosphoethanolamine | SOPE, 18:0-18:1 PE |
| | | 1,2-dimyristoyl-sn-glycero-3-phosphoethanolamine | DMPE, 14:0 PE |
| | | 1,2-dipalmitoyl-sn-glycero-3-phosphoethanolamine | DPPE |
| | | 1,2-dioleoyl-sn-glycero-3-phosphoethanolamine | 18:1 DOPE |
| | lysophosphatidylethanolamine | 1,2-Distearoyl-sn-glycero-3-phosphoethanolamine | DSPE |
| | | palm itoyloleoyl-phosphatidylethanolamine | POPE |
| | lysoethanolamines | 1-stearoyl-sn-glycero-3-phosphoethanolamine | 18:0 Lyso PE, egg Lyso PE |
| | Inverted Headgroups | 2-((2,3-bis(oleoyloxy)propyl)dimethylammonio)ethyl hydrogen phosphate | DOCP |
| | | 2-((2,3-bis(oleoyloxy)propyl)dimethylammonio)ethyl ethyl phosphate | DOCPe |
| | Sphingosin | (R,E)-2-aminooctadec-4-en-1-ol | 3-deoxy sphingosine |
| | | (2S,3S,4E)-2-aminooctadec-4-ene-1,3-diol | L-threo-sphingosine (d18:1) |
| | | D-erythro-sphingosine | Sphingosine (d18:1) |
| | | D-erythro-sphingosine (C17 base) | Sphingosine (d17:1) |
| | | D-erythro-sphingosine (C20 base) | Sphingosine (d20:1) |
| | | D-erythro-Sphingosine (C22 base) | Sphingosine (d22:1) |
| | | (2S,3R,4E,14Z)-2-aminooctadec-4,14-diene-1,3-diol | 4E,14Z-Sphingadiene |
| | | (2S,3R,4E,8Z)-2-aminooctadec-4,8-diene-1,3-diol | 4E,8Z-Sphingadiene |
| | | (2S,3R,4E,11Z)-2-aminooctadec-4,11-diene-1,3-diol | 4E,11Z-Sphingadiene |
| | | D-erythro-Sphingosine (C16 base) | Sphingosine (d16:1) |
| | | D-erythro-Sphingosine (C14 Base) | Sphingosine (d14:1) |
| | | Mito-Caged Sphingosine | Mito-So |
| | Sterol-modified phospholipids | 1-palmitoyl-2-cholesterylhemisuccinoyl-sn-glycero-3-phosphocholine | PChemsPC |
| | | 1-oleoyl-2-cholesterylhemisuccinoyl-sn-glycero-3-phosphocholine | OChems PC |
| | | 1-palmitoyl-2-cholesterylcarbonoyl-sn-glycero-3-phosphocholine, | PChcPC |
| | | 1,2-dicholesterylhemisuccinoyl-sn-glycero-3-phosphocholine, | DChemsPC |
| | Ether ester lipids | 1-O-heptadecyl-2-acetyl-sn-glycero-3-phosphocholine, | C17 PAF, C17-02:0 PC (Phosphocholine), |
| | | 1-O-hexadecyl-2-acetyl-sn-glycero-3-phosphocholine, | C16-02:0 PC, |
| | | 1-O-hexadecyl-2-oleoyl-sn-glycero-3-phosphocholine, | C16-18:1 PC, |
| | | 1-O-hexadecyl-2-arachidonoyl-sn-glycero-3-phosphocholine, | C16-20:4 PC, |
| | | 1-O-octadecyl-2-acetyl-sn-glycero-3-phosphocholine, | C18-02:0 PC, |
| | | 1-O-hexadecyl-2-butyryl-sn-glycero-3-phosphocholine, | C16-04:0 PC, |
| | | 1-O-octadecyl-2-butyryl-sn-glycero-3-phosphocholine, | C18-04:0 PC, |
| | | 1-O-hexadecyl-2-(8Z,11Z,14Z-eicosatrienoyl)-sn-glycero-3-phosphocholine, | C16-20:3 PC, |
| | | 1-O-hexadecyl-2-(5Z,8Z,11Z,14Z,17Z-eicosapentaenoyl)-sn-glycero-3-phosphocholine, | C16-20:5 PC, |
| | | 1-O-hexadecyl-2-docosahexaenoyl-sn-glycero-3-phosphocholine | C16-22:6 PC, |
| | | 1-hexadecyl-2-(9Z-octadecenoyl)-sn-glycero-3-phosphoethanolamine | C16-18:1 PE |
| | Diether Lipids | 1-O-hexadecanyl-2-O-(9Z-octadecenyl)-sn-glycero-3-phospho-(1'-rac-glycerol) (ammonium salt) | 16:0-18:1 Diether PG, |
| | | 1-O-hexadecanyl-2-O-(9Z-octadecenyl)-sn-glycero-3-phosphoethanolamine | 16:0-18:1 Diether PE, |
| | | 1-O-hexadecanyl-2-O-(9Z-octadecenyl)-sn-glycero-3-phosphocholine | 16:0-18:1 Diether PC, |
| | | 1,2-di-O-(9Z-octadecenyl)-sn-glycero-3-phosphocholine | 18:1 Diether PC, |
| | | 1,2-di-O-octadecyl-sn-glycero-3-phosphocholine | 18:0 Diether PC, |
| | | 1,2-di-O-hexadecyl-sn-glycero-3-phosphocholine | 16:0 Diether PC, |
| | | 1-O-octadecyl-2-O-methyl-sn-glycero-3-phosphocholine | Edelfosine |
| | Vinyl Ether (Plasmalogen) | 1-(1Z-hexadecenyl)-sn-glycero-3-phosphocholine | C16(Plasm) LPC |
| | | 1-O-1'-(Z)-octadecenyl-2-hydroxy-sn-glycero-3-phosphocholine, | C18(Plasm) LPC |
| | | 1-(1Z-octadecenyl)-2-oleoyl-sn-glycero-3-phosphocholine | C18(Plasm)-18:1 PC |
| | | 1-(1Z-octadecenyl)-2-arachidonoyl-sn-glycero-3-phosphocholine | C18(Plasm)-20:4 PC |
| | | 1-O-1'-(Z)-octadecenyl-2-hydroxy-sn-glycero-3-phosphoethanolamine | C18(Plasm) LPE |
| | | 1-(1Z-octadecenyl)-2-docosahexaenoyl-sn-glycero-3-phosphocholine | C18(Plasm)-22:6 PC |
| | | 1-(1Z-octadecenyl)-2-oleoyl-sn-glycero-3-phosphoethanolamine | C18(Plasm)-18:1 PE |
| | | 1-(1Z-octadecenyl)-2-arachidonoyl-sn-glycero-3-phosphoethanolamine | C18(Plasm)-20:4 PE |
| | | 1-(1Z-octadecenyl)-2-docosahexaenoyl-sn-glycero-3-phosphoethanolamine | C18(Plasm)-22:6 PE |
| | N-Acylglycine | N-palmitoylglycine | PalGly |
| | | N-arachidonoylglycine | NAGly |
| | | N-oleoylglycine | NOGly |
| | Very Long Chain Fatty Acids (VLCFA) | 14Z,17Z,20Z,23Z,26Z,29Z-dotriacontahexaenoic acid | C32:6 fatty acid |
| | Prenols | Coenzyme Q6 (S. cerevisiae) | CoQ6 |
| | | Coenzyme Q8 (E. coli) | CoQ8 |
| | | Dolichol Mixture (13-21) | |
| | | Polyprenol mixture (13-21) | |
| | | Polyprenal mixture (13-21) | |
| | Prostaglandins | Prostaglandin E1 | PGE1 |
| | | Prostaglandin F1α | PGF1α |
| | | Prostaglandin F2α (15 beta epimer) | 15-beta PGF2α |
| | | Prostaglandin F1β | PGF1β |
| | | Prostaglandin F1α(15 beta epimer) | 15beta-PGF1α |
| | | Prostaglandin F1α-d9 | PGF1α-d9 |
| | | Prostaglandin E1-d9 | PGE1-d9 |
| | | Prostaglandin E2 Ethanolamide | PGE2-EA |
| | | Prostaglandin A1 | **PGA1** |
| | | Prostaglandin E2 | **PGE2** |
| | | Prostaglandin F2β | **PGF2β** |
| | | Prostaglandin B1 | PGB1 |
| | | Prostaglandin F2α | PGF2α |
| | | 15-keto Prostaglandin F2α | 15-keto PGF2α |
| | Glycosylated Diacyl Glycerols | 1,2-diacyl-3-O-(α-D-glucopyranosyl)-sn-glycerol (E. coli) | MGlc-DAG |
| | | 1-oleoyl-2-palmitoyl-3-(α-D-galactosyl)-sn-glycerol | BbGL-2 |
| | | 1-palmitoyl-2-oleoyl-3-(β-D-glucosyl)-sn-glycerol | 16:0-18:1 DG glucose |
| | Eicosanoids | 5-Oxo-6E,8Z,11Z,14Z-eicosatetraenoic acid | 5-OxoETE |
| | | 17(S)-hydroxy Docosahexaenoic acid | 17(S)-HDHA |
| | | (±)14(15)-epoxy-5Z,8Z,11Z-eicosatrienoic acid | 14(15) EET |
| | | 15S-hydroxy-5Z,8Z,11Z,13E-eicosatetraenoic acid | 15(S)-HETE |
| | | 15(S)-hydroxy-N-(2-hydroxyethyl)-5Z,8Z,11Z,13E-eicosatetraenamide | 15(S)-HAEA |
| | | 13S-Hydroxy-9Z,11 E-octadecadienoic acid | 13(S)HODE |
| | | 13S-Hydroxy-N-(2-hydroxyethyl)-9Z,11 E-octadecadienamide | 13(S)HODE Ethanolamide |
| | Palmitic Acid-Hydroxy Stearic Acid, PAHSA | 9-(palmitoyloxy)octadecanoic acid | 9-PAHSA |
| | | 5-(palmitoyloxy)octadecanoic acid | 5-PAHSA |
| | | 9'-(palmitoyloxy)octadecanoic acid | 12-PAHSA |
| | | 1-palmitoyl-2-[9'-(palmitoyloxy)octadecanoyl]-sn-glycero-3-phosphocholine | 16:0-(12-PAHSA) PC |
| **Anionic lipids** | phosphatidic acids | 1-palmitoyl-2-oleoyl-sn-glycero-3-phosphate | 16:0-18:1 PA, POPA |
| | lysophosphatidic acids | 1-oleoyl-2-hydroxy-sn-glycero-3-phosphate | 18:1 Lyso PA |
| | | 1-stearoyl-2-hydroxy-sn-glycero-3-phosphate | 18:0 Lyso PA |
| | | 1-heptadecanoyl-2-hydroxy-sn-glycero-3-phosphate (sodium salt) | 17:0 Lyso PA |
| | | 1-arachidonoyl-2-hydroxy-sn-glycero-3-phosphate | 20:4 Lyso PA |
| | | 1-palmitoyl-2-hydroxy-sn-glycero-3-phosphate (sodium salt) | 16:0 Lyso PA |
| | | 1-myristoyl-2-hydroxy-sn-glycero-3-phosphate (sodium salt) | 14:0 Lyso PA |
| | phosphatidylglycerols | Egg L-α-phosphatidylglycerol | EggPG |
| | | 1,2-di-(9Z-octadecenoyl)-sn-glycero-3-phospho-(1'-rac-glycerol), or L-α-Phosphatidyl-DL-glycerol | 18:1 DOPG |
| | | 1-palmitoyl-2-oleoyl-sn-glycero-3-phospho-(1'-rac-glycerol) | POPG |
| | lysophosphatidylglycerols | 1-palmitoyl-2-hydroxy-sn-glycero-3-phospho-(1'-rac-glycerol) | 16:0 Lyso PG |
| | phosphatidylserines | 1,2-dioleoyl-sn-glycero-3-phospho-L-serine | DOPS |
| | | 1-stearoyl-2-oleoyl-sn-glycero-3-phospho-L-serine | SOPS |
| | lysophosphatidylserines | 1-stearoyl-sn-glycero-3-phospho-L-serine | PS (18:1/18:0) |
| | phosphatidylinositols | 1-stearoyl-2-arachidonoyl-sn-glycero-3-phospho-(1'-myo-inositol) | 18:0/20:4-PI |
| | phosphatidylinositolphosphates | 1-stearoyl-2-arachidonoyl-sn-glycero-3-phospho-(1'-myo-inositol-4'phosphate) | 18:0-20:4 PI(4)P |
| | | 1-stearoyl-2-arachidonoyl-sn-glycero-3-phospho-(1'-myo-inositol-4',5'-bisphosphate) | 18:0-20:4 PI(4,5)P2 |
| | | phosphatidylinositol 4,5-bisphosphate | PIP2 |
| | cardiolipins | 1',3'-bis[1,2-dilinoleoyl-sn-glycero-3-phospho]-sn-glycerol. | 18:1 Cardiolipin |
| | | 1',3'-bis[1,2-dimyristoleoyl-sn-glycero-3-phospho]-glycerol | 14:1 Cardiolipin |
| | | 1',3'-bis[1,2-dipalmitoyl-sn-glycero-3-phospho]-glycerol | 16:0 Cardiolipin |
| | | 1',3'-bis[1-Palmitoyl-2-oleoyl-sn-glycero-3-phospho]-glycerol | 16:0-18:1 Cardiolipin |
| | | 1',3'-bis[1,2-dipalmitoleoyl-sn-glycero-3-phospho]-glycerol | 16:1 Cardiolipin |
| | | 1',3'-bis[1,2-Distearoyl-sn-glycero-3-phospho]-glycerol | 18:0 Cardiolipin |
| | Bis(Monoacylglycero)Phosphate (BMP) | bis(monomyristoylglycero)phosphate (S,R Isomer) (ammonium salt), | 14:0 BMP (S,R) |
| | | sn-(3-myristoyl-2-hydroxy)-glycerol-1-phospho-sn-3'-(1',2'-dimyristoyl)-glycerol (ammonium salt) | 14:0 Hemi BMP (S,R) |
| | | bis(monooleoylglycero)phosphate (S,R Isomer) (ammonium salt) | 18:1 BMP (S,R) |
| | | sn-(3-oleoyl-2-hydroxy)-glycerol-1-phospho-sn-3'-(1',2'-dioleoyl)-glycerol (ammonium salt) | 18:1 Hemi BMP (S,R) |
| | | sn-(3-oleoyl-2-hydroxy)-glycerol-1-phospho-sn-1'-(3'-oleoyl-2'-hydroxy)-glycerol (ammonium salt) | 18:1 BMP (S,S) |
| | | sn-(1-oleoyl-2-hydroxy)-glycerol-3-phospho-sn-3'-(1'-oleoyl-2'-hydroxy)-glycerol (ammonium salt) | 18:1 BMP (R,R) |
| | | sn-[2,3-dioleoyl]-glycerol-1-phospho-sn-1'-[2',3'-dioleoyl]-glycerol (ammonium salt) | 18:1 BDP (S,S) |
| Cationic lipids | | 2,3-dioleyloxy-N-[2(sperminecarboxamido)ethyl]-N,N-dimethyl-1-propanaminium trifluoroacetate | DOSPA |
| | | 1,2-dimyristyloxypropyl-3-dimethyl-hydroxy ethyl ammonium bromide | DMRIE |
| | | N-(2,3-dioleyloxy)propyl)-N,N-dimethylammonium chloride | DODMA |
| | | dioctadecylamidoglycyl carboxyspermine | DOGS |
| | | 1,2-dioleoyl-3-dimethylammonium-propane | DODAP |
| | | dioleyl-N,N-dimethylammonium chloride | DODAC |
| | | N-(2,3-dioleyloxy)propyl)-N,N,N-trimethylammonium chloride | DOTMA |
| | | N,N-distearyl-N,N-dimethylammonium bromide | DDAB |
| | | 1,2-dioleoyl-3-trimethylammonium-propane | 18:1 DOTAP |
| | | 3β-(N-(N',N'-dimethylaminoethane)-carbamoyl)cholesterol | DC-Chol |
| **pH sensitive lipids** | | N-(4-carboxybenzyl)-N,N-dimethyl-2,3-bis(oleoyloxy)propan-1-aminium | DOBAQ (cationic) |
| | | 1,2-distearoyl-3-dimethylammonium-propane | DAP (cationic) |
| | | 1,2-dipalmitoyl-sn-glycero-3-succinate | 16:0 DGS |
| | | 1,2-dioleoyl-sn-glycero-3-succinate | 18:1 DGS |
| | | N-palmitoyl homocysteine | PHC |
| **Biodegradable lipids** | | 1,2-dioleoyl-sn-glycero-3-phosphocholine | DOPC |
| | | 1,2-di-(9Z-octadecenoyl)-sn-glycero-3-phospho-(1'-rac-glycerol) | DOPG |
| | | 1,2-distearoyl-sn-glycero-3-phosphoethanolamine | DSPE |
| photoswitchable lipid | | N-[(E)-4-(4-((4-butylphenyl)diazenyl)phenyl)butanoyl]-D-erythro-sphingosine | ACe-1 |
| | | 1-stearoyl-2-[(E)-4-(4-((4-butylphenyl)diazenyl)phenyl)butanoyl]-sn-glycerol | 18:0-PhoDAG |
| | | 1-stearoyl-2-[(E)-4-(4-((4-butylphenyl)diazenyl)phenyl) butanoyl]-sn-glycero-3-phosphocholine | 18:0-azo PC |
| | | N-[(E)-4-(4-((4-butylphenyl)diazenyl)phenyl)butanoyl]-D-erythro-sphingosylphosphorylcholine | Azo SM |
| | | (E)-4-(4-((4-butylphenyl)diazenyl)phenyl)-N-(3-hydroxy-4-methoxybenzyl)butanamide | Trans-AzCA4 |
| | | 4-Butyl-Azo-4:0-Acid-1 | Trans-F AAzo-4 |
| | | 1-(E)-4-(4-((4-butylphenyl)diazenyl)phenyl)butanoyl]-2-hydroxy-sn-glycero-3-phosphate | Azo Lyso PA |

**Table 2: Functional moieties and examples of functionalized lipid**

| Functional Ligand | Example | Reacting moiety /Function |
|---|---|---|
| biotin | 1-oleoyl-2-(12-biotinyl-(aminododecanoyl))-sn-glycero-3-phosphoethanolamine (18:1-12:0 Biotin PE); | Avidin, Streptavidin |
| | 1,2-dipalmitoyl-sn-glycero-3-phosphoethanolamine-N-(biotinyl), 16:0 Biotinyl PE | |
| | 1,2-dioleoyl-sn-glycero-3-phosphoethanolamine-N-(biotinyl), 18:1 Biotinyl PE | |
| | 1,2-dioleoyl-sn-glycero-3-phosphoethanolamine-N-(cap biotinyl), 18:1 Biotinyl Cap PE; | |
| | 1,2-dipalmitoyl-sn-glycero-3-phosphoethanolamine-N-(cap biotinyl), 16:0 Biotinyl Cap PE | |
| N-hydroxysuccinimide ester (NHS), Sulfo-NHS | NHS Palmitic acid N-hydroxysuccinimide ester | Amine |
| nitrilotriacetic acid (NTA)-nickel | 1,2-dioleoyl-sn-glycero-3-[(N-(5-amino-1-carboxypentyl)iminodiacetic acid) succinyl], 18:1 DGS-NTA (Ni) | Histidine (His) tags, e.g. 6 x His-Tag |
| amines | 1,2-dioleoyl-sn-glycero-3-phosphoethanolamine-N-(hexanoylamine),18:1 Caproylamine PE | NHS, sulfo-NHS |
| | 1,2-Dipalmitoyl-sn-Glycero-3-Phosphoethanolamine-N-(hexanoylamine), 16:0 Caproylamine PE | |
| | 1,2-dipalmitoyl-sn-glycero-3-phosphoethanolamine-N-(dodecanylamine), 16:0 Dodecanylamine PE | |
| | 1,2-dioleoyl-sn-glycero-3-phosphoethanolamine-N-(dodecanylamine), 18:1 Dodecanylamine PE | |
| arginylglycylaspartic acid (RGD) | 1,2-distearoyl-sn-glycero-3-phosphoethanolamine-N-[4-(p-(cysarginylglycylaspartate-maleimidomethyl)-cyclohexane-carboxamide], DSPE-RGD | Integrin receptors on target cells |
| maleimides, aromatic maleimides | 1,2-dipalmitoyl-sn-glycero-3-phosphoethanolamine-N-[4-(p-maleimidomethyl) cyclohexane-carboxamide] (sodium salt), 16:0 PE MCC; | Thiol (e.g. thiolated antibodies) |
| N-[4-(p-maleimidophenyl)-butyryl], MPB; 4-(N-maleimidomethyl) cyclohexane-1-carboxyl ate, MCC | 1,2-dioleoyl-sn-glycero-3-phosphoethanolamine-N-[4-(p-maleimidomethyl) cyclohexane-carboxamide] (sodium salt), 18:1 PE MCC; | |
| | 1,2-dioleoyl-sn-glycero-3-phosphocholine (N-aminoethyl), 18:1 aminoethyl PC; | |
| | 1,2-dioleoyl-sn-glycero-3-phosphoethanolamine-N-[4-(p-maleimidophenyl)butyramide] (sodium salt), 18:1 MPB PE | |
| | 1,2-dipalmitoyl-sn-glycero-3-phosphoethanolamine-N-[4-(p-maleimidophenyl) butyramide] (sodium salt), 16:0 MPB PE | |
| pyridyldithiopropionate (PDP) | 1,2-dioleoyl-sn-glycero-3-phosphoethanolamine-N-[3-(2-pyridyldithio)propionate] (sodium salt), 18:1 PDP PE | maleimide-functionalized antibodies bind to sulfhydryl groups obtained after reduction of a PDP-phopholipid |
| | 1 ,2-d i palm itoyl-sn-glycero-3-phosphoethanolam i ne- N-[3-(2-pyridyl dithi o) propionate] (sodium salt), 16:0 PDP PE | |
| pyridyl disulfide (DPS) | | maleimide |
| dithiopyridinyl 4,4'-dithiodipyridine (4-PDS or 4-DTDP), | | maleimide |
| N-benzylguanine | 1,2-dioleoyl-sn-glycero-3-phosphoethanolamine-N-benzylguanine, 18:1 PE-benzylguanine | SNAP-tag |
| | 1,2-dioleoyl-sn-glycero-3-phosphoethanolamine-N-[benzylguanine(polyethylene glycol)-2000], 18:1 PE-PEG2000-benzylguanine | |
| fluorescent dye molecule, such as lissamine rhodamine B sulfonyl, Atto488, Alexa Fluor 488, Alexa Fluor 647, Fluorescein, N-(7-Nitrobenz-2-Oxa-1,3-Diazol-4-yl (NBD), Cy5, Cy5.5, Cy7, Topfluor® Alexa Fluor488, Topfluor® Alexa Fluor594 | 1,2-dioleoyl-sn-glycero-3-phosphoethanolamine-N-(lissamine rhodamine B sulfonyl) (RhB DOPE or LissRhod PE), | |
| | 1,1'-dioctadecyl-3,3,3',3'-tetramethylindotricarbocyanine iodide (DiR); | |
| | 1,1'-dioctadecyl-3,3,3',3'-tetramethylindodicarbocyanine (DiD) | |
| sulfhydryl / thiol group | 1,2-Dipalmitoyl-sn-Glycero-3-Phosphothioethanol (DPPTE) / 16:0 Ptd Thioethanol | Maleimides, Iodoacetamides, benzylic halide, and bromomethylketones, |
| Carboxyacyl such as Succinyl, Glutaryl, dodecanoyl | 1,2-dioleoyl-sn-glycero-3-phosphoethanolamine-N-(succinyl) (sodium salt), 18:1 Succinyl PE; | |
| | 1,2-dipalmitoyl-sn-glycero-3-phosphoethanolamine-N-(succinyl) (sodium salt), 16:0 Succinyl PE; | |
| | 1,2-dioleoyl-sn-glycero-3-phosphoethanolamine-N-(glutaryl) (sodium salt), 18:1 Glutaryl PE; | |
| | 1,2-dipalmitoyl-sn-glycero-3-phosphoethanolamine-N-(glutaryl) (sodium salt), 16:0 Glutaryl PE; | |
| | 1,2-dioleoyl-sn-glycero-3-phosphoethanolamine-N-(dodecanoyl) (sodium salt), 18:1 Dodecanyl PE; | |
| | 1,2-dipalmitoyl-sn-glycero-3-phosphoethanolamine-N-(dodecanoyl) (sodium salt), 16:0 Dodecanoyl PE | |
| cyanuric chloride | cyanur-DSPE | coupling to amine-containing biomolecules such as peptides, antibodies, nanoparticles |
| | cyanur-PEG2000-PE (ammonium salt) | |
| | 1,2-distearoyl-sn-glycero-3-phosphoethanolamine-N-[cyanur(polyethylene glycol)-2000] (ammonium salt), DSPE-PEG(2000) Cyanur | |
| | 1,2-dipalmitoyl-sn-glycero-3-phosphoethanolamine-N-(cyanur), 16:0 Cyanur PE | |
| Folate | 1,2-dipalmitoyl-sn-glycero-3-phosphoethanolamine-N-(6-((folate)amino)hexanoyl), 16:0 Folate Cap PE, | Folate receptor on cancer cells (endocitosis) |
| Carbohydrate/Glycan: for example β-galactose, α-mannose-, β-mannose-, and α-fucose | 1,2-dipalmitoyl-sn-glycero-3-phospho((ethyl-1',2',3'-triazole)triethyleneglycolmannose), 16:0 PA-PEG3-mannose | Carbohydrate binding cell receptor |
| | β -galactose, α-mannose-, β-mannose-, and α-fucose; | |
| | 1,2-dioleoyl-sn-glycero-3-phosphoethanolamine-N-lactosyl (ammonium salt), (18:1 Lactosyl PE) | |
| | 1,2-diacyl-3-O-(α-D-glucopyranosyl)-sn-glycerol (E. coli), MGlc-DAG | |
| | 1-oleoyl-2-palmitoyl-3-(α-D-galactosyl)-sn-glycerol, BbGL-2 | |
| | 1-palmitoyl-2-oleoyl-3-(β-D-glucosyl)-sn-glycerol, 16:0-18:1 DG glucose | |
| Square | 1,2-dioleoyl-sn-glycero-3-phosphoethanolamine-N-square, 18:1 PE-Square | Square is a dye for Resonance Energy Transfer (RET), Flow Cytometry |
| | 1,2-distearoyl-sn-glycero-3-phosphoethanolamine-N-square, 18:0 PE-square | |
| Galloyl | 1,2-dipalmitoyl-sn-glycero-3-galloyl (16:0 DG Galloyl) | Self-adhering lipid so that bilayers strongly adhere to each other |
| Azide | 1,2-dioleoyl-sn-glycero-3-phosphoethanolamine-N-[azido(polyethylene glycol)-2000] (ammonium salt), DOPE-PEG(2000) Azide | Photochemically induced cross-linking with transmembrane peptides |
| Carboxylic acid | 1,2-dioleoyl-sn-glycero-3-phosphoethanolamine-N-[carboxy(polyethylene glycol)-2000] (sodium salt), DOPE-PEG(2000) Carboxylic acid | Amine moieties |
| Chelators: nitrilotriacetic acid (NTA), diethylenetriamine pentaacetic acid, DTPA | 1,2-dipalmitoyl-sn-glycero-3-phosphoethanolamine-N-diethylenetriaminepentaacetic acid (16:0 PE-DTPA), | gadolinium chelated with diethylenentriaminepenta acetyl (DTPA) provides contrast in magnetic resonance imaging |
| | 1,2-dioleoyl-sn-glycero-3-[(N-(5-amino-1-carboxypentyl)iminodiacetic acid)succinyl] (18:1 DGS-NTA), | |
| | 1,2-dioleoyl-sn-glycero-3-[(N-(5-amino-1-carboxypentyl)iminodiaceticacid) succinyl] (Cobalt salt) (18:1 DGS-NTA)(Co), | |
| | 1,2-dioleoyl-sn-glycero-3-[(N-(5-amino-1-carboxypentyl)iminodiacetic acid) succinyl] (nickel salt) (18:1 DGS-NTA)(Ni), | |
| | 1,2-dimyristoyl-sn-glycero-3-phosphoethanolamine-N-diethylenetriaminepenta-acetic acid (copper salt), (14:0 PE-DTPA(Cu)), | |
| | DTPA-bis(stearylamide) (gadolinium salt), (DTPA-BSA (Gd)), | |
| | 1,2-distearoyl-sn-glycero-3-phosphoethanolamine-N-diethylenetriaminepentaacetic acid (gadolinium salt), (18:0 PE-DTPA (Gd)), | |
| | bis(1,2-dimyristoyl-sn-glycero-3-phosphoethanolamine)-N-N'-diethylenetriaminepentaacetic acid (gadolinium salt) (bis(14:0 PE)-DTPA(Gd)), | |
| | bis(1,2-dipalmitoyl-sn-glycero-3-phosphoethanolamine)-N-N'-diethylenetriaminepentaacetic acid (gadolinium salt) (bis(16:0 PE)-DTPA(Gd)), | |
| | bis(1,2-distearoyl-sn-glycero-3-phosphoethanolamine)-N-N'-diethylenetriaminepentaacetic acid (gadolinium salt) (bis(18:0 PE)-DTPA(Gd)), | |
| | 1,2-distearoyl-sn-glycero-3-phosphoethanolamine-N-diethylenetriaminepentaacetic acid (18:0 PE-DTPA). | |
| Mag netic resonance imaging (MRI) imaging | 1,2-dipalmitoyl-sn-glycero-3-phosphoethanolamine-N-diethylenetriami nepentaacetic acid (gadolinium salt), (16:0 PE-DTPA (Gd)), | |
| | 1,2-distearoyl-sn-glycero-3-phosphoethanolamine-N-diethylenetriaminepentaacetic acid (gadolinium salt), (18:0 PE-DTPA (Gd)), | |
| | bis(1,2-dimyristoyl-sn-glycero-3-phosphoethanolamine)-N-N'-diethylenetriaminepentaacetic acid (gadolinium salt) (bis(14:0 PE)-DTPA(Gd)), | |
| | bis(1,2-dipalmitoyl-sn-glycero-3-phosphoethanolamine)-N-N'-diethylenetriaminepentaacetic acid (gadolinium salt) (bis(16:0 PE)-DTPA(Gd)), | |
| | bis(1,2-distearoyl-sn-glycero-3-phosphoethanolamine)-N-N'-diethylenetriaminepentaacetic acid (gadolinium salt) (bis(18:0 PE)-DTPA(Gd)), | |
| | 1,2-distearoyl-sn-glycero-3-phosphoethanolamine-N-diethylenetriaminepentaacetic acid (18:0 PE-DTPA). | |
| polyethylene glycol | 1,2-dipalmitoyl-sn-glycero-3-phosphoethanolamine-N-[methoxy(polyethylene glycol)-350], 18:1 PEG350 PE | Surface passivation |
| PEG200, PEG350, PEG550, PEG750, PEG1000, PEG2000, PEG3000, PEG5000, PEG20000, PEG50000, | 1,2-dioleoyl-sn-glycero-3-phosphoethanolamine-N-[methoxy(polyethylene glycol)-750], 18:1 PEG750 PE | |
| | 1,2-dioleoyl-sn-glycero-3-phosphoethanolamine-N-[methoxy(polyethylene glycol)-1000], 18:1 PEG1000 PE | |
| Diacetylene | 1,2-bis(10,12-tricosadiynoyl)-sn-glycero-3-phosphoethanolamine, 23:2 Diyne PE [DC(8,9)PE] | Photopolymerization |
| | 1-palmitoyl-2-(10,12-tricosadiynoyl)-sn-glycero-3-phosphocholine, 16:0-23:2 Diyne PC | |
| | 1-palmitoyl-2-(10,12-tricosadiynoyl)-sn-glycero-3-phosphoethanolamine, 16:0-23:2 Diyne PE | |
| | 1,2-bis(10,12-tricosadiynoyl)-sn-glycero-3-phosphocholine, 23:2 Diyne PC [DC(8,9)PC] | |
| Diphytanoyl Lipids | 1,2-diphytanoyl-sn-glycero-3-phosphoethanolamine-N-(7-nitro-2-1,3-benzoxadiazol-4-yl), 4ME 16:0 NBD PE (NBD-DPhPE) | Lipids containing diphytanoyl fatty acid chains allow to produce stable planar lipid membranes |
| | 1,2-diphytanoyl-sn-glycero-3-phosphocholine, 4ME 16:0 PC | |
| | 1,2-diphytanoyl-sn-glycero-3-phosphoethanolamine, 4ME 16:0 PE | |
| | 1,2-diphytanoyl-sn-glycero-3-phospho-(1'-rac-glycerol), 4ME 16:0 PG | |
| | 1,2-diphytanoyl-sn-glycero-3-phosphate, 4ME 16:0 PA | |
| | 1,2-diphytanoyl-sn-glycero-3-phospho-L-serine, 4ME 16:0 PS | |
| | phytanoyl Coenzyme A, 4ME 16:0 Coenzyme A | |
| | 1,2-di-O-phytanyl-sn-glycero-3-phosphocholine, 4ME 16:0 Diether PC | |
| | 1,2-di-O-phytanyl-sn-glycero-3-phosphoethanolamine, 4ME 16:0 Diether PE | |
| | 1,2-di-O-phytanyl-sn-glycerol, 4ME 16:0 Diether DG | |
| Fluorinated lipids | 1-palmitoyl-2-(16-fluoropalmitoyl)-sn-glycero-3-phosphocholine, 16:0-16:0 (16-F) PC | |
| Brominated Lipid | 1,2-di-(9,10-dibromo)stearoyl-sn-glycero-3-phosphocholine, 18:0 (9,10dibromo) PC | Fluorescence quenching |
| | 1-palmitoyl-2-stearoyl(4,5)dibromo-sn-glycero-3-phosphocholine, 16:0-18:0(4,5-dibromo) PC | |
| | 1-palmitoyl-2-(6,7-dibromo)stearoyl-sn-glycero-3-phosphocholine, 16:0-18:0 (6-7BR) PC | |
| | 1-palm itoyl-2-(9, 1 0-di bromo)stearoyl-sn-glycero-3-phosphocholine, 16: 0-18:0 (9-10BR) PC | |
| | 1-palmitoyl-2-(11,12-dibromo)stearoyl-sn-glycero-3-phosphocholine, 16:0-18:0 (11-12BR) PC | |

Preferably, the functional ligand is selected from biotin, N-hydroxysuccinimide ester, nitrilotriacetic acid -nickel, amine, carboxylic acid, maleimides, dithiopyridinyl, pyridyl disulfide, pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, and a chelator. Sulfhydryls, also called thiols, exist in proteins in the side-chain of cysteine (Cys, C) amino acids. Sulfhydryl-reactive chemical groups include haloacetyls, maleimides, aziridines, acryloyls, arylating agents, vinylsulfones, pyridyl disulfides, TNB-thiols and disulfide reducing agents.
Different lipids which are offered for thioether conjugation contain maleimide, aromatic maleimides such as N-[4-(p-maleimidophenyl)-butyryl] (MPB) or 4-(N-maleimidomethyl)cyclohexane-1-carboxylate (MCC) group. The maleimide function group of MCC which contains an aliphatic cyclohexane ring is more stable toward hydrolysis in aqueous reaction environments rather than the aromatic phenyl group of MPB.

Carbohydrates are selected from the group comprising β-galactose, α-mannose-, β-mannose-, and α-fucose. It has been shown that said carbohydrates can be conjugated to cholesterols to be incorporated into liposomes, and *in vitro* results showed that the sugar-conjugated liposomes are efficiently recognized by cells that overexpress carbohydrate-binding receptors on their surface (Rajabi and Mousa, 2016, Current Pharmaceutical Biotechnology, 17, 8).

SNAP-tag is a self-labeling protein tag commercially available in various expression vectors. SNAP-tag is a 182 residues polypeptide (19.4 kDa) that can be fused to any protein of interest and further specifically and covalently tagged with a suitable ligand, such as a fluorescent dye.

Functionalized and non-functionalized lipids are available from a number of commercial sources including Avanti Polar Lipids (Alabaster, Alabama).

In some embodiments, the molar percentage (mol %) of a cationic lipid typically comprises from 0% to 10%, from 10% to 20%, from 10% to 30%, from 10% to 40%, %, from 10% to 50%, from 10% to 60%, from 20% to 30%, from 20% to 40%, from 20% to 50%, from 20% to 60% of the total lipid present in vesicle.

In some embodiments, the molar percentage (mol %) of an anionic lipid typically comprises from 0% to 10%, from 10% to 20%, from 10% to 30%, from 10% to 40%, %, from 10% to 50%, from 10% to 60%, from 20% to 30%, from 20% to 40%, from 20% to 50%, from 20% to 60% of the total lipid present in vesicle.

In some embodiments, the molar percentage (mol %) of neutral lipid typically comprises from 49% to 99%, from 49% to 89%, from 49% to 79%, from 49% to 69%, %, from 59% to 99%, from 59% to 89%, from 59% to 79%, from 59% to 69% of the total lipid present in vesicle.

According to a particular aspect, the present invention is directed to a method for preparation of monodisperse cell-targeting giant unilamellar vesicles comprising the following steps:
a) providing a polymer shell-stabilized giant unilamellar vesicle, by the following step:
   a') merging a water phase comprising at least one lipid, and cations, and an oil phase comprising an amphiphilic copolymer,
   a") optionally integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) optionally removing the polymer shell from the polymer shell-stabilized giant unilamellar vesicles obtained in step c),
wherein the at least one lipid of step a') is selected from the group comprising
a neutral lipid selected from the group comprising ceramide, sphingomyelin, cephalin, cholesterol, cerebrosides, diacylglycerols, phosphatidylcholines, lysophosphatidylcholines, phosphatidylethanolamines, lysophosphatidylethanolamine, lysoethanolamines, inverted headgroup lipids, sphingosins, sterol-modified phospholipids, ether ester lipids, diether lipids, vinyl ether (plasmalogen);
an anionic lipid selected from the group comprising phosphatidic acids, lysophosphatidic acid derivatives, phosphatidylglycerols, lysophosphatidylglycerols, phosphatidylserines, lysophosphatidylserines, phosphatidylinositols, phosphatidylinositolphosphates, cardiolipins, Bis(Monoacylglycero)Phosphate derivatives;
a cationic lipid selected from the group comprising dioleyl-N,N-dimethylammonium chloride; N-(2,3-dioleyloxy)propyl)-N,N,N-trimethylammonium chloride; N,N-distearyl-N,N-dimethylammonium bromide; N-(2,3- dioleyloxy)propyl)-N,N,N-trimethylammonium chloride; 3β-(N-(N',N'-dimethylaminoethane)-carbamoyl)cholesterol; 1,2-dimyristyloxypropyl-3-dimethyl-hydroxy ethyl ammonium bromide; 2,3-dioleyloxy-N-[2(sperminecarboxamido)ethyl]-N,N-dimethyl-1-propanaminium trifluoroacetate; dioctadecylamidoglycyl carboxyspermine; N-(2,3-dioleyloxy)propyl)-N,N-dimethylammonium chloride and 1,2-dioleoyl-3-dimethylammonium-propane;
a pH- sensitive lipid selected from the group comprising lipid N-(4-carboxybenzyl)-N,N-dimethyl-2,3-bis(oleoyloxy)propan-1-aminium, 1,2-distearoyl-3-dimethylammonium-propane, 1,2-dipalmitoyl-sn-glycero-3-succinate, 1,2-dioleoyl-sn-glycero-3-succinate, N-palmitoyl homocysteine ;
a photoswitchable lipid;
acylglycine derivatives, prenol derivatives, prostaglandine derivatives, glycosylated diacyl glycerols, eicosanoid derivatives, (palmitoyloxy)octadecanoic acid derivatives, diacetylene derivatives, diphytanoyl derivatives, fluorinated lipids, brominated lipids, lipopolysaccharides;
one of the aforementioned lipids coupled to a functional ligand selected from biotin, N-hydroxysuccinimide ester, nitrilotriacetic acid -nickel, amine, carboxylic acid, maleimides, dithiopyridinyl, pyridyl disulfide, pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, a chelator;
one of the aforementioned lipids coupled to polyethyleneglycol with a molecular weight comprised between 350 and 50,000 g/mol.

### REGULATION OF ATTRACTIVE AND REPULSIVE GUV-CELL INTERACTIONS FOR TARGETED DELIVERY

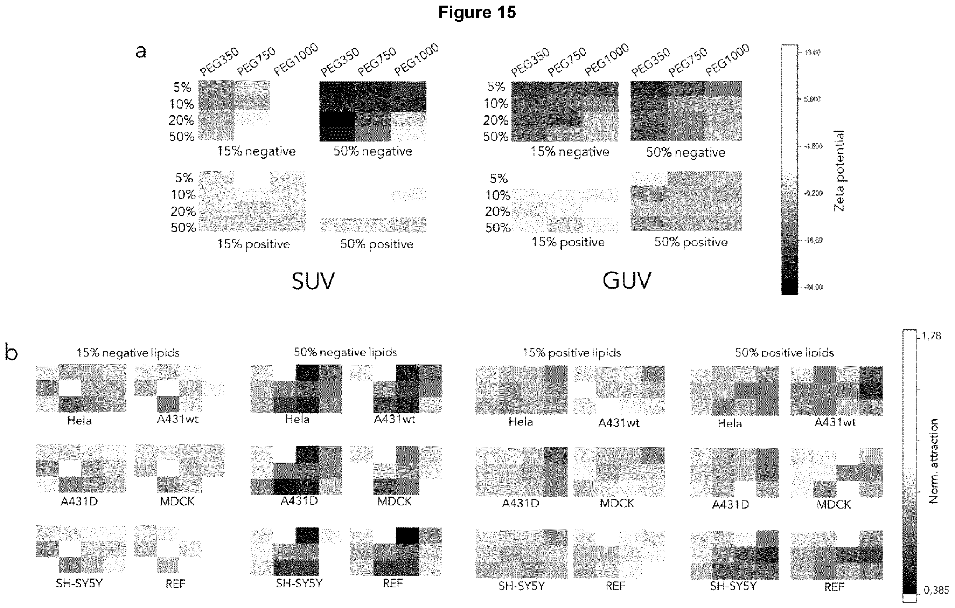

The inventors have here developed a PEG-based passivation strategy with the aim to regulate the specific or unspecific interactions between cells and giant unilamellar vesicles, in order to promote the ligand-based specific attractive interactions, while suppressing charge-mediated unspecific interactions (Figures 15 - 19).

In particular, the inventors found that the Zeta-potential of both, negatively and positively charged vesicles, can be decreased by increasing PEG chain length and rate of PEGylation.

Polyethylene glycol can be conjugated to any lipid and preferentially to a phosphatidylethanolamine or, alternatively, to a ceramide using standard coupling reactions known to and used by those of skill in the art. In addition, preformed polyethylene glycol-phosphatidylethanolamine conjugates are commercially available from Avanti Polar Lipids (Alabaster, Alabama).

Polyethylene glycols of varying molecular weights can be used to form the bilayer stabilizing components of the present invention. Polyethylene glycols of varying number of ethylene glycol units (and therefore molecular weight) are commercially available from a number of different sources or, alternatively, they can be synthesized using standard polymerization techniques well-known to those of skill in the art. Suitable PEG molecules for the scope of the present invention have a molecular weight comprised in the range between 350 - 50,000 g/mole, corresponding to PEG350 - PEG50000. For example, the following PEG molecules can be used: PEG200, PEG350, PEG550, PEG750, PEG1000, PEG2000, PEG3000, PEG5000, PEG20000, PEG50000.

Preferential PEGylated lipids for the methods described in this invention are: 1,2-dipalmitoyl-sn-glycero-3-phosphoethanolamine-N-[methoxy(polyethylene glycol)-350], 18:1 PEG350 PE, 1,2-dioleoyl-sn-glycero-3-phosphoethanolamine-N-[methoxy(polyethylene glycol)-750], 18:1 PEG750 PE, 1,2-dioleoyl-sn-glycero-3-phosphoethanolamine-N-[methoxy(polyethylene glycol)-1000], 18:1 PEG1000 PE.

The giant unilamellar vesicles contain PEGylated lipids at molar concentration up to 5%, preferentially up to 10%, preferentially up to 20%, preferentially up to 50% preferentially up to 60%, preferentially up to 70%, preferentially up to 80%. It is particularly preferred when the PEGylated lipids are present at a molar concentration of 50 %.

Thus, according to a particular aspect, the present invention is directed to a method for preparation of monodisperse cell-targeting giant unilamellar vesicles comprising the following steps:
a) providing a polymer shell-stabilized giant unilamellar vesicle:
   a') merging a water phase comprising at least one lipid, and cations, and an oil phase comprising an amphiphilic copolymer,
   a") optionally integrating one or more proteins or fragments thereof into the polymer shell stabilized giant unilamellar vesicle provided in step a')
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) optionally removing the polymer shell from the polymer shell-stabilized giant unilamellar vesicles obtained in step c),
wherein at least one lipid of step a') is coupled to polyethyleneglycol with a molecular weight comprised between 350 and 50,000 g/mol.

### FINE-TUNING OF CHARGE-MEDIATED GUV - CELL INTERACTIONS

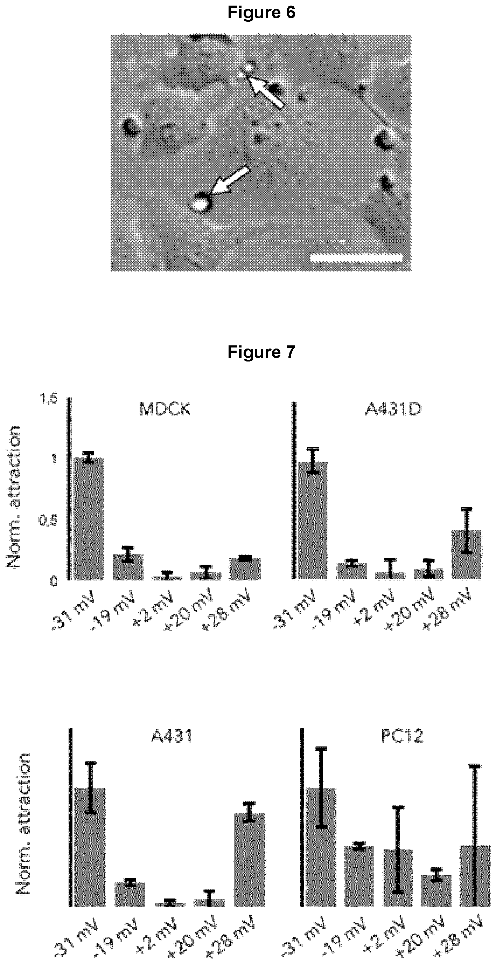

The inventors have found that the charge of the giant unilamellar vesicles can be fine-tuned between highly positive to highly negative by adjusting respective lipid formulations. The production of differently charged giant unilamellar vesicles has the huge technical advantage to allow controlling their interaction with target cells.
The inventors could show that giant unilamellar vesicles comprising varying amounts of a negatively charged lipid such as 1,2-dioleoyl-sn-glycero-3-phospho-rac-(1-glycerol) have a negative Zeta-potential, and are taken up by the cells by endocytosis wherein reside within the cytoplasm (Figures 7, 8, 9).

Therefore, an embodiment of the present invention is directed to a method for preparation of monodisperse cell-targeting giant unilamellar vesicles comprising the following steps:
a) providing a polymer shell-stabilized giant unilamellar vesicle by the following step:
   a') merging a water phase comprising at least one anionic lipid, at least one neutral lipid, and optionally one neutral lipid functionalized with a fluorescent dye molecule, and cations, and an oil phase comprising an amphiphilic copolymer,
   a") optionally integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) optionally removing the polymer shell from the polymer shell-stabilized giant unilamellar vesicles obtained in step c).

The list of suitable anionic lipid and neutral lipid is described in Table 1. Table 2 reports the list of suitable fluorescent dyes.

Therefore, an embodiment of the present invention is directed to a method for preparation of monodisperse cell-targeting giant unilamellar vesicles comprising the following steps:
a) providing a polymer shell-stabilized giant unilamellar vesicle by the following step:
   a') merging a water phase comprising at least one anionic lipid, at least one neutral lipid, and optionally one neutral lipid functionalized with a fluorescent dye molecule, and cations, and an oil phase comprising an amphiphilic copolymer,
   a") optionally integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) optionally removing the polymer shell from the polymer shell-stabilized giant unilamellar vesicles obtained in step c),
wherein the anionic lipid is selected from the group comprising phosphatidic acids, lysophosphatidic acid, phosphatidylglycerols, lysophosphatidylglycerols, phosphatidylserines, lysophosphatidylserines, phosphatidylinositols, phosphatidylinositolphosphates, cardiolipins, bis(Monoacylglycero)Phosphates, and
wherein the neutral lipid is selected from the group comprising ceramide, sphingomyelin, cephalin, cholesterol, cerebrosides, diacylglycerols, phosphatidylcholines, phosphatidylglycerols, lysophosphatidylcholines, phosphatidylethanolamines, and lysoethanolamines, and
wherein the fluorescent dye molecule is selected from the group comprising lissamine rhodamine B sulfonyl, Atto488, Alexa Fluor 488, Alexa Fluor 647, Fluorescein, N-(7-Nitrobenz-2-Oxa-1,3-Diazol-4-yl (NBD), Cy5, Cy5.5, Cy7, Topfluor® Alexa Fluor488, Topfluor® Alexa Fluor594.

In some embodiments, the molar percentage (mol %) of an anionic lipid typically comprises from 0% to 10%, from 10% to 20%, from 10% to 30%, from 10% to 40%, %, from 10% to 50%, from 10% to 60%, from 20% to 30%, from 20% to 40%, from 20% to 50%, from 20% to 60% of the total lipid present in vesicle.

In some embodiments, the molar percentage (mol %) of neutral lipid typically comprises from 49% to 99%, from 49% to 89%, from 49% to 79%, from 49% to 69%, %, from 59% to 99%, from 59% to 89%, from 59% to 79%, from 59% to 69% of the total lipid present in vesicle.

In some embodiments, the molar percentage (mol %) of neutral lipid functionalized with a fluorescent dye molecule typically comprises from 0.1% to 5%, from 0.5% to 5%, from 1% to 5%, from 1% to 4%, %, from 1% to 3% the total lipid present in vesicle.

In particular, good results have been obtained with a molar percentage of the anionic lipid comprised between 20 - 50 %, and of the neutral lipid comprised between 49 - 99 %.

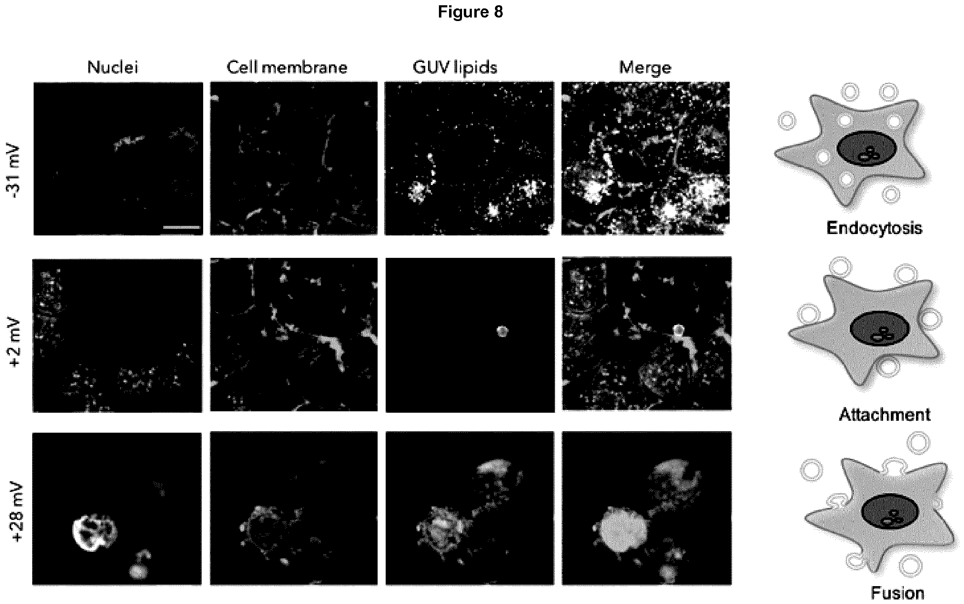

In contrast, giant unilamellar vesicles comprising varying amounts of a positively charged lipid such as 1,2-dioleoyl-3-trimethylammonium-propane (DOTAP) have a positive Zeta-potential and their interactions with the cells depend from the amplitude of their Zeta- potentials; vesicles with a Zeta potential value of about 2mV attach to the membranes of target cells, vesicles with a Zeta potential value higher than 2mV fuse with the membranes of target cells (Figure 7, and 8,).

Therefore, an embodiment of the present invention is directed to a method for preparation of monodisperse cell-targeting giant unilamellar vesicles comprising the following steps:
a) providing a polymer shell-stabilized giant unilamellar vesicle by the following step:
   a') merging a water phase comprising at least one cationic lipid, at least one neutral lipid, and optionally one neutral lipid functionalized with a fluorescent dye molecule, and cations, and an oil phase comprising an amphiphilic copolymer,
   a") optionally integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) optionally removing the polymer shell from the polymer shell-stabilized giant unilamellar vesicles obtained in step c).

The list of suitable cationic lipid and neutral lipid is described in Table 1. Table 2 reports the list of suitable fluorescent dyes.

Thus, a particular embodiment of the present invention is directed to a method for preparation of monodisperse cell-targeting giant unilamellar vesicles comprising the following steps:
a) providing a polymer shell-stabilized giant unilamellar vesicle by the following step:
   a') merging a water phase comprising at least one **cationic** lipid, at least one neutral lipid, and optionally one neutral lipid functionalized with a fluorescent dye molecule, and cations, and an oil phase comprising an amphiphilic copolymer,
   a") optionally integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) optionally removing the polymer shell from the polymer shell-stabilized giant unilamellar vesicles obtained in step c),
wherein the cationic lipid is selected from the group comprising dioleyl-N,N-dimethylammonium chloride ("DODAC"); N-(2,3-dioleyloxy)propyl)-N,N,N-trimethylammonium chloride ("DOTMA"); N,N-distearyl-N,N-dimethylammonium bromide ("DDAB"); N-(2,3- dioleyloxy)propyl)-N,N,N-trimethylammonium chloride ("DOTAP"); 3β-(N-(N',N'-dimethylaminoethane)-carbamoyl)cholesterol ("DC-Chol"); N-(1,2-dimyristyloxyprop-3-yl)-N,N-dimethyl-N-hydroxy ethyl ammonium bromide ("DMRIE"); cationic liposomes comprising DOTMA and 1,2-dioleoyl-sn-3-phosphoethanolamine ("DOPE"); cationic liposomes comprising N-(1-(2,3-dioleyloxy)propyl)-N-(2-(sperminecarboxamido)ethyl)-N,N-dimethylammonium trifluoroacetate ("DOSPA") and DOPE; cationic lipids comprising dioctadecylamidoglycyl carboxyspermine ("DOGS") in ethanol; N-(2,3-dioleyloxy)propyl)-N,N-dimethylammonium chloride ("DODMA") and 1,2-dioleoyl-3-dimethylammonium-propane ("DODAP"), and
wherein the neutral lipid is selected from the group comprising ceramide, sphingomyelin, cephalin, cholesterol, cerebrosides, diacylglycerols, phosphatidylcholines, phosphatidylglycerols, lysophosphatidylcholines, phosphatidylethanolamines, and lysoethanolamines, and
wherein the fluorescent dye molecule is selected from the group comprising lissamine rhodamine B sulfonyl, Atto488, Alexa Fluor 488, Alexa Fluor 647, Fluorescein, N-(7-Nitrobenz-2-Oxa-1,3-Diazol-4-yl (NBD), Cy5, Cy5.5, Cy7, Topfluor® Alexa Fluor488, Topfluor® Alexa Fluor594.

In some embodiments, the molar percentage (mol %) of a cationic lipid typically comprises from 0% to 10%, from 10% to 20%, from 10% to 30%, from 10% to 40%, %, from 10% to 50%, from 10% to 60%, from 20% to 30%, from 20% to 40%, from 20% to 50%, from 20% to 60% of the total lipid present in vesicle.

In some embodiments, the molar percentage (mol %) of neutral lipid typically comprises from 49% to 99%, from 49% to 89%, from 49% to 79%, from 49% to 69%, %, from 59% to 99%, from 59% to 89%, from 59% to 79%, from 59% to 69% of the total lipid present in vesicle.

In some embodiments, the molar percentage (mol %) of neutral lipid functionalized with a fluorescent dye molecule typically comprises from 0.1% to 5%, from 0.5% to 5%, from 1% to 5%, from 1% to 4%, %, from 1% to 3% the total lipid present in vesicle.

In particular, good results have been obtained with a molar percentage of the cationic lipid comprised between 1 - 51 %, and of the neutral lipid comprised between 49 - 99%.

### BIOFUNCTIONALIZATION OF THE GIANT UNILAMELLAR VESICLES.

The inventors have also developed a toolbox of strategies for bio-orthogonal functionalization of the giant unilamellar vesicle surface with specific targeting biomolecules, with the aim to provide a cell type specific delivery of therapeutic compounds.

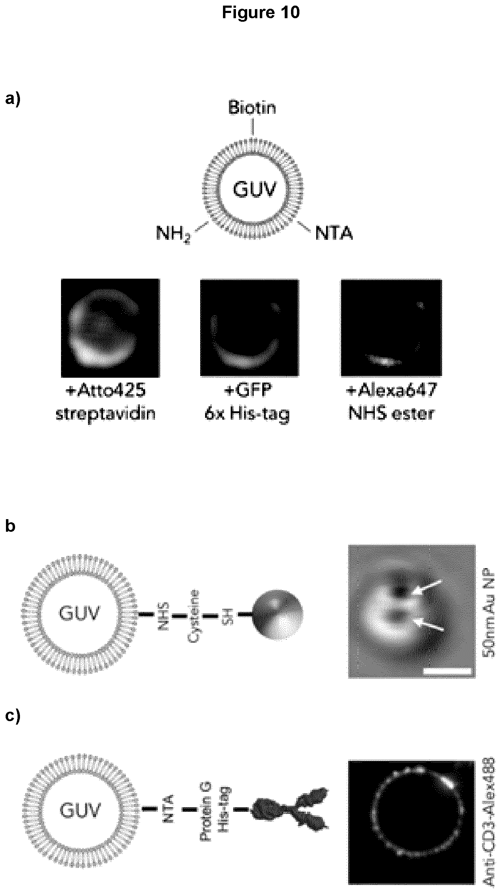

To exemplarily demonstrate the diversity of giant unilamellar vesicle biofunctionalization possibilities, giant unilamellar vesicles were produced by microfluidic droplet splitting using biotinylated lipids for coupling to streptavidin tagged proteins, NTA-Ni²⁺-functionalized lipids for coupling to histidine tagged proteins and DOPE lipids containing primary ammine for linking to N-hydroxysuccimid (NHS) functionalized molecules (Figures 10 - 14).

Suitable functional ligands are selected from the group comprising biotin, N-hydroxysuccinimide (NHS) ester, sulfo-NHS ester, nitrilotriacetic acid (NTA)-nickel, amine, carboxylic acid, maleimide, dithiopyridinyl, N-benzylguanine, carboxyacyl, cyanur, square, galloyl, thiol.
As shown in Table 2, these functional ligands react with particular moieties at high affinity, as for example the ligand biotin reacts with the moiety streptavidin or avidin. Thus, macromolecules of interest can be coupled to the surface of the released giant unilamellar vesicles by using the interaction at high affinity between a functional ligand and the respective reacting moiety.

Table 2 lists the possible functional ligands which can be attached to the lipids, their function and the interacting moieties.

Thus, an embodiment of the present invention is directed to a method for preparation of monodisperse cell-targeting giant unilamellar vesicles comprising the following steps:
a) providing a polymer shell-stabilized giant unilamellar vesicle, by the following step:
   a') merging a water phase comprising at least one lipid, and cations, and an oil phase comprising an amphiphilic copolymer, wherein at least one lipid functionalized with a functional ligand selected from biotin, N-hydroxysuccinimide (NHS) ester, sulfo-NHS ester, nitrilotriacetic acid (NTA)-nickel, amine, carboxylic acid, maleimide, dithiopyridinyl, pyridyl disulfide , pyridyldithiopropionate, N-benzylguanine , carboxyacyl, cyanur, square, galloyl, thiol,
   a") optionally integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) removing the polymer shell from the polymer shell-stabilized giant unilamellar vesicles obtained in step c),
d') coupling the giant unilamellar vesicles with at least one macromolecule comprising at least one moiety reacting with said functional ligands, wherein the macromolecule is selected from the group comprising a carbohydrate, a nucleic acid, a protein or a fragment thereof, a polypeptide, a cell receptor, an imaging probe, a nanoparticle,
e) purifying the giant unilamellar vesicles by centrifugation.

The "macromolecule" to be attached on the giant unilamellar vesicle can be a carbohydrate, a nucleic acid, a protein, a protein fragment, a polypeptide, a cell receptor, an imaging probe, a nanoparticle.

Preferably the protein is selected from the group comprising an antibody, an antigen-binding fragment, a cell receptor, a ligand, an antigen, a cytokine, a cytokine receptor, a glycoprotein, an enzyme.

As used herein the term "fragment" or "active fragment" of a protein refers to a fragment of that protein that retains the ability to be integrated in or specifically coupled to giant unilamellar vesicle. The term "fragment" or "active fragment" of a protein also refers to a fragment of that protein that retains the ability to exert its function in the target cell.

For example, in the case of membrane proteins, a protein fragment refers to a cytosolic domain, a transmembrane domain or an extracellular domain of said protein.

For example, in the case of enzymes, a protein fragment refers to a catalytic domain of that enzyme.

For example, in the case of antibodies, a protein fragment refers to a fragment of the antibody that retains its capacity to bind specifically to the antigen. The antibody or antigen-binding fragment can be derived from natural sources, or partly or wholly synthetically produced. In some embodiments, the antibody is a monoclonal antibody. In some of these embodiments, the monoclonal antibody is an IgG antibody. In certain embodiments, the monoclonal antibody is an IgG₁, IgG₂, IgG₃, or IgG₄. In some other embodiments, the antibody is a polyclonal antibody. In certain embodiments, the antibody fragment, also named antigen-binding fragment, is selected from antigen-binding fragment (Fab), Fab', and F(ab')2, F(ab)2, a viable fragment (Fv), and Fd fragments. In certain embodiments, the antigen-binding fragment is a Single-chain variable fragment (scFv) or (ScFv)2 fragment. In certain other embodiments, the antibody or antigen-binding fragment is a single-domain antibody. In some embodiments, the antibody or antigen binding fragment is a bispecific or multispecific antibody.

For example, in the case of protein antigens, a protein fragment refers to a fragment of the antigen that retains its capacity to induce an immune response in a human or animal, and/or to be specifically recognized by an antibody.

As used herein the term protein or fragment thereof also include "variant" of a protein or of a fragment thereof, and refers to a protein or fragment that shares a certain amino acid sequence identity with the reference protein or fragment upon alignment by a method known in the art. A variant of a protein or of a fragment thereof can include a substitution, insertion, deletion, frameshift or rearrangement in another protein. In some embodiments variants share at least 70%, 80%, 85%, 90%, 95% or 99% sequence identity with the native protein or with the fragment thereof.

Recitation of any protein provided herein encompasses a functional variant of the protein. The term "functional variant" of a protein refers to a variant of the protein that retains the ability to be integrated in or specifically targeted to the giant unilamellar vesicle.

The percentage of "sequence identity" is determined by comparing two optimally aligned protein or polypeptide sequences over a "comparison window" on the full length of the reference sequence. A "comparison window" as used herein, refers to the optimal alignment between the reference and variant sequence after that the two sequences are optimally aligned, wherein the variant nucleic acid or polypeptide sequence in the comparison window may comprise additions or deletions (i.e., gaps) of 20 percent or less, usually 5 to 15 percent, or 10 to 12 percent, as compared to the reference sequences (which does not comprise additions or deletions) for optimal alignment. Identity percentage is calculated by determining the number of positions at which the identical amino acid residues occurs in both sequences to yield the number of matched positions, dividing the number of matched positions by the total number of positions in the reference sequence (i.e., the full length in amino acid or nucleotide) and multiplying the results by 100 to yield the percentage of sequence identity. Two protein or polypeptide sequences are said to be "identical" if the sequence of nucleotides or amino acids in the two sequences is the same when optimally aligned as described above.
The percentage of "sequence identity" can be determined on the comparison window defined above with the help of blastp with the "BLAST 2 Sequences" tool available at the NCBI website. (Tatusova A. et al., FEMS Microbiol Lett. 1999, 174:247 - 250).

Alternatively, a variant sequence may also be any amino acid sequence resulting from allowed substitutions at any number of positions of the parent sequence according to the formula below:
Ser substituted by Ser, Thr, Gly, and Asn;
Arg substituted by one of Arg, His, Gin, Lys, and Glu;
Leu substituted by one of Leu, Ile, Phe, Tyr, Met, and Val;
Pro substituted by one of Pro, Gly, Ala, and Thr;
Thr substituted by one of Thr, Pro, Ser, Ala, Gly, His, and Gin;
Ala substituted by one of Ala, Gly, Thr, and Pro;
Val substituted by one of Val, Met, Tyr, Phe, Ile, and Leu;
Gly substituted by one of Gly, Ala, Thr, Pro, and Ser;
Ile substituted by one of Ile, Met, Tyr, Phe, Val, and Leu;
Phe substituted by one of Phe, Trp, Met, Tyr, lie, Val, and Leu;
Tyr substituted by one of Tyr, Trp, Met, Phe, Ile, Val, and Leu;
His substituted by one of His, Glu, Lys, Gin, Thr, and Arg;
Gln substituted by one of Gin, Glu, Lys, Asn, His, Thr, and Arg;
Asn substituted by one of Asn, Glu, Asp, Gin, and Ser;
Lys substituted by one of Lys, Glu, Gin, His, and Arg;
Asp substituted by one of Asp, Glu, and Asn;
Glu substituted by one of Glu, Asp, Lys, Asn, Gin, His, and Arg;
Met substituted by one of Met, Phe, Ile, Val, Leu, and Tyr.

Preferably the imaging probe is selected from the group comprising fluorescent dyes, colorimetric dyes.

Preferably the carbohydrate is selected from the group comprising polyglycans, lactose, sucrose, β-galactose, α-mannose-, β-mannose-, and α-fucose.

Preferably the nucleic acid is selected from the group comprising naturally or non-naturally occurring DNA, including cDNA, genomic DNA, left handed DNA and RNA, peptide nucleic acid (PNA), nuclear DNA, mitochondrial DNA, RNA, including mRNA, rRNA, tRNA, oligonucleotides, a triple-helix forming molecule, immunostimulatory nucleic acids such as immunostimulatory CpG nucleic acids, small interfering RNA (siRNA) or microRNAs (miRNA) used to modulate gene expression, antisense oligonucleotides used to modulate gene expression, aptamers, ribozymes, a gene or gene fragment, a regulatory sequence

Particular examples of suitable macromolecules are: bovine serum albumin, poly-L-lysine, basic fibroblast growth factor (bFGF), epidermal growth factor (EGF), wheat germ agglutinin (WGA), anti-cadherin, anti-α4 integrin, interleukin-2 (IL2), insulin, bradykinin, tat(HIV)-GFP, cystein, CD95L, e-cadherin, fibronectin, anti-CD3 antibody, gold nanoparticles, arginylglycylaspartic acid (RGD), meuronal cell adhesion molecule (NrCAM), or a fragment of any of the aforementioned.

Slightly reworded, an embodiment of the present invention is directed to a method for preparation of monodisperse cell-targeting giant unilamellar vesicles comprising the following steps:
a) providing a polymer shell-stabilized giant unilamellar vesicle, by the following step:
   a') merging a water phase comprising at least one lipid, and cations, and an oil phase comprising an amphiphilic copolymer, wherein at least one lipid functionalized with a functional ligand selected from biotin, N-hydroxysuccinimide (NHS) ester, sulfo-NHS ester, nitrilotriacetic acid (NTA)-nickel, amine, carboxylic acid, maleimide, dithiopyridinyl, pyridyl disulfide , pyridyldithiopropionate, N-benzylguanine , carboxyacyl, cyanur, square, galloyl, thiol,
   a") optionally integrating one or more proteins or active fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) removing the polymer shell from the polymer shell-stabilized giant unilamellar vesicles obtained in step c),
d') coupling the giant unilamellar vesicles with at least one macromolecule comprising at least one moiety reacting with said functional ligands, wherein the macromolecule is selected from the group comprising a carbohydrate, a nucleic acid, a protein or an active fragment thereof, a polypeptide, a cell receptor, an imaging probe, a nanoparticle,
e) purifying the giant unilamellar vesicles by centrifugation.

In other words, an embodiment of the present invention is directed to a method for preparation of monodisperse cell-targeting giant unilamellar vesicles comprising the following steps:
a) providing a polymer shell-stabilized giant unilamellar vesicle, by the following step:
   a') merging a water phase comprising at least one lipid, and cations, and an oil phase comprising an amphiphilic copolymer, wherein at least one lipid functionalized with a functional ligand selected from biotin, N-hydroxysuccinimide (NHS) ester, sulfo-NHS ester, nitrilotriacetic acid (NTA)-nickel, amine, carboxylic acid, maleimide, dithiopyridinyl, pyridyl disulfide, pyridyldithiopropionate, N-benzylguanine , carboxyacyl, cyanur, square, galloyl, thiol,
   a") optionally integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) removing the polymer shell from the polymer shell-stabilized giant unilamellar vesicles obtained in step c),
d') coupling the giant unilamellar vesicles with at least one macromolecule comprising at least one moiety reacting with said functional ligands, wherein the macromolecule is selected from the group comprising a carbohydrate, a nucleic acid, a protein or a fragment thereof, a polypeptide, a cell receptor, an imaging probe, a nanoparticle,
e) purifying the giant unilamellar vesicles by centrifugation,
wherein the protein or the fragment thereof share at least 70%, 80%, 85%, 90%, 95% or 99% sequence identity with the native protein or with the fragment thereof.

In other words, an embodiment of the present invention is directed to a method for preparation of monodisperse cell-targeting giant unilamellar vesicles comprising the following steps:
a) providing a polymer shell-stabilized giant unilamellar vesicle, by the following step:
   a') merging a water phase comprising at least one lipid, and cations, and an oil phase comprising an amphiphilic copolymer, wherein at least one lipid functionalized with a functional ligand selected from biotin, N-hydroxysuccinimide (NHS) ester, sulfo-NHS ester, nitrilotriacetic acid (NTA)-nickel, amine, carboxylic acid, maleimide, dithiopyridinyl, pyridyl disulfide , pyridyldithiopropionate, N-benzylguanine , carboxyacyl, cyanur, square, galloyl, thiol,
   a") optionally integrating one or more proteins or active fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) removing the polymer shell from the polymer shell-stabilized giant unilamellar vesicles obtained in step c),
d') coupling the giant unilamellar vesicles with at least one macromolecule comprising at least one moiety reacting with said functional ligands, wherein the macromolecule is selected from the group comprising a carbohydrate, a nucleic acid, a protein or an active fragment thereof, a polypeptide, a cell receptor, an imaging probe, a nanoparticle,
e) purifying the giant unilamellar vesicles by centrifugation,
wherein the protein or the active fragment thereof share at least 70%, 80%, 85%, 90%, 95% or 99% sequence identity with the native protein or with the fragment thereof.

### PROTEIN INTEGRATION.

In addition to the protein biofunctionalization of the released giant unilamellar vesicles reported above, one or more proteins or fragments thereof may be integrated into the parent polymer shell-stabilized giant unilamellar vesicle before splitting at the step a").
The proteins may be provided in a buffer soluble form or the proteins may be already incorporated into the wall of small protein liposomes thus forming proteoliposomes, i.e. vesicles having preferably at least one lipid bilayer, into which the one or more proteins have been inserted. These protein liposomes fuse with the given giant unilamellar vesicle inside the polymer shell.
For instance, step a") may be performed by incorporating one or more proteins or fragments thereof into the polymer shell-stabilized giant unilamellar vesicle provided in step a') by electro-microfluidics making use of an injector, which is preferably a pico-injector as described in WO 2018228894 A1. More preferably, the one or more proteins or fragments thereof are incorporated in this embodiment into the polymer shell-stabilized giant unilamellar vesicle by injecting them using the pico-injector in form of respective proteoliposomes.

During step a") a transmembrane protein or a fragment thereof, and/or a cytoskeleton protein or a fragment thereof may be incorporated into the lipid bilayer and/or into the inner space of the polymer shell-stabilized giant unilamellar vesicle, respectively.

The present invention is not limited concerning the kind of protein incorporated into the polymer shell-stabilized giant unilamellar vesicle. For example, proteins selected from the group comprising receptors, ATP-synthase, polymerase, actin, tubulin, antibodies, integrins, ribosome-associated proteins, nucleus associated proteins, signaling proteins, immunologically relevant proteins, antibodies, different ion- pump proteins, adhesion associated proteins and arbitrary combinations of two or more of the aforementioned proteins are suitable for integration into the polymer shell stabilized giant unilamellar vesicle of step a').

Thus, an embodiment of the present invention is directed to a method for preparation of monodisperse cell-targeting giant unilamellar vesicles comprising the following steps:
a) providing a polymer shell-stabilized giant unilamellar vesicle, by the following step:
   a') merging a water phase comprising at least one lipid, and cations, and an oil phase comprising an amphiphilic copolymer,
   a") integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) removing the polymer shell from the polymer shell-stabilized giant unilamellar vesicles obtained in step c),
e) purifying the giant unilamellar vesicles by centrifugation.

Slightly reworded, an embodiment of the present invention is directed to a method for preparation of monodisperse cell-targeting giant unilamellar vesicles comprising the following steps:
a) providing a polymer shell-stabilized giant unilamellar vesicle, by the following step:
   a') merging a water phase comprising at least one lipid, and cations, and an oil phase comprising an amphiphilic copolymer,
   a") integrating one or more proteins or active fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) removing the polymer shell from the polymer shell-stabilized giant unilamellar vesicles obtained in step c),
e) purifying the giant unilamellar vesicles by centrifugation.

In other words, an embodiment of the present invention is directed to a method for preparation of monodisperse cell-targeting giant unilamellar vesicles comprising the following steps:
a) providing a polymer shell-stabilized giant unilamellar vesicle, by the following step:
   a') merging a water phase comprising at least one lipid, and cations, and an oil phase comprising an amphiphilic copolymer
   a") integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) removing the polymer shell from the polymer shell-stabilized giant unilamellar vesicles obtained in step c),
e) purifying the giant unilamellar vesicles by centrifugation,
wherein the protein or the fragment thereof share at least 70%, 80%, 85%, 90%, 95% or 99% sequence identity with the native protein or with the fragment thereof.

In other words, an embodiment of the present invention is directed to a method for preparation of monodisperse cell-targeting giant unilamellar vesicles comprising the following steps:
a) providing a polymer shell-stabilized giant unilamellar vesicle, by the following step:
   a') merging a water phase comprising at least one lipid, and cations, and an oil phase comprising an amphiphilic copolymer,
   a") integrating one or more proteins or active fragments into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) removing the polymer shell from the polymer shell-stabilized giant unilamellar vesicles obtained in step c),
e) purifying the giant unilamellar vesicles by centrifugation,
wherein the protein or the active fragment thereof share at least 70%, 80%, 85%, 90%, 95% or 99% sequence identity with the native protein or with the fragment thereof.

### LYSOSOMAL ESCAPE OF GIANT UNILAMELLAR VESICLE CARGO FOR EFFICIENT CYTOPLASMIC DELIVERY

Three main strategies can be used to facilitate endosomal escape of liposomes:
1 "pH-sensitive lipids", such as DOBAQ, 1,2-distearoyl-3-dimethylammonium-propane.
2 "proton sponge mechanism" involves cationic polymers containing nitrogen atoms that can be protonated (e.g. polyethylenimine -PEI) and which act as a 'proton sponge". They attract the protons in the endosome, leading to diffusion of more protons accompanied by chloride ions into the endosome. When osmotic pressure becomes high enough to destroy the endosome, the DNA particles are released into the cytosol.
3 pH-sensitive fusogenic peptides, used by bacteria to facilitate escape of their cargo from lysosomes to host cells, as these peptides are able to perturb the endosomal membrane and affect release.

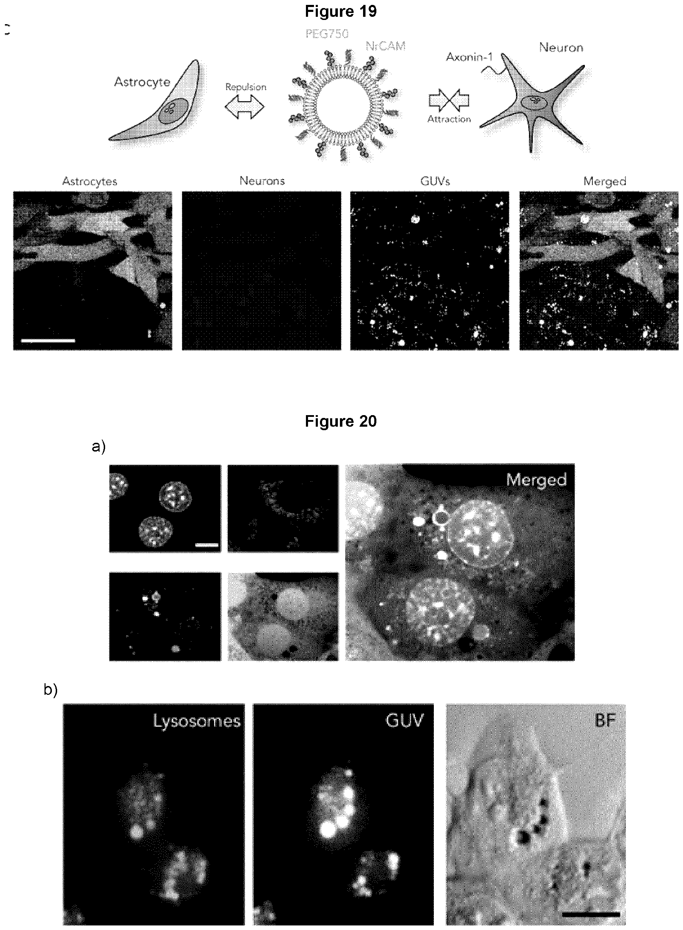

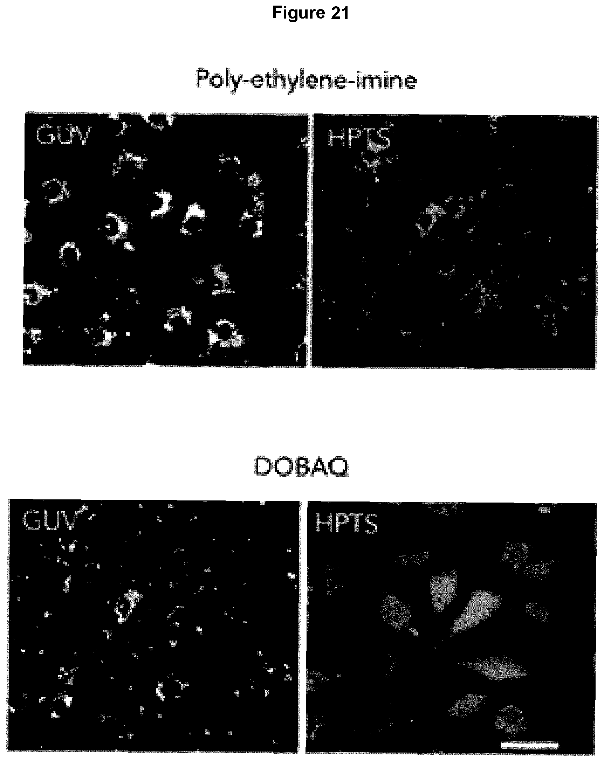

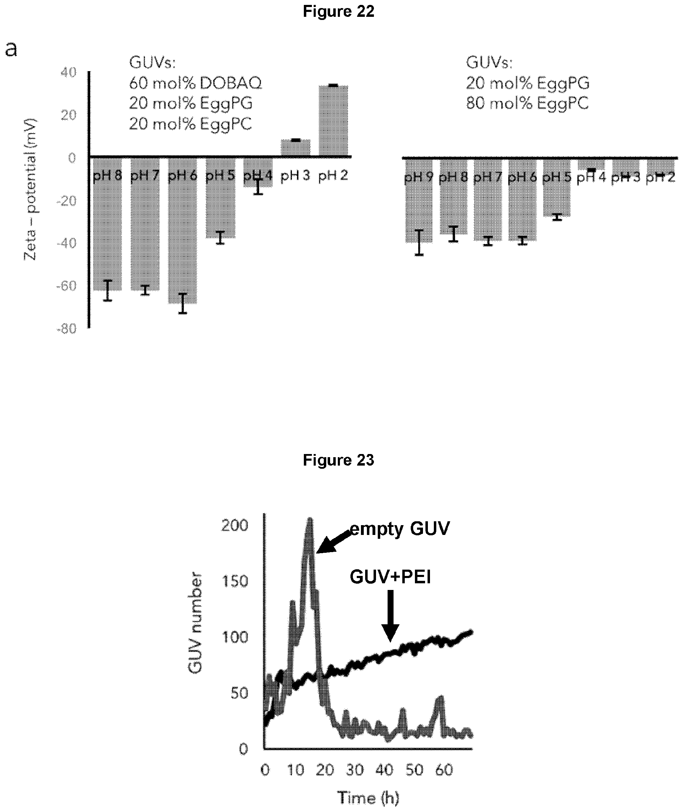

Importantly, the inventors have optimized lysosomal escape mechanisms for efficient release of giant unilamellar vesicle cargos into cells avoiding lysosomal degradation (Figures 20 - 23). This strategy represents a pivotal requirement for giant unilamellar vesicles delivering therapeutic agents such as pharmacological compounds targeting cytoplasmic components. In particular, the inventors found that PEI loaded giant unilamellar vesicle accumulate into the cells and are protected from degradation (Figures 21, 23), potentially because PEI can act in the giant unilamellar vesicle lumen as a potent pH buffer preventing acidic degradation and mature lysosome formation. For approaches in which stable incorporation of giant unilamellar vesicles and their cargo into cells is required, this might represent a promising implementation mechanism. Differently, giant unilamellar vesicle containing N-(4-carboxybenzyl)-N,N-dimethyl-2,3-bis(oleoyloxy)propan-1-aminium (DOBAQ) in the lipid bilayer not only were protected from lysosomal degradation, but also successfully released their cargo into the cytoplasm (Figures 22).

Therefore, an embodiment the present invention is a method for preparation of monodisperse cell-targeting giant unilamellar vesicles comprising the following steps:
a) providing a polymer shell-stabilized giant unilamellar vesicle, by the following step:
   a') merging a water phase comprising at least one lipid, and cations, and an oil phase comprising an amphiphilic copolymer,
   a") optionally integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) optionally removing the polymer shell from the polymer shell-stabilized giant unilamellar vesicles obtained in step c),
wherein the water phase of step a') comprises at least one pH-sensitive lipid at a molar percentage comprised between 20 % - 80 %, or
wherein the water phase of step a') further comprises poly-ethylene-imine at a concentration comprised between 2 - 100 µg/ml.

### TARGETED DELIVERY OF LARGE HEAVY DUTY CARGOES

Moreover, the inventors were able to encapsulate advanced drug delivery cargos into the giant unilamellar vesicles. This paves the way for a more efficient drug administration that would not be possible by the conventional delivery based on small unilamellar vesicles.

The invention contemplates the encapsulation of cargos of interest in the inventive giant unilamellar vesicles, in order to allow their delivery to regions, tissues or cells in *vivo or in vitro.*

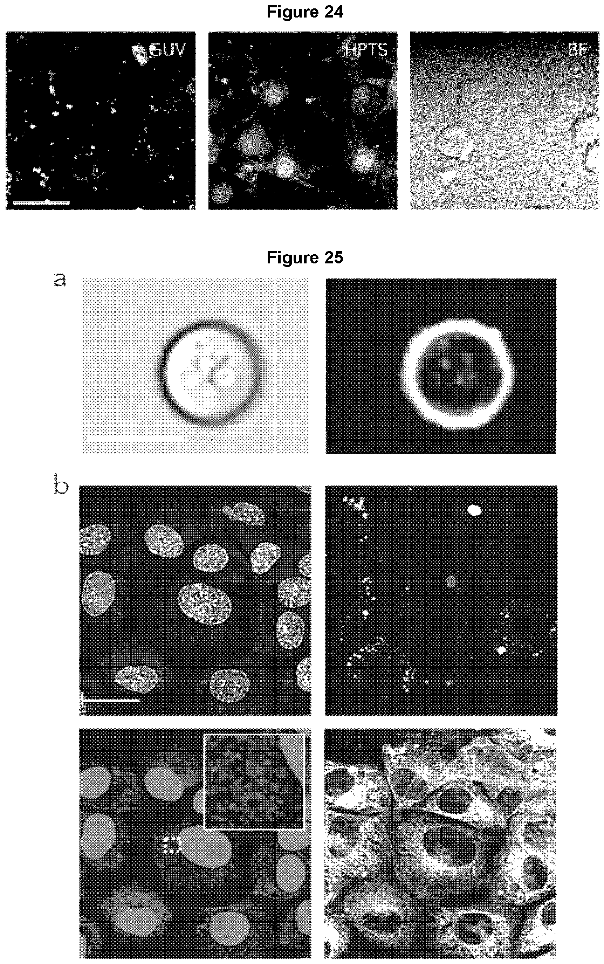

For example, the inventors were able to load purified baculoviruses (BV), considered promising future candidates for transduction and handling of large amounts of genetic material in genome engineering approaches. The inventors achieved successful assembly and release of BV-loaded giant unilamellar vesicles (Figure 25). Following the formation, the BV-loaded DOBAQ-containing giant unilamellar vesicles were incubated with rat embrionic fibloblasts REF52 cells. It was observed intracellular uptake of the giant unilamellar vesicles, as well as release of the BV (stained by Hoechst 33342) into the cells cytoplasm. This was accompanied by the expression of mitochondrial targeted dsRed protein encoded by the BVs, indicating successful giant unilamellar vesicle based transduction of mammalian cells with BV carrier giant unilamellar vesicles. These results highlight the advantages of giant unilamellar vesicle-based drug delivery towards more efficient drug administration of advanced cargoes.

As used herein, the cargo or freight may be an agent selected from the group comprising drug releasing porous particles, molecular imaging agents, diagnostic agents, therapeutic agents, proteins or fragments thereof, polypeptides, peptides, enzymes or fragments thereof, nucleic acids, oligonucleotides, polynucleotides, up-and-coming DNA origami robots, small molecule drugs, virus particles, virus-like particles, microbial antigens, steroids, proteoglycans, lipids, (poly- )carbohydratesmonosaccharides, oligosaccharides, polysaccharides, magnetic particles, nanorods, carbon nanotubes, dentritosomes, polymerosomes, metal nanoparticles and combinations or conjugates thereof.

Encapsulated proteins or fragments thereof can be single or multi-chain proteins and peptides. Examples include antibodies, single chain antibodies, antibody fragments, enzymes, co-factors, receptors, ligands, transcription factors and other regulatory factors, antigens, cytokines, chemokines, and the like. These protein-based agents may or may not be naturally occurring but they are capable of being synthesized within the subject, for example, through the use of genetically engineered cells.

"Diagnostic agent" refers to an agent capable of diagnosing a condition or disease. Diagnostic agents include, but are not limited to, molecular imaging probes such as chromophores, fluorophores, and radiolabels.

"Therapeutic agent" refers to an agent capable of treating and/or ameliorating a condition or disease. Therapeutic agents include, but are not limited to, compounds, drugs, peptides, oligonucleotides, DNA, antibodies, and others.

Therapeutic or diagnostic agents can be encapsulated in the giant unilamellar vesicle according to the invention, and these include without limitation imaging agents, immunomodulatory agents such as immunostimulatory agents and immunoinhibitory agents, antigens, adjuvants, cytokines, chemokines, anti-cancer agents, anti-infective agents, nucleic acids, antibodies or fragments thereof, fusion proteins such as cytokine-antibody fusion proteins, Fc-fusion proteins, and the like.

As used herein, a molecular "imaging agent" is an agent that emits signal directly or indirectly thereby allowing its detection in vivo. Imaging agents include contrast agents and radioactive agents that can be detected using medical imaging techniques such as nuclear medicine scans and magnetic resonance imaging (MRI). Molecular imaging agents for magnetic resonance imaging (MRI) include Gd(DOTA), iron oxide or gold nanoparticles; imaging agents for nuclear medicine include 201T1, gamma-emitting radionuclide 99 mTc; imaging agents for positron-emission tomography (PET) include positron-emitting isotopes, (18)F-fluorodeoxyglucose ((18)FDG), (18)F-fluoride, copper-64, gadoamide, and radioisotopes of Pb(II) such as 203 Pb, and 11In; fluorescent dyes or dye-conjugated nanoparticles. Other imaging agents are fluorescent and colorimetric dyes.

"Microbial antigens" are antigens derived from microbial species such as without limitation bacterial, viral, fungal, parasitic and mycobacterial species. As such, microbial antigens include bacterial antigens, viral antigens, fungal antigens, parasitic antigens, and mycobacterial antigens. Examples of bacterial, viral, fungal, parasitic and mycobacterial species are provided herein. The microbial antigen may be part of a microbial species or it may be the entire microbe.

"Nucleic Acid Agents" that can be delivered to a subject according to the invention include naturally or non-naturally occurring DNA, , left handed DNA and RNA, peptide nucleic acid (PNA), including cDNA, genomic DNA, nuclear DNA, mitochondrial DNA, RNA, including mRNA, rRNA, tRNA, oligonucleotides, a triple-helix forming molecule, immunostimulatory nucleic acids such as immunostimulatory CpG nucleic acids, small interfering RNA (siRNA) or microRNAs (miRNA) used to modulate gene expression, antisense oligonucleotides used to modulate gene expression, aptamers, ribozymes, a gene or gene fragment, a regulatory sequence, up-and-coming DNA origami robots, including analogs, derivatives, and combinations thereof.

Aqueous solutions containing the cargo agents are usually mixed with the water phase of step a') in order to obtain giant unilamellar vesicles containing said cargos.

Thus, the present invention is also directed to a method for preparation of monodisperse cell-targeting giant unilamellar vesicles comprising the following steps:
a) providing a polymer shell-stabilized giant unilamellar vesicle, by the following step:
   a') merging a water phase comprising at least one lipid, and cations, and an oil phase comprising an amphiphilic copolymer,
   a") optionally integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) optionally removing the polymer shell from the polymer shell-stabilized giant unilamellar vesicles obtained in step c),
wherein the water phase of step a') further comprises at least one agent selected from the group comprising drug releasing porous particles, molecular imaging agents, diagnostic agents, therapeutic agents, proteins or fragments thereof, polypeptides, peptides, enzymes, nucleic acids, oligonucleotides, polynucleotides, up-and-coming DNA origami robots, small molecule drugs, virus particles, virus-like particles, microbial antigens, steroids, proteoglycans, lipids, monosaccharides, oligosaccharides, polysaccharides, magnetic particles, nanorods, carbon nanotubes, dentritosomes, polymerosomes, metal nanoparticles and combinations or conjugates thereof.

### SUITABLE COPOLYMER TO STABILIZE THE GIANT UNILAMELLAR VESICLES

In order to allow a good dispersion of the polymer shell stabilized vesicles in the oil phase and in order to allow a good dispersion of the lipid containing aqueous phase within the polymer shell of the vesicle, it is preferred that the polymer shell is made of an amphiphilic copolymer with a hydrophobic end arranged at the outer side and a hydrophilic end arranged at the inner side of the polymer shell.

This may be achieved by forming the polymer shell of the droplet from a diblock copolymer or a triblock copolymer.

Good results are particularly obtained, if the polymer shell of the droplet is made of a block copolymer comprising an hydrophobic block arranged at the outer side and a hydrophilic block arranged at the inner side of the polymer shell. The hydrophobic block may be, but is not restricted to members, e.g. selected from the group consisting of perfluorinated polymers, such as perfluorinated polyethers, polystyrene or poly(olefin oxides), such as poly(propylene oxide), whereas the hydrophilic block may be selected e.g. from polyether glycols, polyetheramine, polyacrylate acid, polymethylacrylate acid or poly[poly(ethylene glycol) methyl ether methacrylate]. Likewise, good results are obtained, if the polymer shell of the droplet is made of a triblock copolymer comprising two hydrophobic perfluorinated polymer end blocks and therebetween a hydrophilic polyether glycol block, wherein the triblock copolymer is folded so that the hydrophobic perfluorinated polymer blocks are arranged at the outer side and that the hydrophilic polyether glycol block is arranged at the inner side of the polymer shell. Examples for the hydrophobic blocks and the hydrophilic blocks are the same as those mentioned above.

Preferably, the perfluorinated polymer block is a perfluorinated polyether block (PFPE) and more preferably a perfluorinated polyether block having a weight average molecular weight of 1,000 to 10,000 g/mol. Likewise preferably, the polyether glycol (PEG) and polyetheramine (JEFFAMINE) blocks have preferably a weight average molecular weight of 100 to 50,000 g/mol. More specifically, suitable examples for the respective copolymers are PFPE-carboxylic acid (Krytox, MW 2500 or 7000 g/mol) and suitable examples for the respective diblock copolymers are PFPE(7000 g/mol)-PEG(1400 g/mol), PFPE(7000 g/mol)-PEG(600 g/mol), PFPE(2500 g/mol)- PEG(600 g/mol), PFPE(4000 g/mol)-PEG(600 g/mol), PFPE(4000 g/mol)- PEG(1400 g/mol), PFPE(2000 g/mol)-PEG(600 g/mol), PFPE(7000 g/mol)- JEFFAMINE(600 g/mol), PFPE(7000 g/mol)-JEFFAMINE (900 g/mol), PFPE(2500 g/mol)- JEFFAMINE(600 g/mol), PFPE(2500 g/mol)- JEFFAMINE(900 g/mol), PFPE(4000 g/mol)- JEFFAMINE(900 g/mol), PFPE(2500 g/mol)- JEFFAMINE(600 g/mol), PFPE(2000 g/mol)-JEFFAMINE (600 g/mol), PFPE(2000 g/mol)- JEFFAMINE (900 g/mol) and suitable examples for the respective triblock copolymers are PFPE(7000 g/mol)-PEG(1400 g/mol)-PFPE(7000 g/mol), PFPE(7000 g/mol)-PEG(600 g/mol)-PFPE(7000 g/mol), PFPE(4000 g/mol)- PEG(1400 g/mol)- PFPE(4000 g/mol) PFPE(2500 g/mol)- PEG(600 g/mol)- PFPE(2500 g/mol), PFPE(2000 g/mol)-PEG(600 g/mol)-PFPE(2000 g/mol), PFPE(7000 g/mol)- JEFFAMINE(900 g/mol)-PFPE(7000 g/mol) PFPE(7000 g/mol)- JEFFAMINE(600 g/mol)-PFPE(7000 g/mol), PFPE(4000 g/mol)- JEFFAM- INE(900 g/mol)-PFPE(4000 g/mol), PFPE(4000 g/mol)- JEFFAMINE(600 g/mol)- PFPE(4000 g/mol), PFPE(2500 g/mol)- JEFFAMINE(900 g/mol)-PFPE(2500 g/mol), PFPE(2500 g/mol)-JEFFAMINE(600 g/mol)-PFPE(2500 g/mol), PFPE(2000 g/mol)- JEFFAMINE(900 g/mol)-PFPE(2000 g/mol) and PFPE(2000 g/mol)- JEFFAMINE(600 g/mol)-PFPE(2000 g/mol). The molecular weight is determined by gel permeation chromatography using a polystyrene standard.

According to a further aspect, the present invention is directed to a method for preparation of monodisperse cell-targeting giant unilamellar vesicles comprising the following steps:
a) providing a polymer shell-stabilized giant unilamellar vesicle, by the following step:
   a') merging a water phase comprising at least one lipid, and cations, and an oil phase comprising an amphiphilic copolymer,
   a") optionally integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) optionally removing the polymer shell from the polymer shell-stabilized giant unilamellar vesicles obtained in step c,
wherein the amphiphilic copolymer of step a') consists of (i) a triblock copolymer comprising two perfluorinated polymer end blocks and one polyether glycol block, or of (ii) a diblock copolymer comprising one perfluorinated polymer end block and a polyether glycol block, wherein the triblock or diblock copolymer is folded so that the perfluorinated polymer end blocks are arranged at the outer side and that the polyether glycol block is arranged at the inner side of the polymer shell.

According to a further aspect, the present invention is directed to a method for preparation of monodisperse cell-targeting giant unilamellar vesicles comprising the following steps:
a) providing a polymer shell-stabilized giant unilamellar vesicle, by the following step:
   a') merging a water phase comprising at least one lipid, and cations, and an oil phase comprising an amphiphilic copolymer,
   a") optionally integrating one or more proteins or fragments thereof into the polymer shell stabilized giant unilamellar vesicle provided in step a'),
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) optionally removing the polymer shell from the polymer shell-stabilized giant unilamellar vesicles obtained in step c,
wherein the amphiphilic copolymer of step a') consists of (i) a triblock copolymer comprising two hydrophobic lipophobic perfluorinated polymer end blocks and one hydrophilic polyether glycol block, or of (ii) a diblock copolymer comprising one hydrophobic and lipophobic perfluorinated polymer end block and one hydrophilic polyether glycol block, wherein the triblock or diblock copolymer is folded so that the perfluorinated polymer end blocks are arranged at the outer side and that the polyether glycol block is arranged at the inner side of the polymer shell.

### SYNTHESIS OF PARENT POLYMER SHELL STABILIZED GIANT UNILAMELLAR VESICLES

The parent polymer shell stabilized giant unilamellar vesicle can be synthetized by using a method known in the prior art (Göpfrich et al., 2018, "Mastering Complexity: Towards Bottom-up Construction of Multifunctional Eukaryotic Synthetic Cells"; Haller et al., 2018 "Charge-controlled microfluidic formation of lipid based single- and multicompartment systems"; Göpfrich et al., 2019, "One-Pot Assembly of Complex Giant Unilamellar Vesicle-Based Synthetic Cells, Weiss et al. 2017 Nature Materials).

The at least one lipid comprised in the water phase of step a') may be provided as an aqueous dispersion of small, large or giant unilamellar lipid-vesicles. However, it is preferred if the at least one lipid is provided as small or large well- dispersed unilamellar vesicles.

Good results are obtained within this regard, when the at least one lipid is comprised in the water phase of step a') in form of small or large unilamellar lipid-vesicles, wherein the small or large unilamellar lipid-vesicles have been e.g. formed by dissolving the lipid(s) in a solvent, such as chloroform, drying the so obtained mixture under inert gas atmosphere, resuspending the dried lipid in an aqueous buffer, vortexing the mixture and homogenizing the vesicle size by extruding the so obtained mixture through a filter. For instance, the filter may be a polycarbonate filter with a pore size of 50 nm. Alternatively, in the case of proteoliposomes, the large unilamellar lipid-vesicles may be formed through detergent removal.

Alternatively, the at least one lipid is comprised in the water phase of step a') in form of small, large or giant unilamellar lipid-vesicles, which have been formed by an electroforming process, preferably by a process comprising the steps of dissolving the lipid(s) in a solvent, such chloroform, of spreading the so obtained mixture onto two indium oxide coated glasses, evaporating the solvent, filling the space between the two glasses with water and applying an alternative electrical potential of 1 to 100 Hz at 0.1 to 10 Volt for 0.1 to 10 hours.

Concerning the technique for merging a water phase comprising at least one lipid and an oil phase comprising an amphiphilic copolymer to obtain the parent polymer shell stabilized giant unilamellar vesicle of step a), the present invention is not limited. For example, any suitable microfluidics or other techniques for water-in-oil emulsion may be used. However, in accordance with one particular preferred embodiment of the present invention, the merging of a water phase comprising at least one lipid and of an oil phase comprising an amphiphilic copolymer during step a) is performed in a flow-focusing microfluidic device as described in WO 2018/228894 A1. In this technique, two-phases, namely the continuous phase and the dispersed phase, meet at a flow-focusing junction. The flow-focusing junction consists of three inlet channels converging into a main channel or outlet channel, respectively, via a narrow orifice. For example, two of three inlet channels are arranged in the vertical direction, wherein both vertical inlet channels converge at the flow-focusing junction, one coming from above and one coming from below the flow-focusing junction. Furthermore, the third inlet channel is arranged in the horizontal direction and meets the other two inlet channels at the flow-focusing junction coming from the left side. The main channel or outlet channel, respectively, is also arranged in the horizontal direction and starts with its narrow orifice on the side opposite the terminal end of the horizontal inlet channel. During the operation, the continuous oil phase comprising the amphiphilic copolymer(s) (which later form the polymer shell) dispersed or dissolved in oil, flows through the two vertical inlet channels, wherein both continuous oil phase partial streams converge at the flow-focusing junction. The dispersed aqueous phase including the lipid(s) flows through the horizontal inlet channel and is squeezed at the flow-focusing junction by the oil-phase flowing through the two vertical inlet channels. Both phases pass through the small orifice that is located downstream the three inlet channels, wherein the stream of the dispersed phase becomes narrow and breaks into droplets of the lipid containing aqueous phase, wherein the droplets are covered by the amphiphilic copolymer(s) thus forming a polymer shell, with the lipophilic or hydrophobic end of the copolymer being oriented at the outer shell side towards the continuous oil phase and the hydrophilic end of the copolymer being oriented at the inner shell side towards the dispersed, lipid containing aqueous phase. The droplet size can be adjusted by the flow rates of the two phases, by the flow rate ratio and by the channel geometries. Alternatively, the continuous oil phase comprising the amphiphilic copolymer(s) later forming the polymer shell dispersed or dissolved in oil, and the dispersed aqueous phase including the lipid(s) may be merged using other flow microfluidic techniques, comprising for example a T-flow focusing junction.

### MICROFLUIDIC DEVICE

A particular embodiment of the invention is directed to a method for preparation of monodisperse cell-targeting giant unilamellar vesicles comprising the following steps:
a) providing a polymer shell-stabilized giant unilamellar vesicle ;
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm,
d) optionally removing the polymer shell from the polymer shell-stabilized giant unilamellar vesicles obtained in step c),
wherein step b) comprises mechanically dividing said polymer shell stabilized giant unilamellar vesicle into two smaller polymer shell stabilized giant unilamellar vesicles using a microfluidic device comprising at least one Y- shaped junction, wherein said Y-shaped junction consists of one inlet channel and two outlet channels, and wherein step c) comprises repeating the step b) by using four or more sequential generations of Y-shaped junctions, wherein the inlet channel of each Y-shaped junction consists of the outlet channel of the previous Y-shaped junction.

Another embodiment of the present invention is a microfluidic device for preparing polymer shell stabilized giant unilamellar vesicles having a diameter smaller than 10 µm, comprising a division zone and a flow-rate control system,
wherein the division zone comprises one or more sequential generations of Y-shaped junctions, wherein each Y-shaped junction consists of one inlet channel and two outlet channels, and wherein the inlet channel of each Y-shaped junction consists of the outlet channel of the previous junction.

In a preferred embodiment of the present invention said microfluidic device further comprises a generation zone of a parent polymer shell stabilized giant unilamellar vesicle positioned upstream the division zone, said generation zone comprising a flow-focusing junction consisting of a horizontal inlet channel and two vertical inlet channels, wherein said three inlet channels converge into an outlet channel through a narrow orifice, wherein said outlet channel is connected to the division zone.

Therefore, a preferred embodiment of the invention is directed to a microfluidic device for preparing polymer shell stabilized giant unilamellar vesicles having a diameter smaller than 10 µm, comprising a division zone and a flow-rate control system,
wherein the division zone comprises one or more sequential generations of Y-shaped junctions, wherein each Y-shaped junction consists of one inlet channel and two outlet channels, and wherein the inlet channel of each Y-shaped junction consists of the outlet channel of the previous junction,
and further comprising a generation zone of a parent polymer shell stabilized giant unilamellar vesicle positioned upstream the division zone,
said generation zone comprising a flow-focusing junction consisting of a horizontal inlet channel and two vertical inlet channels, wherein said three inlet channels converge into an outlet channel through a narrow orifice, wherein said outlet channel is connected to the division zone.

### Description of the Figures

- Figure 1: shows a phase-contrast microscopy image showing the microfluidic device for mechanical fivefold division of the parent polymer shell stabilized giant unilamellar vesicle, allowing formation of 32 daughter polymer shell stabilized giant unilamellar vesicle from a single parent vesicle having 60 µm diameter. Inset shows time course of mechanical division of a droplet at a Y-junction from a higher magnification recording in bright field. Scale bar is 300 µm, scale bar in inset is 60 µm.
- Figure 2: shows single plane fluorescence confocal microscopy images of mechanically divided vesicles loaded with AlexaFluor 405 (links upper row), vesicles labelled with 1 mol% LissRhod PE (right upper row), green fluorescent protein (links, lower row) and 1 µm fluorescent polystyrene beads (right lower row). Upper right corner shows coefficient of variation (CV) of vesicle mean fluorescence intensity (n = 665 single droplets). Scale bar is 40 µm.
- Figure 3: shows single plane fluorescence confocal microscopy images of giant unilamellar vesicles (20 mol% EggPG, 79 mol% EggPC and 1 mol% LissRhod PE) obtained by mechanical division and released in PBS. Scale bar is 25 µm.
- Figure 4: Optimization of intraluminal lipid concentration of small unilamellar vesicles for production by mechanical splitting of polymer shell stabilized giant unilamellar vesicles. Polymer shell stabilized vesicles containing lipid concentrations of 12 mM, 6 mM, 3 mM, 1.5 mM and 0.75 mM were mechanically splitted and the number of giant unilamellar vesicles before and after release of the polymer shell was counted to obtain the release efficiencies.
- Figure 5: Assessment of mechanical stability of giant unilamellar vesicles kept at 4 °C without mechanical agitation and giant unilamellar vesicles shaken at 800 rpm/ 37 °C. Results are shown as average value with SD from three technical replicates.
- Figure 6: shows representative phase contrast image of mechanically splitted giant unilamellar vesicles incubated with rat embryonic fibroblasts REF52 cells. White arrows indicate single giant unilamellar vesicles. Scale bar is 10 µm.
- Figure 7: shows attraction quantification of differently charged giant unilamellar vesicles (Zeta-potential as indicated on x-axis) with cell lines of endothelial (MDCK), epithelial (A431D and A431) and adrenal (PC12) origin 24 hours after incubation (results are shown as average value normalized to -31 mV average and SD from three technical replicates).
- Figure 8: shows representative single plane fluorescence confocal microscopy images and schematic representation of charge-mediated interactions between giant unilamellar vesicles and A431D cells, 24 hours after incubation and several washing steps. Nuclei (first column) were stained with Hoechst 33342, cell membranes (second column) were stained with WGA-AlexaFluor488 and giant unilamellar vesicles (third column) were visualized by the fluorescent signal of LissRhod-PE lipids incorporated into giant unilamellar vesicles. Respective giant unilamellar vesicle charge is indicated on the left side of the image. Scale bar is 20 µm.

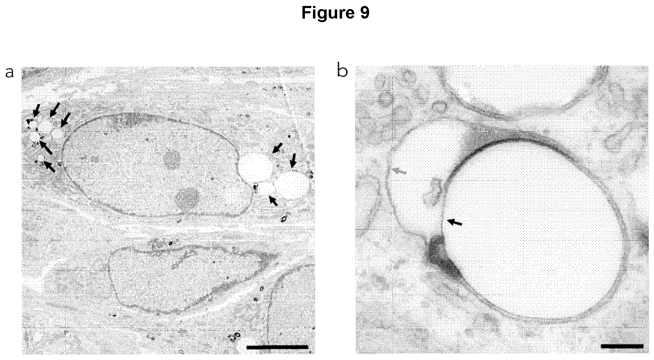

- Figure 9: shows transmission electron microscopy (TEM) of internalized giant unilamellar vesicles. (a) Horizontal TEM overview of a REF52 cell layer incubated for 16 hours with negatively charged giant unilamellar vesicles. Arrows point to giant unilamellar vesicles within the cytoplasm. Scale bar is 2.5 µm. (b) Higher magnification horizontal TEM view of a giant unilamellar vesicle internalized into a cell. Black arrow point on the giant unilamellar vesicle membrane, cyan arrow points to the endosomal membrane. Scale bar is 200 nm.
- Figure 10: shows strategies for giant unilamellar vesicles functionalization. (a) Schematic representation and single plane fluorescence confocal microscopy images of triple-functionalized giant unilamellar vesicles produced by a splitting microfluidic device. The fluorescence corresponds to Biotin-binding Atto425-streptavidin, NTA²⁺-binding Histagged GFP and ammine-binding NHS-Alexa647. (b and c) Schematic representations and microscopy images of the sequential giant unilamellar vesicles functionalization with 50 nm gold nanoparticles in (b) and with antibodies in (c). Right panels in (b) and (c) show phase contrast image of gold nanoparticle (indicated by white arrows) linked giant unilamellar vesicles and fluorescence confocal image of AlexaFluor488-linked Anti-CD3 IgG immobilized via His-tagged ProteinG to NTA²⁺ functionalized lipids, respectively. Scale bars are 1 µm and 2 µm for (b) and (c), respectively

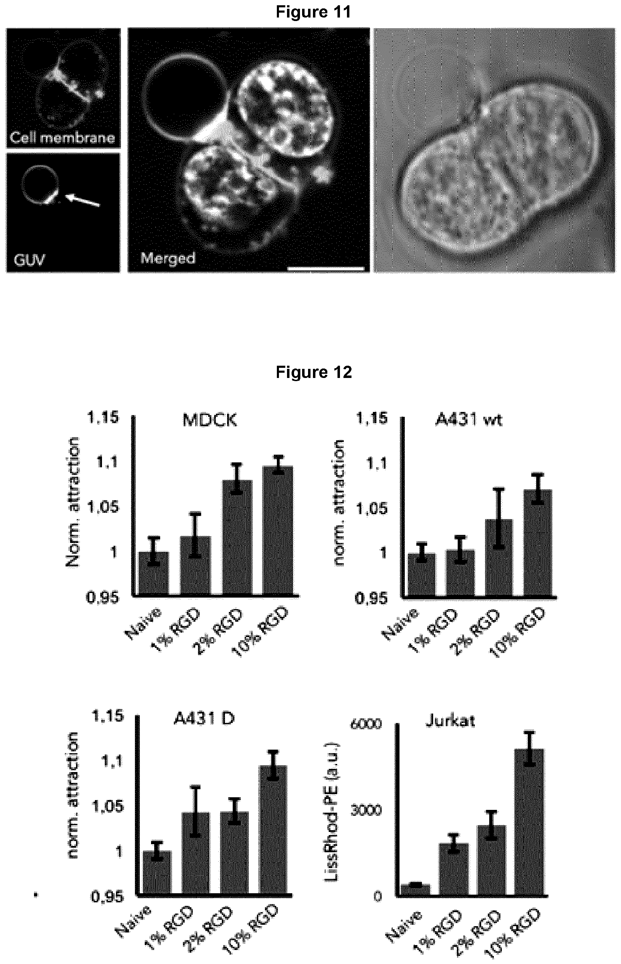

- Figure 11: shows formation of an interaction area between anti-CD3 functionalized giant unilamellar vesicles and CD3⁺ Jurkat T-cells after incubation for 24 hours. Cell membranes were stained with WGA-AlexaFluor488 (upper left image), giant unilamellar vesicles were visualized by incorporation of LissRhod-PE fluorescent lipids (lower left image) and nuclei in composite were stained with Hoechst 33342 (right image). Arrow indicates site of lipid clustering at the giant unilamellar vesicle-cell interface. Right image shows bright field. Scale bar is 6 µm.
- Figure 12: shows attraction analysis of giant unilamellar vesicles functionalized with the RGD peptide at increasing concentrations (1 - 10 %) and incubated with different cell lines. The presented results are normalized to the attraction of naive giant unilamellar vesicles without RGD, and shown as mean value ± SD from three technical replicates. Attraction of RGD-functionalized giant unilamellar vesicles to suspension Jurkat cells was assessed by flow cytometry.

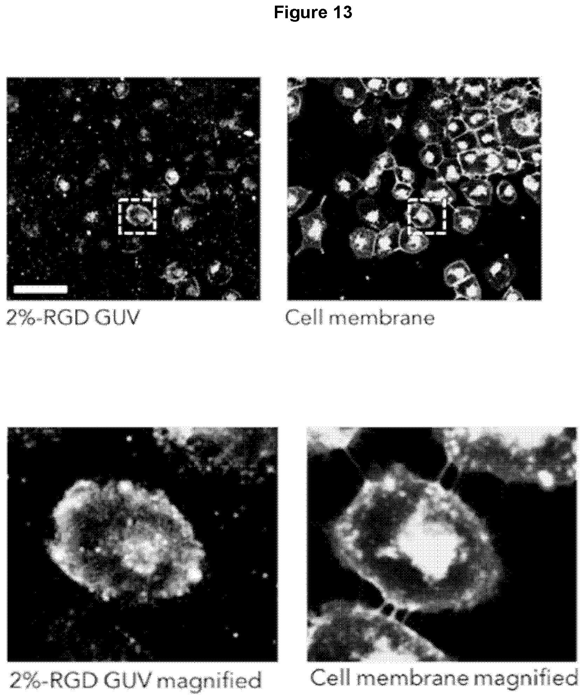

- Figure 13: Representative fluorescence confocal microscopy images of fluorescently labeled giant unilamellar vesicles (LissRhod-PE) decorated with 2 mol% RGD ligands and incubated with membrane stained (WGA-AlexaFluor488) A431D cells. The insets show magnified images with accumulation of giant unilamellar vesicles at the cell periphery and in the perinuclear region observed after incubation for 24 hours. Scale bar is 60 µm.

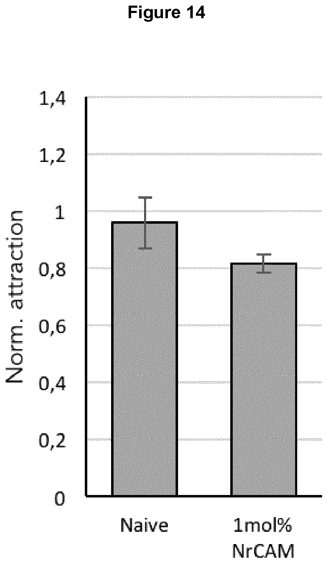

- Figure 14: shows comparison of interactions between cells and giant unilamellar vesicle with or without NrCAM on their surface. Attraction values of naive and recombinant NrCAM functionalized non-charged giant unilamellar vesicles incubated for 24 hours with SH-SY5Y cells. Note that no significant increase in attraction can be achieved by functionalization as non-specific lipid-based interactions appear stronger than specific ligand-receptor based attraction.
- Figure 15: shows PEGylation of giant unilamellar vesicles for giant unilamellar vesicle - cell repulsion. (a) Grey scale representation of Zeta-potentials of small unilamellar vesicles and giant unilamellar vesicles containing different amounts of positively (DOTAP) or negatively (DOPG) charged lipids and decorated with varying amount of PEG of increasing molecular weight. (b) Attraction analysis of giant unilamellar vesicles from a) with six different cell lines.

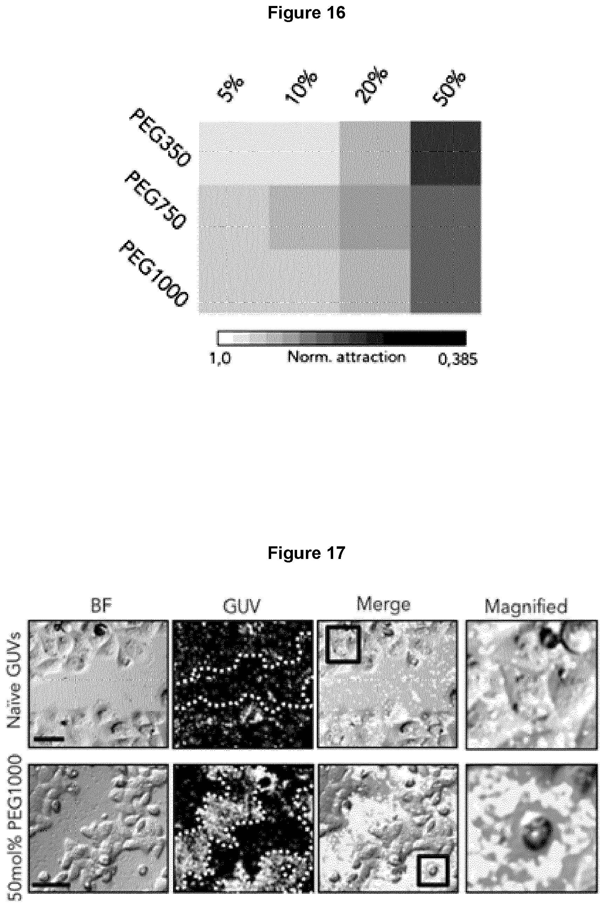

- Figure 16: shows regulation of attractive and repulsive giant unilamellar vesicle-cell interactions. Heat map of the attraction analysis between A431D cells and PEG-functionalized giant unilamellar vesicles containing positively charged lipids at 15 mol%.
- Figure 17: shows representative bright field (first column) and fluorescence (second column) confocal microscopy images of A431 cells interfaced with naive negatively charged giant unilamellar vesicles (upper row) or 50 mol% PEG1000-functionalized negatively charged giant unilamellar vesicles (lower row). Naive giant unilamellar vesicles are in direct contact with the cells while PEGylated giant unilamellar vesicles accumulate in the intercellular space (merged image, third column) and form contact inhibition zones with the cells (fourth column magnified from areas indicated in merged images). White dotted lines indicate periphery of cell groups deduced form the bright field image. Scale bars are 15 µm.

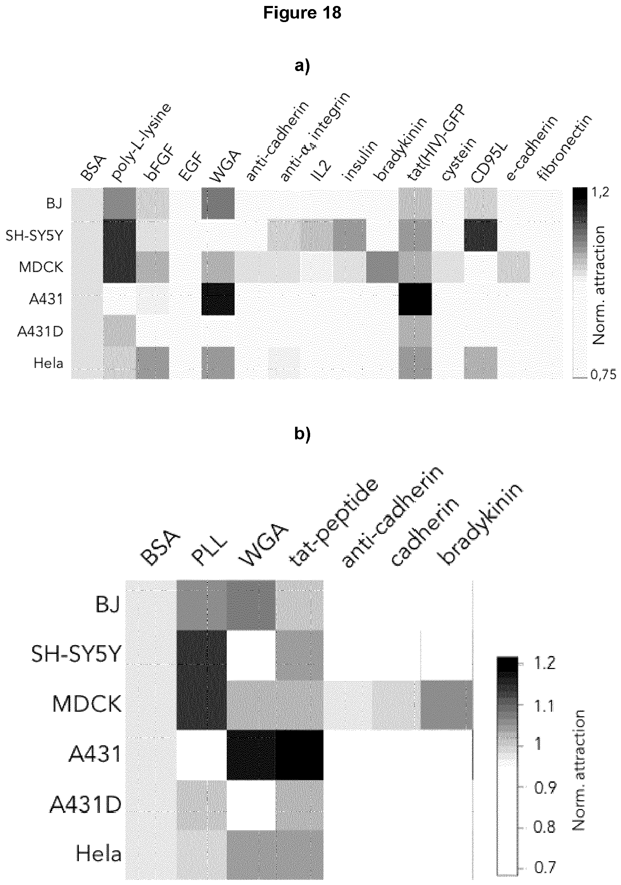

- Figure 18: shows attraction of biofunctionalized giant unilamellar vesicles. a) Attraction values of giant unilamellar vesicles decorated with 15 different peptides and proteins (coupled to the giant unilamellar vesicle surface via 1 mol% NHS lipids) for 6 cell lines. All values were normalized to the attraction of BSA coupled giant unilamellar vesicles. b) Attraction values of non-specific (i.e., BSA, poly-L-lysine (PLL), WGA and tat-peptide) and specific (i.e., anti-cadherin, recombinant cadherin and bradykinin) protein-biofunctionalized giant unilamellar vesicles to human fibroblasts BJ cells, neuroblastoma SH-SY5Y cells, endothelial MDCK cells, epithelial A431 cells, dexamethasone treated epithelial transformed A431 cells and Hela cells. All values were normalized to the attraction of BSA-coupled giant unilamellar vesicles.
- Figure 19: shows schematic representation (upper panel) of PEGylated giant unilamellar vesicles biofunctionalized with NrCAM in co-culture with astrocytes and neuronal cells, differing for the expression of axonin-1 receptor. Lower panel show representative single plane confocal microscopy images of the astrocyte- neuron co-culture following 24 hours of incubation with NrCAM - PEGylated giant unilamellar vesicles. Hs683 astrocytes and SH-SY5Y neurons were stained with CellTracker Green and Blue, respectively. Giant unilamellar vesicles were visualized by incorporation of LissRhod-PE fluorescent lipids. Scale bar is 50 µm.
- Figure 20: shows lysosomal degradation of the vesicles and the assessment of the mechanisms for lysosomal escape to allow intracellular giant unilamellar vesicle cargo release. (a) Representative fluorescence confocal microscopy images of REF52 cells loaded with endosomal entrapped negatively charged giant unilamellar vesicles. Nuclei (upper left image) were stained with Hoechst 33342, endosomes (upper center image) were labeled by staining with WGA-AlexaFluor488 for 24 hours, giant unilamellar vesicles (lower left image) were visualized by incorporation of LissRhod-PE fluorescent lipids and cytoplasm (lower center image) was stained by CellTracker Blue. Merged image (right image) shows that giant unilamellar vesicles reside within the cytoplasm and are entrapped in endosomal vesicles. Scale bar is 10 µm. (b) Representative fluorescence microscopy images of REF52 cells showing colocalization of giant unilamellar vesicles with lysosomal compartments 24 hours after incubation. Lysosomes (left panel) were stained with LysoTracker Green and giant unilamellar vesicles (center panel) were visualized by incorporation of LissRhod-PE fluorescent lipids. Right panel shows bright field (BF) images. Scale bar is 10 µm.
- Figure 21: shows representative fluorescence confocal microscopy images of the assessment of poly-ethylene-imine (PEI)-, GALA peptide- and DOBAQ-mediated lysosomal escape for HPTS-loaded giant unilamellar vesicles incubated with REF52 cells for 24 hours. Scale bar is 50 µm.
- Figure 22: shows dynamic light scattering measurements of giant unilamellar vesicle Zeta-potentials at different pH for giant unilamellar vesicles containing 60mol% of the pH-sensitive lipid DOBAQ or giant unilamellar vesicles with pH-sensitive lipids. Note the transition of the DOBAQ containing giant unilamellar vesicles from negative to positive Zeta-potentials with the decrease of the pH. Results are shown as mean and SD values from three technical replicates.
- Figure 23: shows quantification of intracellular giant unilamellar vesicle degradation. A431D cells were incubated with giant unilamellar vesicles and the giant unilamellar vesicles were quantified over time. While "empty" giant unilamellar vesicles show prompt degradation after uptake, giant unilamellar vesicles loaded with PEI show progressive intracellular accumulation indicating successful escape from lysosomal degradation.
- Figure 24: shows representative fluorescence confocal microscopy and bright field images of primary mouse hippocampal neurons (right panel) incubated with giant unilamellar vesicles (left panel, visualized by incorporation of 1mol% LissRhod PE lipids) loaded with HPTS (center panel). Scale bar is 30 µm.
- Figure 25: shows delivery of heavy duty cargos by the giant unilamellar vesicles. (a) Representative bright field (left panel) and corresponding confocal microscopy (right panel) images of baculovirus-loaded giant unilamellar vesicles. Scale bar is 10 µm. (b) Maximal z-projection of fluorescence confocal microscopy images of REF52 cells (nuclei stained with Hoechst 33342, upper row left image) incubated with baculovirus (lower row left image, by oversaturation of Hoechst 33342 channel) containing DOBAQ carrier giant unilamellar vesicles (upper row right image) for 24 hours. Note expression of mitochondria targeted dsRed (lower row right image). Scale bar is 25 µm.
- Figure 26: Microfluidic device comprising the generation zone of the parent polymer shell stabilized vesicle, and the splitting units to generate smaller giant unilamellar vesicles of diameter smaller than 10 µm. (1): Oil phase inlet, introducing the oil phase to the device; (2): optional oil filter structure, for retention and filtering of large contaminants like dust; (3): aqueous phase inlet, introducing the aqueous phase(s) into the devices. Optionally, more than one single inlet can be used (here two separate inlets); (4): optional aqueous phase filter structure for large contaminants, for retention and filtering of large contaminants like dust; (5): for multiple aqueous inlets: aqueous inlets junction. Merges the separate aqueous inlets, if only one aqueous inlet is used, not such structure is needed. (6): Flow focusing junction (example of a "Generation Zone", area of formation of the parent polymer shell stabilized giant unilamellar vesicle. (7): splitting architecture. Sequential mechanical splitting of the parent droplet ("Splitting Unit"). (8): Stabilization plane. Wide area where splitted droplets can stabilize and mix, here the droplets are surrounded by excess oil and enough surfactant is provided to stabilized the small droplets (as splitting leads to a surfaces increase wherefore more surfactant is needed; (9): Outlet channel, leeds the droplets to the outlet; (10): Outlet, where splitted and stabilized polymer shell stabilized giant unilamellar vesicles exit the device.

The following examples are included to demonstrate preferred embodiments of the invention. It should be appreciated by those of skill in the art that the techniques disclosed in the following examples represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the scope of the invention as described in the following claims.

### Examples

### Materials

EggPG L-α-phosphatidylglycerol (Egg, Chicken), EggPC L-α-phosphatidylcholine (Egg, Chicken), 18:1 DOPG 1,2-dioleoyl-sn-glycero-3-phospho-(1'-rac-glycerol), 18:1 DOPC 1,2-dioleoyl-sn-glycero-3-phosphocholesteroline, 18:1 DOPE 1,2-dioleoyl-sn-glycero-3-phosphoethanolamine, LissRhod PE 1,2-dioleoyl-sn-glycero-3-phosphoethanolamine-N-(lissamine rhodamine B sulfonyl), 18:1 DGS-NTA(Ni) 1,2-dioleoyl-sn-glycero-3-[(N-(5-amino-1-carboxypentyl)iminodiacetic acid)succinyl] (nickel salt), 18:1 DOTAP 1,2-dioleoyl-3-trimethylammonium-propane, 18:1-12:0 Biotin PE 1-oleoyl-2-(12-biotinyl-(aminododecanoyl))-sn-glycero-3-phosphoethanolamine, DSPE-RGD 1,2-distearoyl-sn-glycero-3-phosphoethanolamine-N-[4-(p-(cysarginylglycylaspartate-maleim idomethyl)-cyclohexane-carboxamide], 18:1 PEG350 PE 1,2-dipalmitoyl-sn-glycero-3-phosphoethanolamine-N-[methoxy(polyethylene glycol)-350], 18:1 PEG750 PE 1,2-dioleoyl-sn-glycero-3-phosphoethanolamine-N-[methoxy(polyethylene glycol)-750], 18:1 PEG1000 PE 1,2-dioleoyl-sn-glycero-3-phosphoethanolamine-N-[methoxy(polyethylene glycol)-1000] and extrude set with 50 nm pore size polycarbonate filter membranes were purchased from Avanti Polar Lipids, USA. All lipids were stored in chloroform at -20 °C and used without further purification. DyLight 405 NHS Ester, AlexaFluor647-NHS, anti CD3 (16-0038-81), anti CD3-Alexa488 (53-0037-42), Hydroxypyrene-1,3,6-trisulfonic acid trisodium salt (HPTS), Hoechst 33342, CellTracker Blue CMAC dye, CellTracker Green CMFDA dye, LysoTracker Green DND-26 dye, wheat germ agglutinin (WGA)-AlexaFluor conjugates, Dulbecco's Modified Eagle Medium (DMEM) high Glucose, 1:1 DMEM:F12, RPIM-1640, FluoroBrite DMEM (high glucose), heat inactivated fetal bovine serum, penicillin-streptomycin (10,000 U/mL), GlutaMax Supplement, L-Glutamine (200 mM), trypsin-EDTA (0.05 %) with phenol red, phosphate buffered saline, Basic fibroblast growth factor (aminoacids 10 - 155), Epithermal growth factor and AlexaFluor405 dye were purchased from Thermo Fischer Scientific, Germany. NHS Palmitic acid N-hydroxysuccinimide ester, 97 % L-cysteine, 50 nm Au nanoparticles, heat-inactivated horse serum, Lectin (Wheat Germ Agglutinin), 1H, 1H,2H,2H-perfluoro-1-octanol (PFO) de-emulsifier, Bradykinin, polyethylenimine (branched, Mw ∼25,000), Atto425-Biotin, human Interleukin 2, recombinant Insulin, fibronectin from bovine plasma, Poly-L-lysine and DOBAQ N-(4-carboxybenzyl)-N,N-dimethyl-2,3-bis(oleoyloxy)propan-1-aminium were purchased from Sigma Aldrich, Germany. Polydimethylsiloxan (PDMS) Sylgard 184 was purchased from Dow Corning, USA. Protein G His-tag was purchased from BioVision, USA. Bovine Albumin fraction V (BSA) was purchased from Carl Roth, Germany. His-tagged NrCAM 8425-NR-050 and human recombinant cadherin was purchased from was purchased from R&D Systems, USA. Fluoresbrite YG Microspheres 1.00 µm were purchased from Polysciences Europe, Germany. Perfluoropolyether-polyethylene glycol (PFPE-PEG) block-copolymer fluorosurfactant was purchased from Ran Biotechnologies, USA. Anti-VE-Cadherin and anti-alpha4-integrin (CD49d) antibodies were purchased from Santa Cruz (Sc-28644) and Millipore (MAB1383). Recombinant human CD95L was purchased from BioLegend, USA. A431, Hela, Hs683, SH-SY5Y and Jurkat cell lines were obtained from ATCC, USA. REF52 cell lines were a generous gift from Prof. Benjamin Geiger (Weizmann Institute Rechovot). PC12 cells were a generous gift from Amin Rustom (Institute for Neurobiology, Heidelberg). Primary mouse hippocampal neurons were obtained from the Institute of Neurobiology, Interdisciplinary Center for Neurosciences in Heidelberg, Germany. Purified Baculovirus were obtained from Martin Pelosse (Commissariat a l'énergie atomique et aux energies alternatives, France), and were produced as described in Mansouri et al., 2016, Highly efficient baculovirus-mediated multigene delivery in primary cells, *Nature Communications.* Tat-HIV-GFP peptides were a generous gift from Rüdiger Arnold (Life Science Lab, German Cancer Research Center).

### Microfluidic-based production of giant unilamellar vesicles

For the production of polymer shell stabilized giant unilamellar vesicles (also referred to as "water-in-oil droplets" or only "droplets") a solution of small unilamellar vesicles with lipid compositions as given in table 4 was prepared. Briefly, lipids dissolved in chloroform were mixed at receptive ratios in glass vials and dried under a gentle nitrogen stream. The dried lipid films were rehydrated to a final lipid concentration of 3 mM in production buffers given in table 4 for 30 min. Subsequently, the solution was shaken for 5 min at more than 600 rpm. The resulting liposome solution was extruded at least 9 times through 50 nm pore size polycarbonate filter. Small unilamellar vesicle solutions were stored at 4 °C for up to 3 days or used for polymer shell stabilized giant unilamellar vesicle production immediately.

Droplet-based microfluidic mechanical splitting devices were fabricated using poly(dimethylsiloxane) (PDMS). The complete devices were produced as previously described using photo- and soft-lithography methods (Soft Lithography, Xia Y and Whitesides, GM, 1998). Flow rates were controlled by a Elveflow OB1 MK3 - microfluidic flow control system. If not stated otherwise, for the formation of giant unilamellar vesicles within microfluidic droplets, the small unilamellar vesicle solutions were diluted to a final lipid concentration of 1.5 mM. For droplet formation, the small unilamellar vesicle solutions were introduced into the aqueous channel of the microfluidic devices. Negatively charged giant unilamellar vesicles were formed using 1.25 mM PFPE(7000 g/mole)-PEG(1500 g/mole)-PFPE(7000 g/mole) triblock surfactant dissolved in FC-40. Positively charged giant unilamellar vesicles were formed using 0.5 % PEG-based fluorosurfactant diluted in FC-40 (cat#: 008-FluoroSurfactant-1 G, RAN Biotechnologies). For formation of giant unilamellar vesicles containing DOBAQ lipids for lysosomal escape, 1.25 mM PFPE(2500 g/mole)-PEG(600 g/mole)-PFPE(2500 g/mole) triblock surfactant diluted in FC-40 was used. A ratio of the aqueous to oil phase of approximately 1:4 was used. Droplets were formed at the flow-focusing junction of the splitting device and collected from the outlet of the microfluidic chip into a microtube. Following the collection, polymer shell stabilized giant unilamellar vesicles were allowed to equilibrate for minimum 2 hours at 4 °C before the release.

### Release of giant unilamellar vesicles from the polymer shell

For the release of the giant unilamellar vesicles into an aqueous buffer, following the formation of polymer shell-stabilized giant unilamellar vesicles, excess oil phase was removed from the microtube and the layer of polymer shell stabilized giant unilamellar vesicles was mixed with the destabilizing agent PFO, added at a ratio 1 : 1 in the respect of the aqueous production "intraluminal" buffer (PBS, water, or DMEM). Thereafter, a volume of release buffer, also at a ratio 1 : 1 in the respect of the aqueous production "intraluminal" buffer, was added as a single drop or layer to the collected polymer shell stabilized giant unilamellar vesicles. The respective separated layers were mixed by gentle agitation of rotation of the collection tube.
Following 30 min of equilibration, the aqueous phase containing giant unilamellar vesicles was transferred into a 2 ml microtube.
Finally, a volume of release buffer was added at a ratio 1 : 1 in the respect of the aqueous production buffer and the released giant unilamellar vesicles were centrifuged at >10.000 g for 15 min. The supernatant was discarded and pellet was suspended to the desired concentration.
The release buffer is preferably the same buffer used as production buffer (also named "intraluminal" buffer), which is encapsulated by the polymer shell-stabilized giant unilamellar vesicles.

Table 6 reports the lipid compositions, functionalizations and buffers used in the Examples of the present invention.

### Dynamic light scattering

Zeta potentials of giant unilamellar vesicles and original small unilamellar vesicles were measured with a Malvern Zetasizer Nano ZS system at a total lipid concentration of 15 µM in PBS. Equilibration time was set to 600 s at 25 °C, followed by three repeat measurements for each sample at a scattering angle of 173° using the in-build automatic run-number selection. Material refractive index was set to 1.4231 and solvent properties to η = 0.8882, n = 1.33 and ε = 79.0. For giant unilamellar vesicle zeta-potential measurements, equilibration time and number of individual measurements were set to 120 s and 2 repeats, respectively. Zeta-potential of DOBAQ containing giant unilamellar vesicles was assessed by diluting giant unilamellar vesicles to a final lipid concentration of 15 µM in PBS solution adjusted to desired pH with 4 N NaOH or 10% HCI. All zeta-potential measurements were performed at least in duplicates.

### Assessment of droplet homogeneity

To assess transmission heterogeneity of intraluminal droplet contents, water-in-oil droplets were produced at a flow focusing junction of a droplet splitting device. PBS aqueous phase containing 10 mM MgCl₂, 1 mM AlexaFluor405, 1 µM His-tagged GFP, 1.08x109 particles/ ml Fluoresbrite YG Microspheres (diameter = 1.00 µm) was used. Droplets were collected and mean droplet fluorescence intensity for all fluorophores was measured from single plane fluorescence confocal images using global thresholding segmentation and the Particle analyzer tool of ImageJ software.

### Quantitative Mass Spectrometry (MS)

For quantitative mass spectrometry analysis of original small unilamellar vesicle and giant unilamellar vesicle lipid content, giant unilamellar vesicles were produced from the small unilamellar vesicle composed of 33 mol% DOTAP, 33 mol% DOPE, 33 mol% DOPC, 1 mol% LissRhod PE with 0.5 % RAN Biotechnologies PEG-based fluorosurfactant diluted in FC-40. Relative quantitative mass spectrometry was performed using a Sciex QTRAP 4500 mass spectrometer hyphenated with a Shimadzu Nexera HPLC system. The instruments were controlled via Sciex Analyst 1.7 software. Samples were diluted 1 to 1000 in MeOH and subsequent, fractionation was performed using a Supelco Titan C18 column (0.21 x 10 cm, 1.9 µ) operated at 45 °C. The isocratic method featured a flow rate of 0.5 ml/ min using a 10 mM NH4Ac solution in 98 % MeOH. The MS experiments were performed in multiple reaction monitoring (MRM) mode using the following instrument settings: curtain gas 35 psi, ionization voltage 5500 V, nebulizer gas 30 psi, heater gas 60 psi, heater temperature 180 °C and a CAD gas set to 9. The following compound specific parameters were used:

| **ID** | **Precursor mass [Da]** | **Fragment mass [Da]** | **Dwell time [msec]** | **Declustering potential [V]** | **Collision Energie [V]** | **Cell exit potential [V]** |
|---|---|---|---|---|---|---|
| DOPE | 744.498 | 603.500 | 110 | 91 | 33 | 16 |
| DOTAP | 662.528 | 603.500 | 50 | 166 | 41 | 20 |
| DOPC | 786.528 | 184.000 | 50 | 161 | 39 | 14 |
| LissRhod | 1301.605 | 682.000 | 110 | 40 | 67 | 24 |

Data analysis was performed using Sciex Analyst 1.7 and MultiQuant 3.0.2 software. Calculated concentrations were normalized using small unilamellar vesicle sample with the following initial lipid ratios DOPE 33 / DOTAP 33/ DOPC 33/ Liss Rhod PE 1.

### Quantification of release efficiency and stability

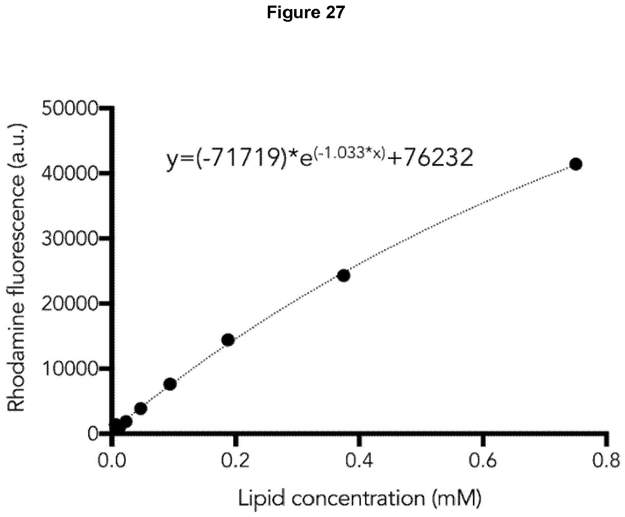

Release efficiency and mechanical stability of the giant unilamellar vesicles after agitation was assessed by manually counting the polymer shell stabilized giant unilamellar vesicles and the released giant unilamellar vesicles with a Neubauer chamber mounted on a fluorescence microscope. Total lipid concentration of released and purified giant unilamellar vesicles was quantified by measuring giant unilamellar vesicle solution fluorescence (Figure 27). Respective fluorescence was normalized to a standard small unilamellar vesicle dilution curve (fitted to one phase exponential decay) of known concentration and with equal ratio of fluorescently labeled lipids. For incubation of giant unilamellar vesicles with cell lines, total lipid concentrations between 1.5 µM - 50 µM were used.

### Quantification of lysosomal degradation

For quantification of lysosomal degradation of giant unilamellar vesicles, A431 D monolayers were incubated with fluorescently labeled giant unilamellar vesicles and monitored by live cell fluorescence time-lapse microscopy. Total giant unilamellar vesicle number in the field of view was counted by global threshold segmentation and the ImageJ build-in particle analyzer plug-in for each time frame.

### Cell culture

Fibroblast REF52 cells, endothelial MDCK cells, epithelial A431 and A431D cells, cervical cancer Hela cells and astrocytes Hs683 cells were cultured in Dulbecco's Modified Eagle Medium supplemented with 4.5 g/l glucose, 1 % L-glutamine, 1 % penicillin/ streptomycin and 10 % fetal bovine serum. Neuroblastoma SH-SY5Y cells were cultured in a 1:1 mixture of F12:DMEM supplemented with 1 % L-glutamine, 1 % penicillin/streptomycin and 10% fetal bovine serum. Human T-lymphocyte Jurkat cells were cultured in RPMI-1640 medium supplemented with 1 % penicillin/ streptomycin and 10 % fetal bovine serum. Adrenal PC12 cells were cultured in RPMI-1640 medium supplemented with 1% L-glutamine, 1 % penicillin/streptomycin 5% fetal bovine serum and 10% heat-inactivated horse serum. Cell cultures were routinely cultured at 37 °C and 5 % CO₂ atmosphere and passaged at approx. 80 % confluency using 0.05 % trypsin/EDTA treatment. Jurkat cells were passaged by diluting 1 ml of Jurkat culture to 4 ml of fresh culture medium.

### Cell staining

Live cells were stained with Hoechst 33342 at a final concentration of 5 µg/ml to visualize cell nuclei. Cytoplasms were stained with CellTracker Blue CMAC dye and CellTracker Green CMFDA dye following the manufactures instructions. Lysosomes were stained with LysoTracker Green DND-26 dye following manufacturer's instructions. Cell membranes were stained with wheat germ agglutinin (WGA)-AlexaFluor conjugates. Membranes were stained by adding WGA conjugates to a final concentration of 50 µg/ml to cells grown in fully-supplemented growth medium for 10 min at room temperature. To reduce endocytotic dye uptake, stained cells were handled for imaging at room temperature. WGA is known to stain also endosomal and golgi-associated vesicles. To stain endosomal giant unilamellar vesicle uptake, cells incubated with giant unilamellar vesicles were incubated with a final concentration of 5 µg/ml WGA-AlexaFluor conjugates for 24 hours.

### Confocal microscopy and live cell imaging

For fluorescence confocal microscopy observations, cell lines were cultured in 8-well Nunc LabTeK glass bottom culture slides filled with at least 400 µl of culture medium. Confocal microscopy was performed with a laser scanning microscope LSM 800 (Carl Zeiss AG). Images were acquired with a 20x (Objective Plan-Apochromat 20x/ 0.8 M27, Carl Zeiss AG) and a 63x immersion oil objective (Plan-Apochromat 63×/ 1.40 Oil DIC, Carl Zeiss AG). Images were analyzed with ImageJ (NIH). Adjustments of image brightness and contrast or background correction was performed always on the whole image and special care was taken not to obscure or eliminate any information from the original image. For images with speckled noise signals, 2 pixel median filters were applied. For cell fixation prior to confocal microscopy analysis, cell cultures were washed twice with PBS and subsequently fixed with 2 - 4 % PFA for at least 20 min. For time-lapse live cell imaging a Leica DMi8 inverted fluorescent microscope equipped with a sCMOS camera and 10x HC PL Fluotar (NA 0.32, PH1) objective was used. Cells were cultured in 8-well Nunc LabTeK glass bottom culture slides in FluoroBrite DMEM (high glucose) medium supplemented with GlutaMAX, 10 % FBS and 1 % Pen/Strep.

### Transmission electron microscopy

REF52 cells incubated for 16 hours with giant unilamellar vesicles were fixed in 2.5% glutaraldehyde dissolved in a 0.1 M Na₃PO₄ solution for 30 min at room temperature. Cells were further fixed in 0.4% uranyl acetate overnight. Fixed cells were subsequently dehydrated with a 50 %, 60 %, 70%, 80 %, 90 %, and 100 % ethanol series and embedded in resin over night at 60 °C. 85 nm ultrathin sections were prepared and contrasted with lead acetate or osmium tetroxide. A Zeiss EM 10 CR transmission electron microscope was used for imaging. If necessary, image contrast, brightness and sharpness were adjusted using the build-in ImageJ plug-ins.

### Flow cytometry

For flow-cytometry analysis of the attraction between RGD functionalized giant unilamellar vesicles and Jurkat cells, rhodamine B-giant unilamellar vesicles with varying RGD density (0, 1, 2, 10 mol%, see table 6) were incubated with Jurkat cells for 24 hours. Cell were subsequently centrifuged for 5 min at 250 g and the supernatant containing non-bound or non-uptaken giant unilamellar vesicles was discarded. Cells were resuspended in fresh culture medium and for each condition, giant unilamellar vesicle fluorescence within the cells was quantified with a BD LSR Fortessa Cell Analyzer (BD Bioscience) using the blue laser line in the PE channel (λ_{em max}= 575). From the acquisition of the scattered parameters, a gate was set to discriminate between Jurkat cells, debris and possible clumps. Subsequentially, singlet cells were identified from the aforementioned Jurkat population. Lastly, based on this gating strategy, fluorescence intensity associated with the cells periphery was recorded and quantified.

### Attraction assay

Cells were seeded in triplicates in 100 µl of their corresponding growth media to form a confluent monolayer after 24 h of incubation in 96 flat-bottom well-plates. Giant unilamellar vesicle (labeled with LissRhod PE lipids) solutions were added to a final lipid concentration of 1.5 µM and incubated for 24 h. Fluorescence of each well was measured using an Infinite M200 TECAN plate reader controlled by TECAN iControl software with in-build gain optimization and excitation/emission setting adjusted to 550/585 nm, respectively. Subsequently, wells were washed 3 times with 100 µl PBS using a multichannel pipette and residual fluorescence was measured again. Fluorescence intensity after washing was normalized to intensity before washing in order to account for any variation in sample preparation. All samples were measured in triplicates at 4 individual position/ well in order to account for variation in cell monolayer density. For comparison of specific attraction of biofunctionalized giant unilamellar vesicles, all attraction values were normalized to the attraction of BSA functionalized giant unilamellar vesicles and the respective cell line in order to reference all attraction values for each cell type to a common moderately non-reactive protein.

A total lipid concentration of 3 µM of anti-CD3 functionalized giant unilamellar vesicles and Jurkats cells were incubated for 24 hours in order to perform fluorescence confocal microscopy observation of the attraction and formation of contact sides. Prior to imaging, cells were stained with Hoechst 33342 and WGA-AlexaFluor647 as described above.

### Quantification of preferential giant unilamellar vesicle uptake in co-culture

For quantification of preferential giant unilamellar vesicle uptake in SH-SY5Y/Hs683 co-culture experiments, SH-SY5Y and Hs683 cell were separately stained with CellTracker Blue CMAC (and CellTracker Green CMFDA, respectively. Cells were co-seeded in a 10:1 SH-SY5Y:Hs683 ratio in a 1:1 mixture of F12:DMEM supplemented with 1 % L-glutamine, 1 % penicillin/ streptomycin and 10% fetal bovine serum together with giant unilamellar vesicles composed of 20 mol% PEG750 PE, 20 mol% EggPG, 58 mol% EggPC, 1 mol% LissRhod PE and 1mol% palmitic acid NHS coupled with 1.5 µM His-tagged recombinant NrCAM for 24 hours. Cell cultures were subsequently washed 3 times with PBS and fixed for 20 min with 4% PFA followed by fluorescence confocal microscopy analysis with appropriate laser excitation. Total area of SH-SY5Y and Hs683 cells in a field of view was calculated from single plane confocal images. Total number of giant unilamellar vesicles in respective areas was determined by global threshold segmentation and subsequently normalized to the total cell area. For example, total area of SH-SY5Y and Hs683 cells shown in Figure 19 is 20083.37 µm² and 40741.67 µm², respectively. They contain 1491 (= 0.0742 giant unilamellar vesicles/ µm²) and 578 (= 0.0141 giant unilamellar vesicles / µm²) giant unilamellar vesicles, respectively, which corresponds to an increase of 523% for SH-SY5Y cells.

### Encapsulation of baculoviruses

For intracellular delivery of baculoviruses (BVs) encoding a mitochondrial targeted dsRed (Discosoma red) fluorescent protein, solutions containing BVs were mixed in a 1:100 ratio with the small unilamellar vesicle solution. Importantly, for polymer shell stabilized giant unilamellar vesicle production, PBS with 60 mM MgCl₂ was used, as lower manganese concentrations inhibited polymer shell stabilized giant unilamellar vesicle formation and release rates. Released and purified giant unilamellar vesicles containing baculoviruses were then incubated with REF52 cells for 24 h and subsequently stained with 8 µg/ml Hoechst33342. Expression of mitochondrial targeted dsRed protein and baculovirus localization was assessed my fluorescence confocal microscopy. For visualization of intracellular baculoviruses staining of baculovirus-DNA was imaged by overexposing the Hoechst33342 channel.

### Giant unilamellar vesicle's functionalization and PEGylation

Functionalization of giant unilamellar vesicles was always performed on released giant unilamellar vesicles. If not stated otherwise, giant unilamellar vesicle's functionalization was carried in PBS and in the dark on a horizontal shaker at room temperature. After functionalization, giant unilamellar vesicles were centrifuged at >10.000 g for at least 15 min and resuspended in PBS. For NHS based coupling reactions, giant unilamellar vesicles were kept whenever possible at 4°C and released from droplets not later than 1 hour after production. NHS coupling reactions were performed for at least 3 hours. For NHS and NTA based functionalizations, respective proteins and peptides were added in 2 - 5 fold excess to total functionalized lipids as calculated from the total lipid concentration. For example, fluorescence quantification showed total lipid concentration of 150 µM (corresponding to approx. release efficiency of 10 %) and giant unilamellar vesicles were produced from small unilamellar vesicles with 1 mol% palmitic acid-NHS lipids, a minimum of 1.5 µM of the protein to be coupled was added (about 50 % of the NHS coupled lipids would reside within the inner membrane leaflet and not be accessible for coupling). In case of the WGA coupling, the lectin was coupled to the giant unilamellar vesicle in 10 times less molar concentration compared to the presented 5 mol% NHS ligand. Functionalization of giant unilamellar vesicles with RGD peptides was performed by introducing a desired amount of DSPE-RGD into the lipid mixture for small unilamellar vesicle production.

For the sequential functionalization of giant unilamellar vesicles with gold nanoparticles (AuNP), giant unilamellar vesicles containing 1 mol% NHS were incubated with 3 µM L-cysteine for 6 hours. Subsequently, 50 nm Au nanoparticles were added to a final concentration of 10 µg/ml and shaken at 300 rpm overnight. Functionalization of giant unilamellar vesicles with IgG antibodies was performed by incubating 3 µM His-tag Protein G to giant unilamellar vesicles containing 1 mol% 18:1 DGS-NTA(Ni) lipids for 1 hour. Subsequently, a final concentration of 3 µM of respective IgG dissolved in 1 % BSA was added to mixture and incubated for 1 hour. In order to avoid cross reactions, whenever multiple functionalizations based on different coupling reactions were performed, as in the case of triple functionalization, proteins to be coupled via NHS were incubated with the giant unilamellar vesicles first before performing the other couplings, e.g. biotin-streptavidin coupling.

PEGylation of giant unilamellar vesicles with poly-ethylene-glycol polymers was performed by introducing a desired amount of PEG350-PE, PEG750-PE or PEG1000-PE into the lipid mixture for production of the initial small unilamellar vesicles.

### Lysosomal escape mechanisms

For all tested lysosomal escape approaches, polymer shell stabilized giant unilamellar vesicles were produced in PBS + 10 mM MgCl₂ + 50 mM 8-Hydroxypyrene-1,3,6-trisulfonic acid trisodium salt (HPTS) using 1.25 mM triblock PFPE-PEG-PFPE 2500-600-2500 surfactant dissolved in FC-40. Released giant unilamellar vesicles were purified by centrifugation and incubated with REF52 cells for 24 hours. Intracellular HPTS fluorescence distribution was subsequently assessed by fluorescence confocal microscopy (Excitation 460nm, Emission 510nm). Imaging parameters were kept constant when comparing between the different approaches. For analysis of lysosomal escape via poly-ethylene-imine (PEI) proton sponge mechanism, giant unilamellar vesicles composed of 20 mol% EggPG, 79 mol% EggPC and 1 mol% LissRhod PE were loaded during droplet production with 44 µg/ml poly-ethylene-imine. For analysis of lysosomal escape via DOBAQ mediated intralysosomal fusion, giant unilamellar vesicles composed of 1 mol% LissRhod PE, 60 mol% DOBAQ, 20 mol% EggPG, and 19 mol% EggPC were produced.

### Example 1. Microfluidic mechanical splitting of polymer shell stabilized giant unilamellar vesicles.

Polymer shell stabilized giant unilamellar vesicles ("water-in-oil-droplet") with size below 5 µm were obtained using a self-developed droplet-based microfluidic device consisting of a flow focusing junction for water-in-oil-droplet production and a multi-Y-shaped microfluidic droplet splitting unit (Figure 1). After production of a parent water-in-oil-droplet with a 60 µm diameter, the splitting unit design allowed for high-throughput mechanical droplet splitting in up to five consecutive division steps creating droplets with a final diameter of 2.90 µm ± 0.45 µm (n = 202).

To assess transmission heterogeneity of intraluminal contents from the mother to the daughter droplets, fluorescence confocal microscopy was used to analyze droplet-entrapped fluorescent content and respective signal intensity distribution among droplets before and after splitting (Figure 2). The results revealed only little inter-droplet variation of signal intensity for the low molecular weight fluorophore AlexaFluor 405 before splitting (Coefficient of variation (CV) before = 5.6 %, n = 29) and after splitting (CV after = 10.7 %, n = 665), green fluorescent protein (CV before = 18.5 %, n = 29 and CV after = 14.8 %, n = 665) and 100 nm fluorescently labelled small unilamellar vesicles composed of 20 mol% EggPG, 79 mol% EggPC and 1 mol% LissRhod PE (CV before = 68.0 %, n=29 and CV after = 15.4 %, n = 665). Based on this, it could be concluded that luminal composition of splitted droplets resembles the composition of the mother droplets, showing that the mechanical splitting approach is suitable for the controlled production of small water in oil-droplets with defined and tunable compositions at high production rates (e.g. 2.5x10⁵ at the focusing T-junction and 8x10⁶ droplets/min after five-fold mechanical splitting, respectively). Importantly, peripheral distribution of lipid fluorescence in the divided droplets was observed, suggesting successful mechanical division of the polymer shell stabilized giant unilamellar vesicles.

### Releasing of the giant unilamellar vesicles from the polymer shell

Following addition of destabilizing surfactants to the collected mechanically-splitted polymer shell-giant unilamellar vesicles (see Methods), the inventors were able to release large quantities of giant unilamellar vesicles (diameter = 1.400 µm ± 0.202 µm, n = 122) into an aqueous phase (Figure 3). A concentration of 1.5 mM was found to be the optimal required concentration for the initial solution of small unilamellar vesicles, achieving over 50% release efficiency of giant unilamellar vesicles, corresponding to a successful giant unilamellar vesicles release from approximately every second droplet (Figure 4). Therefore, using optimized conditions, this method allows for a production rate of giant unilamellar vesicles of approximately 4x10⁶ giant unilamellar vesicles/min.

### Symmetric droplet splitting

A full control over the physicochemical and biological properties of giant unilamellar vesicles is a pivotal requirement for biomedical and synthetic biology applications. Therefore, mass spectrometry (see Method section) was used to assess quantitatively the lipid composition of the formed polymer shell stabilized giant unilamellar vesicles. The results revealed that the lipid ratio of splitted giant unilamellar vesicles resembles that of the initial small unilamellar vesicles used during production of the parent polymer shell stabilized giant unilamellar vesicle and hence no lipid ratio change occurs during microfluidic handling and mechanical droplet splitting (Table 3 below).

**Table 3.**

| Quantitative mass spectroscopy (MS) was performed on small unilamellar vesicles containing DOPE, DOTAP, DOPC and LissRhod PE lipids and splitted giant unilamellar vesicles produced from these small unilamellar vesicles. Results are shown as LissRhod PE- normalized values). No considerable loss of specific lipid subsets was found. MS experiments were performed in triplicate. The coefficients of variation (CV) for the small unilamellar vesicle sample were <7.5 %. CVs for the giant unilamellar vesicle samples was <10 % | | | | |
|---|---|---|---|---|
| Lipid Concentration | DOPE | DOTAP | DOPC | LissRhod PE |
| Concentration before division | 33.00 | 33.00 | 32.99 | 0.99 |
| Concentration ratio *versus* LissRhodPE | 33.22 | 33.22 | 33.21 | 1.00 |
| Concentration after division | 21.49 | 21.10 | 21.95 | 0.65 |
| Concentration ratio *versus* LissRhodPE | 32.98 | 32.38 | 33.68 | 1.00 |
| Ratio change | 0.73 | 2.53 | -1.39 | 0.00 |

### Mechanical stability

Additionally, a basic assessment of the mechanical stability of formed giant unilamellar vesicles revealed that approximately 90% of the giant unilamellar vesicles tolerate incubation at 37°C and mechanical agitation on a horizontal shaker at 800 rpm for 24h. This suggests that they can sustain considerable mechanical stress and are therefore potentially robust enough for drug delivery applications (Figure 5).

### Release of the giant unilamellar vesicles and maintenance under physiological conditions

To successfully interface giant unilamellar vesicles with living cells, it is of major importance that the giant unilamellar vesicles can be produced and maintained under physiological buffer conditions. Therefore, release efficiency of mechanically splitted giant unilamellar vesicles in serum supplemented culture medium was systematically assessed using vesicles filled with serum supplemented cell culture medium. Similarly to PBS and water, it was obtained up to 45% release efficiency in cell culture medium (Table 4 below). Following the release, giant unilamellar vesicles were incubated with rat embryonic fibroblast (REF52) in cell culture. Importantly, time-lapse microscopy analysis showed that giant unilamellar vesicles remained stable during 20 h of incubation (Figure 6).

**Table 4: Quantification of release efficiencies of giant unilamellar vesicles for different combinations of intraluminal (production buffer) and extraluminal release buffers.**

| **Production buffer** | **release Buffer** | | |
|---|---|---|---|
| | **Water (eff %)** | **PBS (eff %)** | **DMEM (eff %)** |
| **Water** | 17 % | 13 % | 3 % |
| **PBS** | 5 % | 5 % | 4 % |
| **DMEM** | 4 % | 5 % | 45 % |

### Example 2. Fine-tuning of charge-mediated giant unilamellar vesicle - cell interactions.

Charge-mediated interaction might serve, if precisely controlled, as a potent instrument to guide the interactions between cells and giant unilamellar vesicles. Therefore, the interaction spectrum of differently charged giant unilamellar vesicles with various cell lines in vitro was systematically investigated . To this end, splitted giant unilamellar vesicles were produced with varying amounts of positively (DOTAP) and negatively (DOPG) charged lipids and their respective Zeta-potential was measured by dynamic light scattering after releasing from the polymer shell (Table 5 below). The results showed that the charge of giant unilamellar vesicles can be fine-tuned between highly positive to highly negative by adjusting respective lipid formulations (table 5 below).

**Table 5: Zeta-potential values of the released giant unilamellar vesicles obtained by microfluidic mechanical division of polymer shell stabilized giant unilamellar vesicles having different lipid compositions.**

| **LIPID** | **Molar percentage** | | | | |
|---|---|---|---|---|---|
| DOTAB | 0 | 0 | 0 | 20 | 50 |
| DOPC | 49 | 79 | 99 | 79 | 49 |
| DOPG | 50 | 20 | 0 | 0 | 0 |
| LissRhodPE | 1 | 1 | 1 | 1 | 1 |
| **Zeta potential (mV)** | **-31** | **-19** | **+2** | **+2** | **+28** |

To quantify the interactions between cells and giant unilamellar vesicles, a plate reader-based attraction assay was implemented and cell lines of endothelial (MDCK), epithelial (A431D and A431) and adrenal (PC12) origin were interfaced with respective giant unilamellar vesicles. These cell lines were selected in order to cover a wide spectrum of possible target tissues with different surface expression patterns. For all tested cell lines, it was found a strong correlation between the charge of giant unilamellar vesicles and the intensity of cell attraction, where higher charged giant unilamellar vesicles showed an increased attraction compared to less and non-charged giant unilamellar vesicles (Figure 7). For example, in the case of A431D cells, which is a frequently used model cell line in carcinoma research, giant unilamellar vesicles with a Zeta-potential of -31 mV showed an almost 100 times higher attraction when compared to giant unilamellar vesicles with +2 mV Zeta-potential. At the same time, giant unilamellar vesicles with a Zeta-potential of +28 mV increased in attraction by about 50 times compared to giant unilamellar vesicles with +2 mV Zeta -potential. However, as this quantitative assay is not able to discriminate between different types of interactions (e.g. uptake, attachment, fusion or engulfment), the qualitative nature of the interaction between giant unilamellar vesicles and cells was further investigated by fluorescence confocal microscopy. As shown in Figure 8, three distinct types of interactions between giant unilamellar vesicles and A431D cells could be induced: Endocytosis and attachment were mainly observed for the negatively-charged and neutral giant unilamellar vesicles, respectively. In case of positively charged giant unilamellar vesicles, colocalization of lipid fluorescence with the cell membrane staining (in many cases accompanied by morbid cell morphologies) was observed, indicating that fusion between both membranes occurred. To assure that negatively charged giant unilamellar vesicles are indeed taken up by the cells, two additional analyses were performed: First, the cell cytoplasm was stained and performed z-resolved confocal fluorescence microscopy of internalized fluorescently labeled giant unilamellar vesicles (data not shown). Second, he cells incubated with respective giant unilamellar vesicles were fixed and analysed at transmission electron microscopy (Figure 9). Both assessments proved that giant unilamellar vesicles are indeed taken up by the cells and reside within their cytoplasm. Taken together, these results revealed an important effect of giant unilamellar vesicle charge on the nature of giant unilamellar vesicle-cell interactions and highlight the ability of the developed method for giant unilamellar vesicle charge control.

### Example 3. Biofunctionalization of giant unilamellar vesicles formed by the splitting microfluidic device

Despite the fact that charge-mediated cellular uptake of giant unilamellar vesicles is an efficient process, it fails to provide a cell type specific delivery of therapeutic compounds. Therefore, the inventors aimed to establish a ligand directed uptake of giant unilamellar vesicles by developing a toolbox of strategies for bio-orthogonal functionalization of the giant unilamellar vesicle surface with ligands targeting specific moieties and cell types. To exemplarily demonstrate the diversity of giant unilamellar vesicle biofunctionalization possibilities, giant unilamellar vesicles were produced by microfluidic mechanically splitting using biotinylated lipids for coupling to streptavidin tagged proteins, nitrilotriacetic acid-nichel (NTA-Ni²⁺)-functionalized lipids for coupling to histidine tagged proteins and DOPE lipids containing primary ammine for linking to N-hydroxysuccimid (NHS) functionalized molecules. Triple orthogonal functionalization of the released giant unilamellar vesicles was achieved by adding streptavidin functionalized Atto425, histidine tagged green fluorescent protein and NHS-functionalized Alexa647 (Figure 10a). Moreover, more complex sequential functionalization strategies were tested. Towards this end, cysteine functionalized gold nanoparticles were immobilized to the giant unilamellar vesicle lipids via NHS-chemistry (Figure 10b). Additionally, a multistep approach was tested to couple immunoglobulins (e.g. anti-CD3) via NTA-immobilized histidine tagged protein G (Figure 10c).

Notably, antibodies offer great selectivity for particular cell surface antigens, wherefore antibody based targeting has previously been shown to greatly enhance specific small unilamellar vesicle delivery to defined cell subsets. To assess the functionality of anti-CD3-coated giant unilamellar vesicles, they were incubated with CD3⁺ Jurkat cells (see Method Section). When analyzed by confocal microscopy, the formation of an attachment site between the giant unilamellar vesicles and the cells, reminiscent of a "minimal" immunological synapse, was observed (Figure 11), indicating successful giant unilamellar vesicle-cell coupling. In contrast, giant unilamellar vesicles without anti-CD3 coating did not show this complex interaction architecture.

### RGD- mediated endocitosis

In order to systematically assess the possibility to apply attractive, receptor-specific giant unilamellar vesicle-cell interactions for targeted giant unilamellar vesicle delivery, negatively charged and RGD biofunctionalized giant unilamellar vesicles were produced with varying ligand densities. RGD was used for giant unilamellar vesicles biofunctionalization (see Method Section), since integrin receptor-based endocytosis has previously been tested to enhance liposomal drug delivery due to the ability of integrin proteins to function as natural intracellular signal transducer for the initiation of endocytic events. Therefore, RGD-giant unilamellar vesicles were interfaced with adherent cell lines expressing RGD-binding integrin receptors and their attractions measured. As a control, the same cells were incubated with non-biofunctionalized, naive giant unilamellar vesicles. For all tested cell lines (Figure 12), it was found that RGD ligand density on the periphery correlates with giant unilamellar vesicle attraction and that giant unilamellar vesicle-cell coupling could be increased by approximately 10 % when applying 10 mol%-RGD ligand decoration. In case of non-adherent Jurkat T-cells, which express high levels of α₄β₁ integrin, 10 mol%-RGD coating increased giant unilamellar vesicle-cell coupling even by 15-fold (as measured by fluorescence flow cytometry). The nature of interaction of fluorescently labelled RGD functionalized giant unilamellar vesicles was further analyzed by confocal microscopy, revealing that when interfaced with A431D cells, giant unilamellar vesicles mostly accumulate at the cell periphery, the region of highest integrin density, and in the perinuclear region, suggesting RGD-integrin mediated endocytotic giant unilamellar vesicle uptake by the cells (Figure 13).

### Example 4. PEG-based passivation strategy to regulate attractive and repulsive giant unilamellar vesicle-cell interactions for enhance targeted delivery

Although biofunctionalization of giant unilamellar vesicles with anti-CD3 and RGD ligands successfully increased giant unilamellar vesicle-cell interactions, charge-driven and other non-specific attractions at the giant unilamellar vesicle-cell interface might still be high enough to interfere with the ligand-based cell type specific uptake. For example, when incubating non-charged giant unilamellar vesicles functionalized with NrCAM protein with SH-SY5Y neuroblastoma cells (NrCAM positive), the measured attraction was comparable to that of non-functionalized and non-charged giant unilamellar vesicles (Figure 14b). This indicates that non-specific lipid-cell interactions are considerably high. Giant unilamellar vesicles covered by poly-ethylenglycol (PEG) were synthetized to shield these electrostatic and non-specific interactions. Initially it was tested the shielding potential of PEG at different concentrations and molecular weight for giant unilamellar vesicles of different charges. To this aim, giant unilamellar vesicles were produced with negatively and positively charged lipids at lipid ratios between 15 and 50 mol%. Lipids linked to PEG350, PEG750 or PEG1000 were tested at ratios comprised between 5, 10, 20 and 50 mol% for the. Zeta-potential of respective small unilamellar vesicles before production of droplet-stabilized giant unilamellar vesicles and Z-potential of released giant unilamellar vesicles were measured (Figure 15a). The results revealed that the Z-potential of both, negatively and positively charged vesicles decreased with increasing PEG chain length and rate of PEGylation. Additionally, it was tested whether PEGylation of giant unilamellar vesicles might be used to shield giant unilamellar vesicle surface charge, thus introducing a repulsive behavior between giant unilamellar vesicles and cells. Towards this end, the PEGylated giant unilamellar vesicles were incubated with six different cell lines established from different tissues and measured respective giant unilamellar vesicle attraction (Figure 15b). The results showed that for all tested cell lines, giant unilamellar vesicle PEGylation basically decreased the charge-mediated attraction between the giant unilamellar vesicles and the cells. Moreover, this effect was more pronounced in cases of higher PEGylation rates and longer PEG length. For example, in the case of giant unilamellar vesicles interactions with A431D carcinoma cells, giant unilamellar vesicles equipped with 5 mol% PEG350 showed almost 50 % more attraction compared to giant unilamellar vesicles equipped with 50 mol% PEG350 (Figure 16). Consistently, confocal microscopy analysis showed that naive negatively charged giant unilamellar vesicles were usually localized within or above cells and only a small fraction was found between single cells or cell groups (Figure 17 upper panel). In contrast, PEGylated giant unilamellar vesicles were observed mostly accumulating in the intercellular space, forming contact inhibition zones between the giant unilamellar vesicle-accumulations and individual cells (Figure 17 lower panel). Most probably, this behavior can be attributed to the repulsive giant unilamellar vesicle-cell interactions.

In conclusion, the inventors were able to develop complementary strategies for the regulation of specific attractive interactions between cells and giant unilamellar vesicles, in order to promote the ligand-based specific interactions, by using a PEG-based passivation strategy to suppress charge-mediated interactions.

### Example 5 Combination of PEGylation and ligand-directed cell interactions.

After establishing the approaches to control and fine-tune the attractive and repulsive interactions between cells and giant unilamellar vesicles, both approaches were combined followed by the screening of cell type selectivity for giant unilamellar vesicle targeting. Towards this end, giant unilamellar vesicles were produced comprising 20 mol% negatively charged DOPG lipids, 59 mol% neutral DOPC lipids, 20 mol% PEG750-linked lipids and 1 mol% NHS coupled lipids for ligand immobilization. In this lipid composition, the net strength of ligand-receptor interactions between the giant unilamellar vesicle and the target cell needs to be strong enough to overcome the PEG-mediated repulsion, eventually allowing cell type specific endocytosis induced by the negative giant unilamellar vesicle charge. To assess the specificity, 15 types of differently functionalized giant unilamellar vesicles were first screened, each of which was expected to be either specific for a given cell type (e.g. anti-cadherin antibodies or bradykinin) or non-specific (e.g. poly-L-lysine) by measuring respective attraction values for six different carcinogenic cell lines. Respective carcinogenic cells might resemble potentially interesting targets in a giant unilamellar vesicle-based tumor treatment. In order to reference all attraction values for each cell type to a common moderately non-reactive protein, all values were normalized to the attraction of BSA-coupled giant unilamellar vesicles (Figure 18a). Figure 18b shows the summary of the attraction between the six differently functionalized giant unilamellar vesicles and six cell lines of different origin. Giant unilamellar vesicles coated with peptides and proteins which do not bind to cells in a specific manner showed high attraction to basically all cell types, as the non-specific attractions are able to overcome the repulsive PEG-barrier in all cases; examples thereof are poly-L-lysine, which mostly interacts based on electrostatic interaction, wheat germ agglutinin WGA, which binds to the glycocalyx of cells, or the HIV derived tat-peptide, which is an arginine rich peptide which penetrates cell membranes mostly based on hydrophobic interactions. However, when targeting more specific receptors, like the bradykinin specific G-protein coupled receptors, which are abundantly expressed in endothelial cells, by functionalizing giant unilamellar vesicles with the vasodilator bradykinin, specific attraction to endothelial cells was achieved. This attraction was increased up to 40 % compared to the other cell lines and similar results could be obtained when targeting cadherin proteins by coating with recombinant cadherin or anti-cadherin antibodies. This comparison shows that fine tuning of attractive interactions and PEG-based shielding of giant unilamellar vesicle surface charges and ligands is a well-suited strategy to gain control over the giant unilamellar vesicle-cells interplay.

Finally, to assess whether the preferential attraction is able to induce a cell-type specific uptake under concurring conditions in a more complex multi-cell type environment, the giant unilamellar vesicle targeting approach was tested in co-culture experiments. Towards this end, astrocyte (Hs683) and neuronal (SH-SY5Y) model cell lines were chose, as these cell types closely grow and interact in the mammalian brain. Moreover, achieving preferential giant unilamellar vesicle uptake by neurons is desirable when developing therapeutic strategies for neuroblastoma or neurodegenerative diseases. To test the preferential attraction, giant unilamellar vesicles were prepared using 20 mol% PEG750, 20 mol% EggPG, 58 mol% EggPC, 1 mol% LissRhod PE and 1 mol% 18:1 DGS-NTA(Ni) lipids and functionalized with a His-tagged neuronal adhesion molecule NrCAM (extracellular domain aa20 - 630) which binds to axonin-1 on neuronal membranes (Figure 19). Following 24 hours of co-culturing giant unilamellar vesicles with the two cell types, confocal microscopy was performed to analyze and to quantify the attraction of giant unilamellar vesicles to each cell type from respective images (see Method Section). The results revealed that neuronal cells contained up to 520 % more giant unilamellar vesicles compared to astrocytes, highlighting the importance of fine-tuning of attractive and repulsive interaction towards targeted delivery of giant unilamellar vesicles in a complex environment.

### Example 6. Lysosomal escape of giant unilamellar vesicles for efficient cytoplasmic cargo delivery.

To investigate the mechanism of giant unilamellar vesicles intracellular uptake, negatively charged giant unilamellar vesicles were incubated with REF52 cells stained for endosomal vesicles and cytoplasm. Confocal microscopy of respective cultures revealed that uptaken giant unilamellar vesicles are surrounded by endosomal membranes (Figure 20a). This observation confirmed that giant unilamellar vesicles enter into the cells by endocytic pathways, e.g. micropinocytosis or phagocytosis, excluding other uptake mechanisms such us direct penetration or sole engulfment of the giant unilamellar vesicles. However, following uptake, a progressive loss of giant unilamellar vesicle fluorescence was observed over time and the giant unilamellar vesicles colocalized with lysosomes (identified by staining with LysoTracker Green DND-26), following 24 hours of incubation (Figure 20b). This observation might be attributed to lysosomal degradation of the vesicles, a process which is frequently observed in case of small unilamellar vesicle-based delivery methods. Yet, many pharmacological compounds target cytoplasmic components, therefore efficient lysosomal escape mechanisms, which allow for the release of giant unilamellar vesicle cargo into the cell and avoid lysosomal degradation, are a pivotal requirement for such applications.

To circumvent this degradation, two independent lysosomal escape mechanisms were assessed (see Method Section), both based on the inhibition of the sudden decrease in pH occurring during endosome-lysosome fusion: 1) Lysosomal escape via a proton sponge mechanism by incorporation of high molecular weight poly-ethylene-imine (PEI) into the giant unilamellar vesicles; 2) Lysosomal escape via intra-lysosomal fusion by incorporation of the pH sensitive lipid DOBAQ into the giant unilamellar vesicle membrane. The retention, degradation and release of giant unilamellar vesicle cargo was exemplarily assessed by loading the giant unilamellar vesicles with the membrane impermeable dye HPTS and observing its intracellular fluorescence distribution 24 hours after incubation of respective giant unilamellar vesicles with A431D cells (Figure 21). HPTS is a highly water-soluble pH indicator with a pKa of approx. 7.3 in aqueous buffer. In the cases of PEI-loaded giant unilamellar vesicles, HPTS fluorescence was exclusively detected in punctuated form, colocalizing with the giant unilamellar vesicle fluorescence inside the cells. This indicates HPTS retention inside the giant unilamellar vesicles and therefore endosomal or lysosomal entrapment, suggesting no successful cytoplasmic cargo release. However, for giant unilamellar vesicles containing 60 mol% DOBAQ, HPTS fluorescence could be found distributed within the whole cell body, proving successful release of HPTS from the giant unilamellar vesicles into the cytoplasm. Indeed, DOBAQ-containing giant unilamellar vesicles show polarity switching of their Z-potential at low pH, as assessed by dynamic light scattering (Figure 22). Notably, when tracking the total intracellular giant unilamellar vesicle number over time, the inventors found that PEI loaded giant unilamellar vesicles accumulated within the cells and did not undergo degradation (Figure 23). This suggest that, even though no cargo release is taking place, PEI loaded giant unilamellar vesicles are protected from degradation, potentially because PEI can act in the giant unilamellar vesicle lumen as a potent pH buffer preventing acidic degradation and mature lysosome formation. For approaches in which stable incorporation of giant unilamellar vesicles and their cargo into cells is required, this might represent a promising implementation mechanism.

The dynamics of endocytosis and lysosomal activity can significantly vary between transformed and non-transformed cells, therefore the inventors aimed to test the approach on primary cells as well. Towards this end, in vitro cultured primary hippocampal neurons, which represent a medically-relevant cell type, were used. Indeed, these cells represent an important target in many therapeutic procedures for neurodegeneration or neuronal tumors. When interfaced with HPTS loaded and DOBAQ containing negatively charged giant unilamellar vesicles, extensive uptake of the giant unilamellar vesicles into the neurons was observed 24 hours after incubation (Figure 24). In order to enhance their attraction to the sialic acid containing glycocalyx, the giant unilamellar vesicles were functionalized with wheat germ agglutinin (WGA). Their accumulation in the perinuclear region suggests incorporation into the cell's intracellular trafficking machinery. Importantly, widespread distribution of HPTS fluorescence within the neuronal soma and in the dendrites was observed. This observation proves that DOBAQ based lysosomal escape of giant unilamellar vesicle cargo is also functional and compatible with primary cells.

### Example 7. Targeted delivery of large heavy duty cargoes

In order to ultimately test the cargo capacity of microfluidically-formed giant unilamellar vesicles for novel therapeutic approaches, microfluidic loading of purified baculoviruses (BV) was performed. BVs are considered as promising future candidates for transduction and handling of large amounts of genetic material in genome engineering approaches. Successful assembly and release of BV-loaded giant unilamellar vesicles was achieved (Figure 25). Following the formation, the BV-loaded DOBAQ-containing giant unilamellar vesicles were incubated with REF52 cells for 24 hours. Confocal microscopy analysis revealed intracellular uptake of the giant unilamellar vesicles, as well as release of the BVs (stained by Hoechst 33342, see Method Section) into the cell cytoplasm. This was accompanied by the expression of mitochondrial targeted dsRed protein encoded by the BV, indicating successful giant unilamellar vesicle based transduction of mammalian cells with the baculovirus and its content. These results highlight the advantages of using giant unilamellar vesicle-based drug delivery for more efficient drug administration of advanced cargoes that would not be possible by conventional delivery methods.

### Example 8. Integration of proteins by microfluidic techniques

Integrin α_{IIb}β₃ (Uniprot ID: P08514/ P05106) was purified from outdated human blood platelets using TBS and Triton X-100 as described by WO 2018/228894 A1. Integrin α_{IIb}β₃ was reconstituted into large unilamellar vesicles by the detergent removal method. Therefore, dried egg PC was dissolved in a buffer containing 0.1 % of Triton X- 00. Integrin α_{IIb}β₃ was added to a 1 : 1000 integrin-lipid ratio. The solution was incubated at 37° C for 2 hours in a shaker at 600rpm. Triton X- 00 was removed in two subsequent washing steps of 3. 5 hours using 50 mg/ml SM-2 Biobeads. The size distribution of liposomes and integrin-liposomes was measured by dynamic light scattering in a Malvern Zetasizer Nano ZS setup (Malvern, UK) to be around 100 to 140 nm.
Simultaneously, polymer shell-stabilized giant unilamellar vesicles were formed as described in example 1.

Following these preparatory steps, the parent polymer shell stabilized giant unilamellar vesicles were fused with the Integrin-liposomes using a pico-injection device as described in WO 2018/228894 A1.

Thereafter, the formed polymer-shell stabilized giant unilamellar vesicles containing Integrin α_{IIb}β₃ integrated in the lipid bilayer have been splitted in smaller polymer-shell stabilized giant unilamellar vesicles by using the microfluidic splitting unit as disclosed in Example 1.

A similar procedure has been used to integrate other transmembrane proteins into the lipid bilayer such as, for example, F₀F₁-ATP synthase (Uniprot ID: P0ABA0), Alpha(α)-hemolysin (Uniprot ID: P09616), and Gramicidin D (PubChem CID: 45267103).

**Table 6 Lipid compositions and buffer used for production of the giant unilamellar vesicles in the Examples of the disclosure**

| Functional Ligand /modification | Moiety/Macromolecule | Composition | Production b./ release b./ functionalization mix | Example Nr |
|---|---|---|---|---|
| NHS-based: palmitic acid NHS lipid | WGA(wheat germ agglutinin); + lysosomal escape | 5 mol% Palmitic acid NHS | PBS + 10 mM MgCl₂, | |
| | | 1 mol% LissRhod PE, | PBS, | |
| | | 20 mol% EggPG, | 10 mg/ ml Wheat germ agglutinin | |
| | | 14 mol% EggPC | | |
| | | 60 mol% DOBAQ, | | |
| | AuNP | 1 mol% palmitic acid NHS, | PBS + 10 mM MgCl₂, | |
| | | 1 mol% LissRhod PE, | PBS, | |
| | | 20 mol% DOPG, | 6 µM L-cysteine, 10µg/ml 50 nm Au nanoparticles, 1% BSA | |
| | | 78 mol% DOPC | | |
| | BSA, poly-L-Lysine; bFGF, EGF, WGA, anti-cadherin, anti-α4 integrin, IL2, insulin, bradykinin Tat-(HIV)-GFP, cystein, CD95L, e-cadherin, fibronectin | 5 mol% palmitic acid NHS, | PBS + 10 mM MgCl₂, | |
| | | 1 mol% LissRhod PE, | PBS, | |
| | | 54 mol% EggPC, | 0.5 - 6 µM proteins and peptides | |
| | | 20 mol% EggPG, | | |
| | | 20 mol% PEG-750 PE | | |
| NTA-based: DGS-NTA (Ni) lipid | add of His-Tag ProtG to released giant unilamellar vesicles , and then IgG antibody | 1 mol% 18:1 DGS-NTA(Ni), | PBS + 10 mM MgCl₂, | |
| | | 1mol% LissRhod PE, | PBS, | |
| | | 20 mol% EggPG, | 3 µM His-tagged Protein G, 3 µM anti-CD3 AF488, 1% BSA | |
| | | 78 mol% EggPC | | |
| | add of NrCAM | 1 mol% 18:1 DGS-NTA(Ni), | PBS + 10 mM MgCl_{2,} | |
| | | 1 mol% LissRhod PE, | PBS, | |
| | | 20 mol% EggPG, | 3 µM His-tagged NrCAM | |
| | | 58 mol% EggPC, | | |
| | | 20 mol% PEG750 PE | | |
| DSPE-RGD | RGD | 0/1/2/10 mol % DSPE-RGD, | | |
| | | 1 mol% LissRhod PE, | | |
| | | 20 mol% DOPG, | | |
| | | 79/78/77/69 mol% DOPC | | |
| NTA and Biotin bound to the lipid | | 1% mol 18:1 DGS-NTA(Ni), | PBS + 10 mM MgCl₂, | |
| | | 1 mol% 18:1-12:0 Biotin PE, | PBS, | |
| | | 1 mol% LissRhod PE, | 1.5 µM 6xHis- GFP, | |
| | | 1 mol% DOPE, | 1.5 µM Atto425-SAV, | |
| | | 20 mol% DOPG, | 1.5 µM Alexa Fluor 647-NHS Ester | |
| | | 77 mol% DOPC | | |
| PEG: PEG350, PE, PEG750 PE, PEG1000 PE in the lipid mixture for SUV production | | 1 mol% LissRhod PE, | PBS + 10 mM MgCl_{2,} or only PBS for DOTAP; | |
| | | 5/10/20/50 mol% | | |
| | | PEG350/750/1000 PE, | PBS | |
| | | 15 mol% EggPG, or DOTAP | | |
| | | 79/74/64/34 mol% EggPC | | |
| | | 1 mol% LissRhod PE, | PBS + 10 mM MgCl_{2,} or only PBS for DOTAP; | |
| | | 5/10/20/50 mol% | | |
| | | PEG350/750/1000 PE, | PBS | |
| | | 50(49) mol% EggPG or DOTAP | | |
| | | 44/39/29/0 mol% EggPC | | |

| Lysosome Escape | Composition | Production buffer / release buffer | Example Nr |
|---|---|---|---|
| PEI | 1 mol% LissRhod PE, | PBS + 10 mM MgCl₂ + 44 µg/ml | |
| | 20 mol% EggPG, | Polyethylenimine (PEI), | |
| | 79 mol% EggPC, | PBS | |
| DOBAQ | 1 mol% LissRhod PE, | PBS + 60 mM MgCl_{2,} | |
| | 20 mol% EggPG, | PBS | |
| | 19 mol% EggPC, | | |
| | 60 mol% DOBAQ, | | |

## Claims

1. A method for preparation of monodisperse cell-targeting giant unilamellar vesicles comprising the following steps:
a) providing a polymer shell-stabilized giant unilamellar vesicle;
b) mechanically symmetrically dividing the polymer shell-stabilized giant unilamellar vesicle into two smaller polymer shell-stabilized giant unilamellar vesicles without harming the giant unilamellar vesicle,
c) repeating step b) by mechanically symmetrically dividing the smaller polymer shell-stabilized giant unilamellar vesicles provided in step b) until polymer shell-stabilized giant unilamellar vesicles reach a desired diameter smaller than 10 µm.
d) optionally removing the polymer shell from the polymer shell-stabilized giant unilamellar vesicles obtained in step c).

2. The method according to claim 1, wherein the polymer shell-stabilized unilamellar vesicle provided in step a) is obtained by the following step:
a') merging a water phase comprising at least one lipid, and cations, and an oil phase comprising an amphiphilic copolymer,
a") optionally integrating one or more proteins or fragments thereof into the polymer shell stabilized giant unilamellar vesicle provided in step a')

3. The method according to any of the claims 1 or 2, wherein step d) comprises removing the polymer shell from the polymer shell-stabilized giant unilamellar vesicles obtained in step c) by adding a deemulsifier.

4. The method according to any one of the claims 1 - 3, further comprising the following step e) after step d):
e) purifying the giant unilamellar vesicles by centrifugation.

5. The method according to any one of the claims 2 - 4, wherein the water phase of step a') comprises at least one lipid selected from the group comprising:
a neutral lipid selected from the group comprising ceramide, sphingomyelin, cephalin, cholesterol, cerebrosides, diacylglycerols, phosphatidylcholines, lysophosphatidylcholines, phosphatidylethanolamines, lysophosphatidylethanolamine, lysoethanolamines, inverted headgroup lipids, sphingosins, sterol-modified phospholipids, ether ester lipids, diether lipids, vinyl ether (plasmalogen);
an anionic lipid selected from the group comprising phosphatidic acids, lysophosphatidic acid derivatives, phosphatidylglycerols, lysophosphatidylglycerols, phosphatidylserines, lysophosphatidylserines, phosphatidylinositols, phosphatidylinositolphosphates, cardiolipins, Bis(Monoacylglycero)Phosphate derivatives;
a cationic lipid selected from the group comprising dioleyl-N,N-dimethylammonium chloride; N-(2,3-dioleyloxy)propyl)-N,N,N-trimethylammonium chloride; N,N-distearyl-N,N-dimethylammonium bromide; N-(2,3- dioleyloxy)propyl)-N,N,N-trimethylammonium chloride; 3β-(N-(N',N'-dimethylaminoethane)-carbamoyl)cholesterol; 1,2-dimyristyloxypropyl-3-dimethyl-hydroxy ethyl ammonium bromide; 2,3-dioleyloxy-N-[2(sperminecarboxamido) ethyl]-N,N-dimethyl-1-propanaminium trifluoroacetate; dioctadecylamidoglycyl carboxyspermine; N-(2,3-dioleyloxy)propyl)-N,N-dimethylammonium chloride and 1,2-dioleoyl-3-dimethylammonium-propane;
a pH-sensitive lipid selected from the group comprising lipid N-(4-carboxybenzyl)-N,N-dimethyl-2,3-bis(oleoyloxy)propan-1-aminium, 1,2-distearoyl-3-dimethylammonium-propane, 1,2-dipalmitoyl-sn-glycero-3-succinate, 1,2-dioleoyl-sn-glycero-3-succinate, N-palmitoyl homocysteine ;
a photoswitchable lipid;
acylglycine derivatives, prenol derivatives, prostaglandine derivatives, glycosylated diacyl glycerols, eicosanoid derivatives, (palmitoyloxy)octadecanoic acid derivatives, diacetylene derivatives, diphytanoyl derivatives, fluorinated lipids, brominated lipids, lipopolysaccharides;
one of the aforementioned lipids coupled to a functional ligand selected from biotin, N-hydroxysuccinimide ester, nitrilotriacetic acid -nickel, amine, carboxylic acid, maleimides, dithiopyridinyl, pyridyl disulfide, pyridyldithiopropionate, N-benzylguanine, carboxyacyl, cyanur, folate, square, galloyl, glycan, thiol, arginylglycylaspartic acid, a fluorescent dye molecule, a magnetic resonance imaging reagent, a chelator;
one of the aforementioned lipids coupled to polyethyleneglycol with a molecular weight comprised between 350 and 50,000 g/mol.

6. The method according to any one of the claims 2 - 5, wherein the water phase of step a') comprises at least one anionic lipid, at least one neutral lipid, and optionally one neutral lipid functionalized with a fluorescent dye molecule.

7. The method according to any one of the claims 2 - 5, wherein the water phase of step a') comprises at least one cationic lipid, at least one neutral lipid, and optionally one neutral lipid functionalized with a fluorescent dye molecule.

8. The method according to any one of the claims 2 - 7, wherein the water phase of step a') comprises at least one lipid functionalized with a functional ligand selected from biotin, N-hydroxysuccinimide (NHS) ester, sulfo-NHS ester, nitrilotriacetic acid (NTA)-nickel, amine, carboxylic acid, maleimide, dithiopyridinyl, pyridyl disulfide, pyridyldithiopropionate, N-benzylguanine, carboxyacyl, cyanur, square, galloyl, thiol,
wherein the method optionally comprises after step d) the following step:
d') coupling the giant unilamellar vesicles with at least one macromolecule comprising at least one moiety reacting with one of said functional ligands, wherein the macromolecule is selected from the group comprising a carbohydrate, a nucleic acid, a protein or a fragment thereof, a polypeptide, a cell receptor, an imaging probe, a nanoparticle.

9. The method according to any one of the claims 2 - 8, wherein the water phase of step a') comprises at least one lipid coupled to polyethyleneglycol with a molecular weight comprised between 350 and 50,000 g/mol.

10. The method according to any one of the claims 2 - 9, wherein the water phase of step a') comprises at least one pH-sensitive lipid at a molar percentage comprised between 20 % - 80 %, or
wherein the water phase of step a') further comprises poly-ethylene-imine at a concentration comprised between 2 - 100 µg/ml.

11. The method according to any one of the claims 2 - 10, wherein the water phase of step a') further comprises at least one agent selected from the group comprising drug releasing porous particles, molecular imaging agents, diagnostic agents, therapeutic agents, proteins or fragments thereof, polypeptides, peptides, enzymes or fragments thereof, nucleic acids, oligonucleotides, polynucleotides, up-and-coming DNA origami robots, small molecule drugs, virus particles, virus-like particles, microbial antigens, steroids, proteoglycans, lipids, monosaccharides, oligosaccharides, polysaccharides, magnetic particles, nanorods, carbon nanotubes, dentritosomes, polymerosomes, metal nanoparticles and combinations or conjugates thereof.

12. The method according to any one of the claims 2 - 11, wherein the amphiphilic copolymer of step a') consists of (i) a triblock copolymer comprising two perfluorinated polymer end blocks and one polyether glycol block, or of (ii) a diblock copolymer comprising one perfluorinated polymer end block and a polyether glycol block, wherein the triblock or diblock copolymer is folded so that the perfluorinated polymer end blocks are arranged at the outer side and the polyether glycol block is arranged at the inner side of the polymer shell.

13. The method according to any one of the claims 1 - 12, wherein step b) comprises mechanically dividing said polymer shell stabilized giant unilamellar vesicle into two smaller polymer shell stabilized giant unilamellar vesicles using a microfluidic device comprising at least one Y- shaped junction, wherein said Y-shaped junction consists of one inlet channel and two outlet channels, and wherein step c) comprises repeating the step b) by using four or more sequential generations of Y-shaped junctions, wherein the inlet channel of each Y-shaped junction consists of the outlet channel of the previous Y-shaped junction.

14. A microfluidic device for preparing polymer shell stabilized giant unilamellar vesicles having a diameter smaller than 10 µm, comprising a division zone and a flow-rate control system,
wherein the division zone comprises one or more sequential generations of Y-shaped junctions, wherein each Y-shaped junction consists of one inlet channel and two outlet channels, and wherein the inlet channel of each Y-shaped junction consists of the outlet channel of the previous junction.

15. The microfluidic device of claim 14, further comprising a generation zone of a parent polymer shell stabilized giant unilamellar vesicle positioned upstream the division zone, said generation zone comprising a flow-focusing junction consisting of a horizontal inlet channel and two vertical inlet channels, wherein said three inlet channels converge into an outlet channel through a narrow orifice, wherein said outlet channel is connected to the division zone.
